# EUROPEAN PATENT SPECIFICATION

(11) **EP 3 918 989 B1**
(45) Date of publication and mention of the grant of the patent: **18.09.2024**
(21) Application number: 21185773.5
(22) Date of filing: 23.12.2011
(51) Int. Cl.: A61B 5/06, A61B 5/055, A61B 8/08, A61B 6/00, A61B 8/00, A61B 10/02, A61B 10/06, A61B 90/98, A61B 34/20, A61B 90/00, A61B 6/50, A61B 5/00

(54) **SYSTEMS AND METHODS FOR GUIDING A MEDICAL INSTRUMENT**
SYSTEME UND VERFAHREN ZUR FÜHRUNG EINES MEDIZINISCHEN INSTRUMENTS
SYSTÈMES ET PROCÉDÉS DE GUIDAGE D'INSTRUMENT MÉDICAL

(30) Priority: 27.05.2011 US 201013118138; 27.05.2011 US 201113118033; 23.12.2010 US 201061426996 P
(43) Date of publication of application: 08.12.2021
(62) Divisional of application: 11850625.2
(73) Proprietor: Bard Access Systems, Inc., Salt Lake City, UT 84116 (US)
(72) Inventor: Silverstein, Fred E., Seattle, WA, 98112 (US); Golden, Robert N., Kirkland, WA, 98033 (US)
(74) Representative: Hoffmann Eitle

(56) References cited:
- WO-A2-2007/033379
- CN-A- 101 122 836
- US-A1- 2004 059 217
- US-A1- 2006 287 595
- US-A1- 2007 055 142
- US-A1- 2007 093 710
- US-A1- 2007 282 197
- US-A1- 2009 062 646
- US-A1- 2009 312 629
- US-A1- 2010 130 858
- BLEZEK D J ET AL: "Virtual reality simulation of regional anesthesia for training of residents", SYSTEM SCIENCES, 2000. PROCEEDINGS OF THE 33RD ANNUAL HAWAII INTERNATIONAL CONFERENCE ON JAN 4-7, 2000, PISCATAWAY, NJ, USA,IEEE, 4 January 2000 (2000-01-04), pages 1669 - 1676, XP010545427, ISBN: 978-0-7695-0493-3
- VASILE NISTOR ET AL: "<title>Immersive training and mentoring for laparoscopic surgery</title>", PROCEEDINGS OF SPIE, VISUAL COMMUNICATIONS AND IMAGE PROCESSING 2005, vol. 6528, 4 April 2007 (2007-04-04), Visual Communications and Image Processing 2005, 2005, Beijing, China, pages 65280Q - 65280Q-11, XP055208768, ISSN: 0277-786X, DOI: 10.1117/12.717199
- MASAHIKO NAKAMOTO ET AL: "Intraoperative Magnetic Tracker Calibration Using a Magneto-Optic Hybrid Tracker for 3-D Ultrasound-Based Navigation in Laparoscopic Surgery", IEEE TRANSACTIONS ON MEDICAL IMAGING, IEEE, USA, vol. 27, no. 2, 1 February 2008 (2008-02-01), pages 255 - 270, XP007908166, ISSN: 0278-0062, DOI: 10.1109/TMI.2007.911003

## Description

US 2009/0062646 A1 discloses a method of operating a remote medical navigation system using ultrasound, employing ultrasound imaging from a medical device to supplement or to replace conventional X-ray imaging of the operating region during navigation.

The publication by Blezek, D J, et al., entitled "Virtual reality simulation of regional anesthesia for training of residents", (System sciences, 2000, Proceedings of the 33rd annual Hawaii international conference on Jan 4.7, 2000; ISBN: 978-0-7695-0493-3) provides relevant disclosure for understanding the background of the present invention. Namely, it discloses an augmented reality surgical visualisation system, comprising magnetic tracking of surgical instrumentation and providing haptic feedback to the user. It fails to disclose an instrument made of rubber.

### BRIEF SUMMARY

The present invention is directed to the virtual tracking system for a virtual medical device of claim 1 and the use of claim 9. The dependent claims refer to preferred embodiments.

Briefly summarized, embodiments of the present invention are directed to an integrated catheter placement system configured for accurately placing a catheter within the vasculature of a patient. The integrated system employs at least two modalities for improving catheter placement accuracy: 1) ultrasound-assisted guidance for introducing the catheter into the patient's vasculature; and 2) a tip location system ("TLS"), or magnetically-based *(e.g.,* via permanent magnet(s) or electromagnet(s)) tracking of the catheter tip during its advancement through the vasculature to detect and facilitate correction of any tip malposition during such advancement.

In one configuration, the integrated system comprises a system console including a control processor, a tip location sensor for temporary placement on a portion of a body of the patient, and an ultrasound probe. The tip location sensor senses a magnetic field of a stylet disposed in a lumen of the catheter when the catheter is disposed in the vasculature. The ultrasound probe ultrasonically images a portion of the vasculature prior to introduction of the catheter into the vasculature. In addition, the ultrasound probe includes user input controls for controlling use of the ultrasound probe in an ultrasound mode and use of the tip location sensor in a tip location mode.

In another configuration, a third modality, *i.e.,* ECG signal-based catheter tip guidance, is included in the system to enable guidance of the catheter tip to a desired position with respect to a node of the patient's heart from which the ECG signals originate.

In addition, configurations of the present disclosure are also directed to a guidance system for assisting with the insertion of a needle or other medical component into the body of a patient. The guidance system utilizes ultrasound imaging or other suitable imaging technology.

In one configuration, the guidance system comprises an imaging device including a probe for producing an image of an internal body portion target, such as a subcutaneous vessel, for instance. One or more sensors are included with the probe. The sensors sense a detectable characteristic related to the needle, such as a magnetic field of a magnet included with the needle.

The system includes a processor that uses data relating to the detectable characteristic sensed by the sensors to determine a position and/or orientation of the needle in three spatial dimensions. The system includes a display for depicting the position and/or orientation of the needle together with the image of the target.

In addition to magnet-based detection, other modalities for detecting the medical component are disclosed, including optically-based and electromagnetic signal-based systems.

In one configuration, a stylet including one or more magnetic elements is removably inserted into the needle to enable tracking of the needle via detection of the magnetic elements by a sensor included with the ultrasound probe. In one embodiment, the sensor is a ring sensor disposed about a portion of the ultrasound probe. In another embodiment, the stylet can additionally include a strain sensor that detects bending of the needle during insertion into the patient. Feedback from the strain sensor can be input into the system and accounted for in order to more accurately depict needle location on the display.

In another configuration, the magnetic element is configured as a donut-shaped passive magnet defining a hole through which the cannula of the needle passes. In yet other embodiments, a guidance system for guiding rigid or other medical instruments is disclosed, together with various example implementations thereof.

These and other features of embodiments and configurations of the present invention will become more fully apparent from the following description and appended claims, or may be learned by the practice of embodiments of the invention as set forth hereinafter. Multiple exemplary embodiments are explained in detail in the following description; those mentioned as being the invention represent the most accurate embodiments which are covered by the appended claims.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a block diagram depicting various elements of an integrated system for intravascular placement of a catheter, according to one example embodiment of the present invention;
FIG. 2 is a simplified view of a patient and a catheter being inserted therein with assistance of the integrated system of FIG. 1;
FIGS. 3A and 3B are views of a probe of the integrated system of FIG. 1;
FIG. 4 is a screenshot of an ultrasound image as depicted on a display of the integrated system of FIG. 1;
FIG. 5 is a perspective view of a stylet employed in connection with the system of FIG. 1 in placing a catheter within a patient vasculature;
FIG. 6 is an icon as depicted on a display of the integrated system of FIG. 1, indicating a position of a distal end of the stylet of FIG. 5 during catheter tip placement procedures;
FIGS. 7A-7E depict various example icons that can be depicted on the display of the integrated system of FIG. 1 during catheter tip placement procedures;
FIGS. 8A-8C are screenshots of images depicted on a display of the integrated system of FIG. 1 during catheter tip placement procedures;
FIG. 9 is a block diagram depicting various elements of an integrated system for intravascular placement of a catheter, according to another example embodiment of the present invention;
FIG. 10 is a simplified view of a patient and a catheter being inserted therein with assistance of the integrated system of FIG. 9;
FIG. 11 is a perspective view of a stylet employed in connection with the integrated system of FIG. 9 in placing a catheter within a patient vasculature;
FIGS. 12A-12E are various views of portions of the stylet of FIG. 11;
FIGS. 13A-13D are various views of a fin connector assembly for use with the integrated system of FIG. 9;
FIGS. 14A-14C are views showing the connection of a stylet tether and fin connector to a sensor of the integrated system of FIG. 9;
FIG. 15 is a cross sectional view of the connection of the stylet tether, fin connector, and sensor shown in FIG. 14C;
FIG. 16 is simplified view of an ECG trace of a patient;
FIG. 17 is a screenshot of an image depicted on a display of the integrated system of FIG. 9 during catheter tip placement procedures;
FIG. 18 is a block diagram depicting various elements of an ultrasound-based guidance system for needles and other medical components, according to one embodiment;
FIG. 19 is a simplified view of a patient and a catheter being inserted therein, showing one possible environment in which the guidance system of FIG. 18 can be practiced;
FIG. 20 is a top view of the ultrasound probe of the guidance system of FIG. 18;
FIG. 21A is a side view of a needle for use with the guidance system of FIG. 18, according to one embodiment;
FIG. 21B is an end view of the needle of FIG.21A;
FIGS. 22A and 22B are simplified views of the ultrasound probe of the guidance system being used to guide a needle toward a vessel within the body of a patient;
FIGS. 23A and 23B show possible screenshots for depiction on the display of the guidance system, showing the position and orientation of a needle according to one embodiment;
FIG. 24 shows various stages of a method for guiding a needle to a desired target within the body of a patient according to one embodiment;
FIG. 25 shows a sensor array for attachment to an ultrasound probe and associated display, according to one embodiment;
FIG. 26 is a simplified view of a needle holder gun for use with the guidance system of FIG. 18, according to one embodiment;
FIG. 27 is a simplified view of an ultrasound probe and needle including elements of an optical guidance system, according to one embodiment;
FIG. 28 shows operation of the ultrasound probe and needle of FIG. 27, according to one embodiment;
FIG. 29 is a simplified view of an ultrasound probe and needle including elements of an electromagnetic signal-based guidance system, according to one embodiment;
FIG. 30 is a simplified view of an ultrasound probe and needle including elements of an electromagnetic signal-based guidance system, according to another embodiment;
FIGS. 31A-31D are various views of a needle and associated components for use with a needle guidance system, according to one embodiment;
FIG. 32 is a side view of a needle for use with a needle guidance system, according to one embodiment;
FIGS. 33A and 33B are various views of a needle for use with a needle guidance system, according to one embodiment;
FIGS. 34A-34G are views of variously shaped magnetic elements for use with a needle guidance system according to one embodiment;
FIG. 35 is a perspective view of a distal portion of a needle cannula including a magnet-bearing stylet disposed therein, according to one embodiment;
FIG. 36 shows the needle of FIG. 35 in use with an ultrasound probe including a ring sensor, according to one embodiment;
FIG. 37 is a perspective view of a needle including a donut magnet disposed on the cannula, according to one embodiment;
FIG. 38 is a side view of a stylet including a strain gauge according to one embodiment;
FIGS. 39A-39B show the stylet and strain gauge of FIG. 38 under bending stress;
FIG. 40 is a side view of a stylet including a flex sensor according to one embodiment;
FIG. 41 illustrates a patient undergoing a medical procedure with equipment including a rigid medical device tracking system working in cooperation with a medical imaging system according to one embodiment;
FIG. 42 illustrates a combination useful in several embodiments for a rigid medical device tracking system according to one embodiment;
FIGS. 43A-43C illustrate the introduction of a rigid medical device through the skin of a patient into a particular area of concern such as a tumor according to one embodiment;
FIG. 44 illustrates the use of a rigid medical device tracking system in a virtual procedure according to one embodiment;
FIG. 45 illustrates a virtual image of a needle including selected structural characteristics and shown as overlaid on top of a CT image according to one embodiment;
FIG. 46 illustrates a virtual image of a needle including selected structural characteristics and shown as overlaid on top of an ultrasound image according to one embodiment;
FIG. 47 illustrates an ultrasound image with both a virtual image overlay and a real time image overlay according to one embodiment;
FIG. 48 illustrates an ultrasound image including a virtual representation of a rigid medical device *(e.g.,* a needle) introduced into a patient's body according to one embodiment;
FIGS. 49A and 49B illustrate a virtual rigid medical device placed close to a patient so that a medical practitioner can anticipate what will actually happen when the real rigid medical device is inserted according to one embodiment;
FIG. 50 illustrates a rigid medical device being tracked in a patient's body, while concurrently another medical device also is tracked in the patient's body according to one embodiment;
FIGS. 51A and 51B illustrate an imaging device using a pressure sensitive technology (*e.g*., pressure ink) to perform a medical procedure on a patient's breast according to one embodiment;
FIG. 52 illustrates an imaging device used to produce a three dimensional representative image of part of a patient's anatomy according to one embodiment;
FIG. 53 illustrates a patient's head shown with fiducial marks according to one embodiment;
FIG. 54 illustrates another embodiment wherein imaging information from one or more medical imaging systems is used to produce a representative view of the structures inside a patient's body according to one embodiment;
FIGS. 55A and 55B illustrate a procedure including a treatment that performs a difficult lumbar puncture according to one embodiment;
FIG. 56 illustrates an orthopedic procedure according to one embodiment;
FIG. 57 illustrates three non-limiting embodiments of rigid medical devices that can be tracked with the rigid medical device tracking system according to one embodiment;
FIGS. 58A and 58B illustrate a rigid medical device tracking system tracking two separate and distinct rigid medical devices according to one embodiment;
FIG. 59 illustrates tracking a curved rigid medical device with an ultrasound medical imaging system according to one embodiment;
FIG. 60 illustrates a rigid medical device including ergonomic features according to one embodiment;
FIG. 61 illustrates a rigid medical device including multiple functions according to one embodiment;
FIG. 62 illustrates a rigid medical device configured as a device with jaws for use in a biopsy for example according to one embodiment;
FIGS. 63A and 63B illustrate a rigid medical device including both a sheath and an integrated brush that is withdrawn into the sheath when not in use, and extended out of the sheath when in use according to one embodiment;
FIGS. 64A-64C illustrate non-limiting functions that may be integrated into a rigid medical device according to one embodiment;
FIG. 65 illustrates a suction biopsy tube that can be integrated into a rigid medical device for cooperative use with a rigid medical device tracking system according to one embodiment;
FIG. 66 illustrates a heater probe integrated into a rigid medical device for cooperative use with a rigid medical device tracking system and for directly heating a targeted area according to one embodiment;
FIG. 67 illustrates an anchor tube integrated into a rigid medical device for cooperative use with a rigid medical device tracking system and for anchoring into a targeted area according to one embodiment;
FIG. 68 illustrates a multiple biopsy tube integrated into a rigid medical device for cooperative use with a rigid medical device tracking system according to one embodiment;
FIG. 69 illustrates a large biopsy tube capable of tissue removal for therapy integrated into a rigid medical device for cooperative use with a rigid medical device tracking system according to one embodiment;
FIG. 70 illustrates another large biopsy tube integrated into a rigid medical device for cooperative use with a rigid medical device tracking system according to one embodiment;
FIG. 71 illustrates an ultrasound imaging probe integrated into a rigid medical device for cooperative use with a rigid medical device tracking system according to one embodiment;
FIG. 72 illustrates a camera-enabled probe integrated into a rigid medical device for cooperative use with a rigid medical device tracking system according to one embodiment;
FIG. 73 illustrates a tube to implant markers that can be used for subsequent therapy such as surgery, biopsy, radiotherapy (external or internal), freezing, or other reasons according to one embodiment;
FIG. 74 illustrates a grasper tube that can be used for holding tissue or other reasons according to one embodiment;
FIG. 75 illustrates a tube for depositing markers within the patient's body for subsequent imaging according to one embodiment;
FIG. 76 illustrates the use of skin and subcutaneous fiducials together with a rigid medical device according to one embodiment;
FIG. 77 illustrates a biomarker tube that includes a series of biomarkers on the tip of the biomarker tube for diagnosis in situ according to one embodiment;
FIG. 78 illustrates a needle with marks read by an encoder to indicate the depth of insertion according to one embodiment;
FIG. 79 illustrates an over tube that can be used to direct a needle or other medical device to a particular area of concern in a patient's body according to one embodiment;
FIG. 80 illustrates various details regarding encoding an over tube according to one embodiment;
FIG. 81 illustrates an over tube embodiment including an optical encoder/decoder according to one embodiment; and
FIG. 82 illustrates a rigid medical device including an identification feature.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a needle placed within the body of a patient is considered a distal end of the needle, while the needle end remaining outside the body is a proximal end of the needle. Also, the words "including," "has," and "having," as used herein, including the claims, shall have the same meaning as the word "comprising."

### I. Assisted Catheter Placement

Embodiments of the present invention are generally directed to a catheter placement system configured for accurately placing a catheter within the vasculature of a patient. In one embodiment, the catheter placement system employs at least two modalities for improving catheter placement accuracy: 1) ultrasound-assisted guidance for introducing the catheter into the patient's vasculature; and 2) a tip location/navigation system ("TLS"), or magnetically-based tracking of the catheter tip during its advancement through the tortuous vasculature path to detect and facilitate correction of any tip malposition during such advancement. The ultrasound guidance and tip location features of the present system according to one embodiment are integrated into a single device for use by a clinician placing the catheter. Integration of these two modalities into a single device simplifies the catheter placement process and results in relatively faster catheter placements. For instance, the integrated catheter placement system enables ultrasound and TLS activities to be viewed from a single display of the integrated system. Also, controls located on an ultrasound probe of the integrated device, which probe is maintained within the sterile field of the patient during catheter placement, can be used to control functionality of the system, thus precluding the need for a clinician to reach out of the sterile field in order to control the system.

In another embodiment, a third modality, *i.e.,* ECG signal-based catheter tip guidance, is included in the integrated system to enable guidance of the catheter tip to a desired position with respect to a node of the patient's heart from which the ECG signals originate. Such ECG-based positional assistance is also referred to herein as "tip confirmation."

Combination of the three modalities above according to one embodiment enables the catheter placement system to facilitate catheter placement within the patient's vasculature with a relatively high level of accuracy, *i.e.,* placement of the distal tip of the catheter in a predetermined and desired position. Moreover, because of the ECG-based guidance of the catheter tip, correct tip placement may be confirmed without the need for a confirmatory X-ray. This, in turn, reduces the patient's exposure to potentially harmful x-rays, the cost and time involved in transporting the patient to and from the x-ray department, costly and inconvenient catheter repositioning procedures, etc.

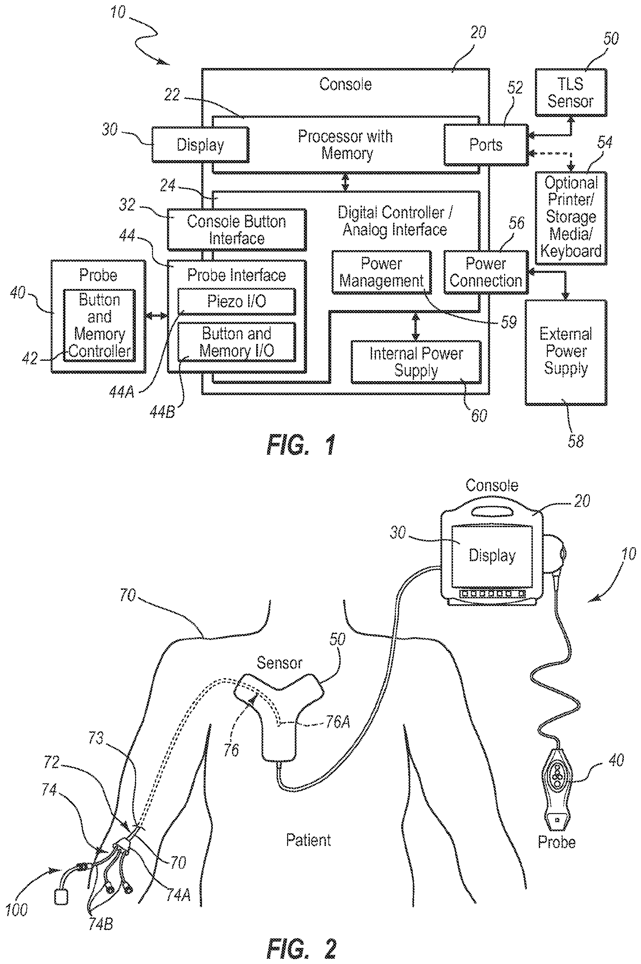

Reference is first made to FIGS. 1 and 2 which depict various components of a catheter placement system ("system"), generally designated at 10, configured in accordance with one example embodiment of the present invention. As shown, the system 10 generally includes a console 20, display 30, probe 40, and sensor 50, each of which is described in further detail below.

FIG. 2 shows the general relation of these components to a patient 70 during a procedure to place a catheter 72 into the patient vasculature through a skin insertion site 73. FIG. 2 shows that the catheter 72 generally includes a proximal portion 74 that remains exterior to the patient and a distal potion 76 that resides within the patient vasculature after placement is complete. The system 10 is employed to ultimately position a distal tip 76A of the catheter 72 in a desired position within the patient vasculature. In one embodiment, the desired position for the catheter distal tip 76A is proximate the patient's heart, such as in the lower one-third (1/3^{rd}) portion of the Superior Vena Cava ("SVC"). Of course, the system 10 can be employed to place the catheter distal tip in other locations. The catheter proximal portion 74 further includes a hub 74A that provides fluid communication between the one or more lumens of the catheter 72 and one or more extension legs 74B extending proximally from the hub.

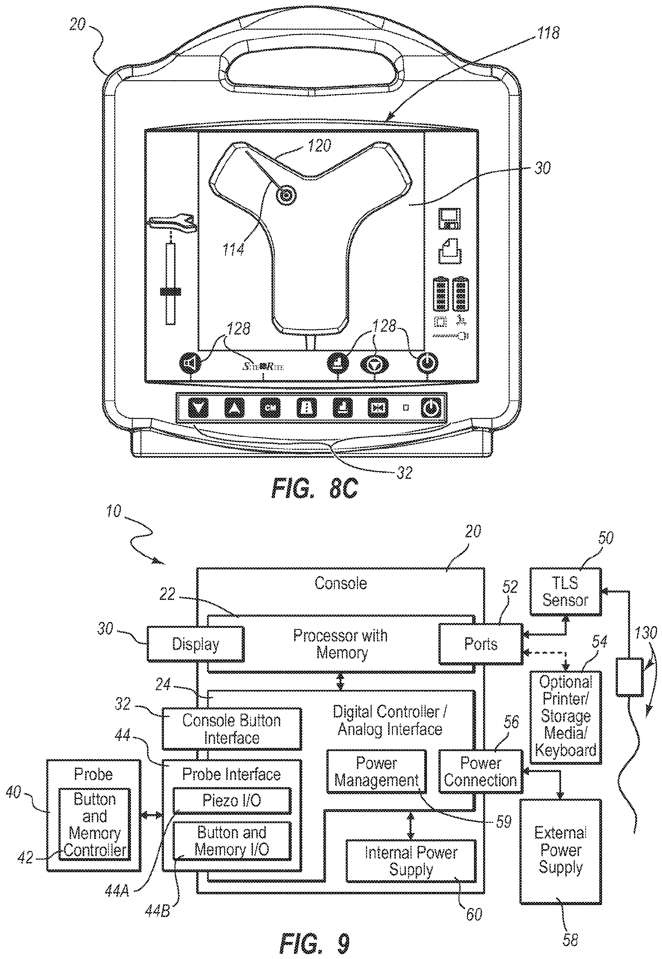

An example implementation of the console 20 is shown in FIG. 8C, though it is appreciated that the console can take one of a variety of forms. A processor 22, including non-volatile memory such as EEPROM for instance, is included in the console 20 for controlling system function during operation of the system 10, thus acting as a control processor. A digital controller/analog interface 24 is also included with the console 20 and is in communication with both the processor 22 and other system components to govern interfacing between the probe 40, sensor 50, and other system components.

The system 10 further includes ports 52 for connection with the sensor 50 and optional components 54 including a printer, storage media, keyboard, etc. The ports in one embodiment are USB ports, though other port types or a combination of port types can be used for this and the other interfaces connections described herein. A power connection 56 is included with the console 20 to enable operable connection to an external power supply 58. An internal battery 60 can also be employed, either with or exclusive of an external power supply. Power management circuitry 59 is included with the digital controller/analog interface 24 of the console to regulate power use and distribution.

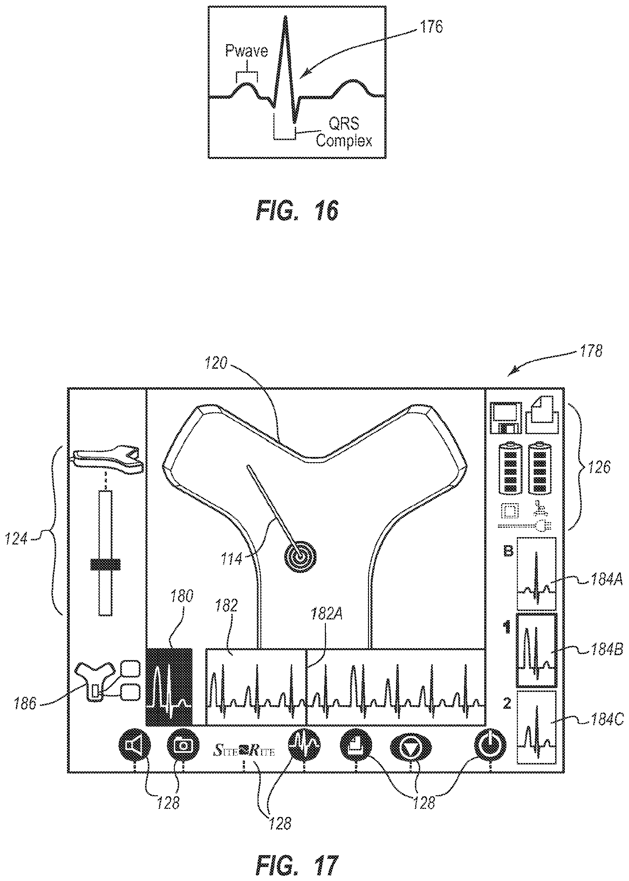

The display 30 in the present embodiment is integrated into the console 20 and is used to display information to the clinician during the catheter placement procedure. In another embodiment, the display may be separate from the console. As will be seen, the content depicted by the display 30 changes according to which mode the catheter placement system is in: US, TLS, or in other embodiments, ECG tip confirmation. In one embodiment, a console button interface 32 (*see* FIGS. 1, 8C) and buttons included on the probe 40 can be used to immediately call up a desired mode to the display 30 by the clinician to assist in the placement procedure. In one embodiment, information from multiple modes, such as TLS and ECG, may be displayed simultaneously, such as in FIG. 17. Thus, the single display 30 of the system console 20 can be employed for ultrasound guidance in accessing a patient's vasculature, TLS guidance during catheter advancement through the vasculature, and (as in later embodiments) ECG-based confirmation of catheter distal tip placement with respect to a node of the patient's heart. In one embodiment, the display 30 is an LCD device.

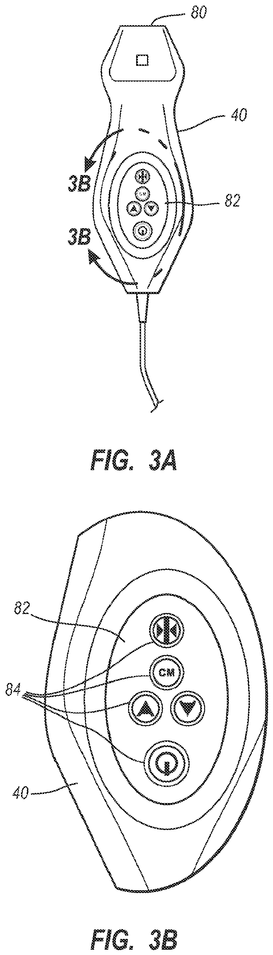

FIGS. 3A and 3B depict features of the probe 40 according to one embodiment. The probe 40 is employed in connection with the first modality mentioned above, *i.e.,* ultrasound ("US")-based visualization of a vessel, such as a vein, in preparation for insertion of the catheter 72 into the vasculature. Such visualization gives real time ultrasound guidance for introducing the catheter into the vasculature of the patient and assists in reducing complications typically associated with such introduction, including inadvertent arterial puncture, hematoma, pneumothorax, etc.

The handheld probe 40 includes a head 80 that houses a piezoelectric array for producing ultrasonic pulses and for receiving echoes thereof after reflection by the patient's body when the head is placed against the patient's skin proximate the prospective insertion site 73 (FIG. 2). The probe 40 further includes a plurality of control buttons 84, which can be included on a button pad 82. In the present embodiment, the modality of the system 10 can be controlled by the control buttons 84, thus eliminating the need for the clinician to reach out of the sterile field, which is established about the patient insertion site prior to catheter placement, to change modes via use of the console button interface 32.

As such, in one embodiment a clinician employs the first (US) modality to determine a suitable insertion site and establish vascular access, such as with a needle or introducer, then with the catheter. The clinician can then seamlessly switch, via button pushes on the probe button pad 82, to the second (TLS) modality without having to reach out of the sterile field. The TLS mode can then be used to assist in advancement of the catheter 72 through the vasculature toward an intended destination.

FIG. 1 shows that the probe 40 further includes button and memory controller 42 for governing button and probe operation. The button and memory controller 42 can include non-volatile memory, such as EEPROM, in one embodiment. The button and memory controller 42 is in operable communication with a probe interface 44 of the console 20, which includes a piezo input/output component 44A for interfacing with the probe piezoelectric array and a button and memory input/output component 44B for interfacing with the button and memory controller 42.

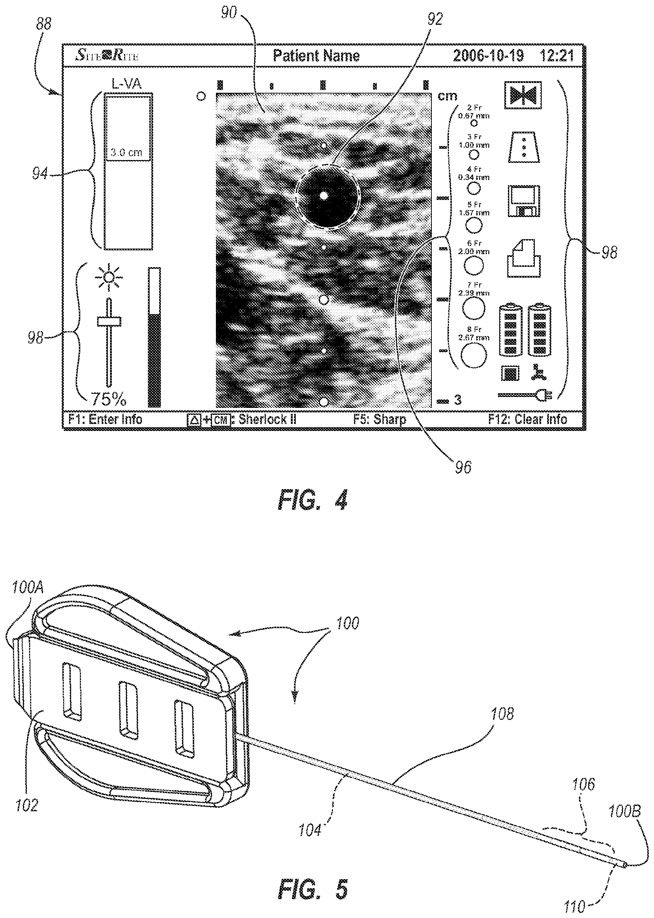

FIG. 4 shows an example screenshot 88 as depicted on the display 30 while the system 10 is in its first ultrasound modality. An image 90 of a subcutaneous region of the patient 70 is shown, depicting a cross section of a vein 92. The image 90 is produced by operation of the piezoelectric array of the probe 40. also included on the display screenshot 88 is a depth scale indicator 94, providing information regarding the depth of the image 90 below the patient's skin, a lumen size scale 96 that provides information as to the size of the vein 92 relative to standard catheter lumen sizes, and other indicia 98 that provide information regarding status of the system 10 or possible actions to be taken, *e.g.,* freeze frame, image templates, data save, image print, power status, image brightness, etc.

Note that while a vein is depicted in the image 90, other body lumens or portions can be imaged in other embodiments. Note that the US mode shown in FIG. 4 can be simultaneously depicted on the display 30 with other modes, such as the TLS mode, if desired. In addition to the visual display 30, aural information, such as beeps, tones, etc., can also be employed by the system 10 to assist the clinician during catheter placement. Moreover, the buttons included on the probe 40 and the console button interface 32 can be configured in a variety of ways, including the use of user input controls in addition to buttons, such as slide switches, toggle switches, electronic or touch-sensitive pads, etc. Additionally, both US and TLS activities can occur simultaneously or exclusively during use of the system 10.

As just described, the handheld ultrasound probe 40 is employed as part of the integrated catheter placement system 10 to enable US visualization of the peripheral vasculature of a patient in preparation for transcutaneous introduction of the catheter. In the present example embodiment, however, the probe is also employed to control functionality of the TLS portion, or second modality, of the system 10 when navigating the catheter toward its desired destination within the vasculature as described below. Again, as the probe 40 is used within the sterile field of the patient, this feature enables TLS functionality to be controlled entirely from within the sterile field. Thus the probe 40 is a dual-purpose device, enabling convenient control of both US and TLS functionality of the system 10 from the sterile field. In one embodiment, the probe can also be employed to control some or all ECG-related functionality, or third modality, of the catheter placement system 10, as described further below.

The catheter placement system 10 further includes the second modality mentioned above, *i.e.,* the magnetically-based catheter TLS, or tip location system. The TLS enables the clinician to quickly locate and confirm the position and/or orientation of the catheter 72, such as a peripherally-inserted central catheter ("PICC"), central venous catheter ("CVC"), or other suitable catheter, during initial placement into and advancement through the vasculature of the patient 70. Specifically, the TLS modality detects a magnetic field generated by a magnetic element-equipped tip location stylet, which is pre-loaded in one embodiment into a longitudinally defined lumen of the catheter 72, thus enabling the clinician to ascertain the general location and orientation of the catheter tip within the patient body. In one embodiment, the magnetic assembly can be tracked using the teachings of one or more of the following U.S. patents: 5,775,322; 5,879,297; 6,129,668; 6,216,028; and 6,263,230. The contents of the afore-mentioned U.S. patents are incorporated herein by reference in their entireties. The TLS also displays the direction in which the catheter tip is pointing, thus further assisting accurate catheter placement. The TLS further assists the clinician in determining when a malposition of the catheter tip has occurred, such as in the case where the tip has deviated from a desired venous path into another vein.

As mentioned, the TLS utilizes a stylet to enable the distal end of the catheter 72 to be tracked during its advancement through the vasculature. FIG. 5 gives an example of such a stylet 100, which includes a proximal end 100A and a distal end 100B. A handle is included at the stylet proximal end 100A, with a core wire 104 extending distally therefrom. A magnetic assembly is disposed distally of the core wire 104. The magnetic assembly includes one or more magnetic elements 106 disposed adjacent one another proximate the stylet distal end 100B and encapsulated by tubing 108. In the present embodiment, a plurality of magnetic elements 106 is included, each element including a solid, cylindrically shaped ferromagnetic stacked end-to-end with the other magnetic elements. An adhesive tip 110 can fill the distal tip of the tubing 108, distally to the magnetic elements 106.

Note that in other embodiments, the magnetic elements may vary from the design in not only shape, but also composition, number, size, magnetic type, and position in the stylet distal segment. For example, in one embodiment, the plurality of ferromagnetic magnetic elements is replaced with an electromagnetic assembly, such as an electromagnetic coil, which produces a magnetic field for detection by the sensor. Another example of an assembly usable here can be found in U.S. Patent No. 5,099,845 entitled "Medical Instrument Location Means," which is incorporated herein by reference in its entirety. Yet other examples of stylets including magnetic elements that can be employed with the TLS modality can be found in U.S. Application No. 11/466,602, filed August 23, 2006, and entitled "Stylet Apparatuses and Methods of Manufacture," which is incorporated herein by reference in its entirety. These and other variations are therefore contemplated by embodiments of the present invention. It should appreciated herein that "stylet" as used herein can include any one of a variety of devices configured for removable placement within a lumen of the catheter to assist in placing a distal end of the catheter in a desired location within the patient's vasculature.

FIG. 2 shows disposal of the stylet 100 substantially within a lumen in the catheter 72 such that the proximal portion thereof extends proximally from the catheter lumen, through the hub 74A and out through a selected one of the extension legs 74B. So disposed within a lumen of the catheter, the distal end 100B of the stylet 100 is substantially co-terminal with the distal catheter end 76A such that detection by the TLS of the stylet distal end correspondingly indicates the location of the catheter distal end.

The TLS sensor 50 is employed by the system 10 during TLS operation to detect a magnetic field produced by the magnetic elements 106 of the stylet 100. As seen in FIG. 2, the TLS sensor 50 is placed on the chest of the patient during catheter insertion. The TLS sensor 50 is placed on the chest of the patient in a predetermined location, such as through the use of external body landmarks, to enable the magnetic field of the stylet magnetic elements 106, disposed in the catheter 72 as described above, to be detected during catheter transit through the patient vasculature. Again, as the magnetic elements 106 of the stylet magnetic assembly are co-terminal with the distal end 76A of the catheter 72 (FIG. 2), detection by the TLS sensor 50 of the magnetic field of the magnetic elements provides information to the clinician as to the position and orientation of the catheter distal end during its transit.

In greater detail, the TLS sensor 50 is operably connected to the console 20 of the system 10 via one or more of the ports 52, as shown in FIG. 1. Note that other connection schemes between the TLS sensor and the system console can also be used without limitation. As just described, the magnetic elements 106 are employed in the stylet 100 to enable the position of the catheter distal end 76A (FIG. 2) to be observable relative to the TLS sensor 50 placed on the patient's chest. Detection by the TLS sensor 50 of the stylet magnetic elements 106 is graphically displayed on the display 30 of the console 20 during TLS mode. In this way, a clinician placing the catheter is able to generally determine the location of the catheter distal end 76A within the patient vasculature relative o the TLS sensor 50 and detect when catheter malposition, such as advancement of the catheter along an undesired vein, is occurring.

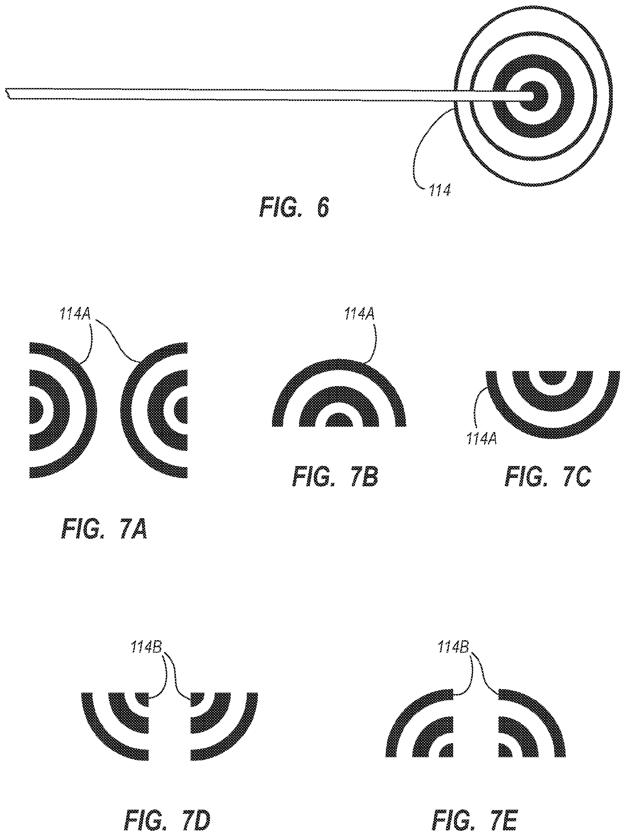

FIGS. 6 and 7A-7E show examples of icons that can be used by the console display 30 to depict detection of the stylet magnetic elements 106 by the TLS sensor 50. In particular, FIG. 6 shows an icon 114 that depicts the distal portion of the stylet 100, including the magnetic elements 106 as detected by the TLS sensor 50 when the magnetic elements are positioned under the TLS sensor. As the stylet distal end 100B is substantially co-terminal with the distal end 76A of the catheter 72, the icon indicates the position and orientation of the catheter distal end. FIGS. 7A-7E show various icons that can be depicted on the on the console display 30 when the magnetic elements 106 of the stylet 100 are not positioned directly under a portion of the TLS sensor 50, but are nonetheless detected nearby. The icons can include half-icons 114A and quarter-icons 114B that are displayed according to the position of the stylet magnetic assembly, *i.e.,* the magnetic elements 106 in the present embodiment, relative to the TLS sensor 50.

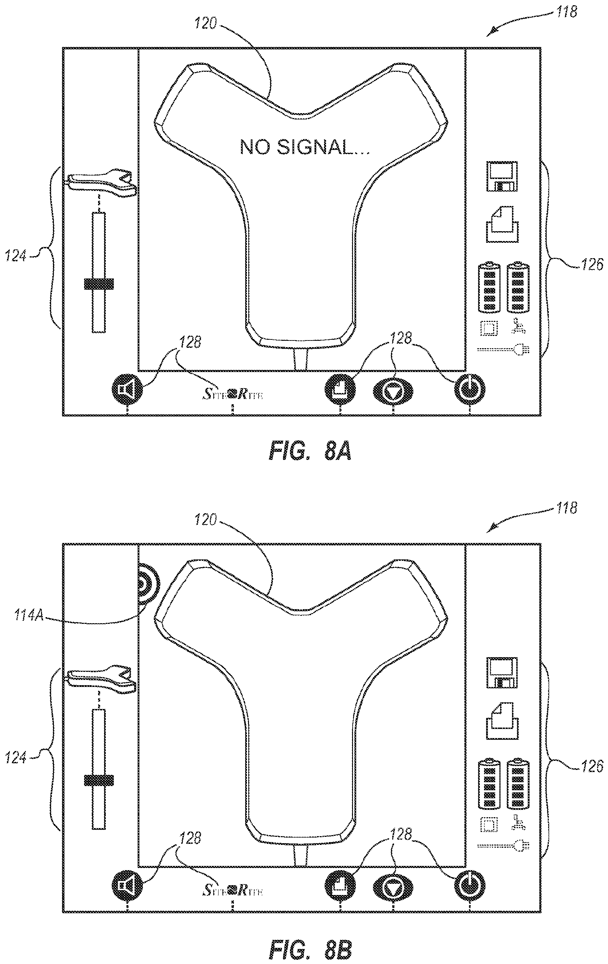

FIGS. 8A-8C depict screenshots taken from the display 30 of the system 10 while in TLS mode, showing how the magnetic assembly of the stylet 100 is depicted. The screenshot 118 of FIG. 8A shows a representative image 120 of the TLS sensor 50. Other information is provided on the display screenshot 118, including a depth scale indicator 124, status/action indicia 126, and icons 128 corresponding to the button interface 32 included on the console 20 (FIG. 8C). Though the icons 128 in the present embodiment are simply indicators to guide the user in identifying the purpose of the corresponding buttons of the button interface 32, in another embodiment the display can be made touch-sensitive so that the icons themselves can function as button interfaces and can change according to the mode the system is in.

During initial stages of catheter advancement through the patient's vasculature after insertion therein, the distal end 76A of the catheter 72, having the stylet distal end 100B substantially co-terminal therewith, is relatively distant from the TLS sensor 50. As such, the display screenshot will indicate "no signal," indicating that the magnetic field from the stylet magnetic assembly has not been detected. In FIG. 8B, the magnetic assembly proximate the stylet distal end 100B has advanced sufficiently close to the TLS sensor 50 to be detected thereby, though it is not yet under the sensor. This is indicated by the half-icon 114A shown to the left of the sensor image 120, representing the stylet magnetic assembly being positioned to the right of the TLS sensor 50 from the perspective of the patient.

In FIG. 8C, the magnetic assembly proximate the stylet distal end 100B has advanced under the TLS sensor 50 such that its position and orientation relative thereto is detected by the TLS sensor. This is indicated by the icon 114 on the sensor image 120. Note that the button icons 128 provide indications of the actions that can be performed by pressing the corresponding buttons of the console button interface 32. As such, the button icons 128 can change according to which modality the system 10 is in, thus providing flexibility of use for the button interface 32. Note further that, as the button pad 82 of the probe 40 (FIG. 3A, 3B) includes buttons 84 that mimic several of the buttons of the button interface 32, the button icons 128 on the display 30 provide a guide to the clinician for controlling the system 10 with the probe buttons 84 while remaining in the sterile field. For instance, if the clinician has need to leave TLS mode and return to US (ultrasound) mode, the appropriate control button 84 on the probe button pad 82 can be depressed, and the US mode can be immediately called up, with the display 30 refreshing to accommodate the visual information needed for US functionality, such as that shown in FIG. 4. This is accomplished without a need for the clinician to reach out of the sterile field.

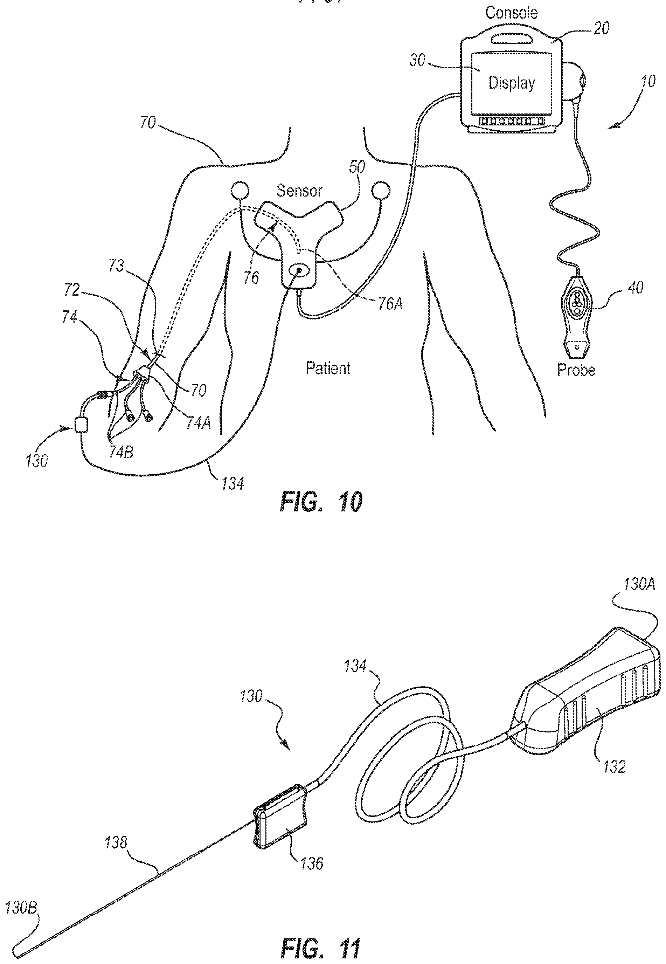

Reference is now made to FIGS. 9 and 10 in describing the integrated catheter placement system 10 according to another example embodiment. As before, the integrated system 10 includes the console 20, display 30, probe 40 for US functionality, and the TLS sensor 50 for tip location functionality as described above. Note that the system 10 depicted in FIGS. 9 and 10 is similar in many respects to the system shown in FIGS. 1 and 2. As such, only selected differences will be discussed below. The system 10 of FIGS. 9 and 10 includes additional functionality wherein determination of the proximity of the catheter distal tip 76A relative to a sino-atrial ("SA") or other electrical impulse-emitting node of the heart of the patient 70 can be determined, thus providing enhanced ability to accurately place the catheter distal tip in a desired location proximate the node. Also referred to herein as "ECG" or "ECG-based tip confirmation," this third modality of the system 10 enables detection of ECG signals from the SA node in order to place the catheter distal tip in a desired location within the patient vasculature. Note that the US, TLS, and ECG modalities are seamlessly combined in the present system 10 and can be employed in concert or individually to assist in catheter placement.

FIGS. 9 and 10 show the addition to the system 10 of a stylet 130 configured in accordance with the present embodiment. As an overview, the catheter stylet 130 is removably predisposed within the lumen of the catheter 72 being inserted into the patient 70 via the insertion site 73. The stylet 130, in addition to including a magnetic assembly for the magnetically-based TLS modality, includes an ECG sensor assembly proximate its distal end and including a portion that is co-terminal with the distal end of the catheter tip for sensing ECG signals produced by the SA node. In contrast to the previous embodiment, the stylet 130 includes a tether 134 extending from its proximal end that operably connects to the TLS sensor 50. As will be described in further detail, the stylet tether 134 permits ECG signals detected by the ECG sensor assembly included on a distal portion of the stylet 130 to be conveyed to the TLS sensor 50 during confirmation of the catheter tip location as part of the ECG signal-based tip confirmation modality. Reference and ground ECG lead/electrode pairs 158 attach to the body of the body of the patient 70 and are operably attached to the TLS sensor 50 to enable the system to filter out high level electrical activity unrelated to the electrical activity of the SA node of the heart, thus enabling the ECG-based tip confirmation functionality. Together with the reference and ground signals received from the ECG lead/electrode pairs 158 placed on the patient's skin, the ECG signals sensed by the stylet ECG sensor assembly are received by the TLS sensor 50 positioned on the patient's chest (FIG. 10). The TLS sensor 50 and/or console processor 22 can process the ECG signal data to produce an electrocardiogram waveform on the display 30, as will be described. In the case where the TLS sensor 50 processes the ECG signal data, a processor is included therein to perform the intended functionality. If the console 20 processes the ECG signal data, the processor 22, controller 24, or other processor can be utilized in the console to process the data.

Thus, as it is advanced through the patient vasculature, the catheter 72 equipped with the stylet 130 as described above can advance under the TLS sensor 50, which is positioned on the chest of the patient as shown in FIG. 10. This enables the TLS sensor 50 to detect the position of the magnetic assembly of the stylet 130, which is substantially co-terminal with the distal tip 76A of the catheter as located within the patient's vasculature. The detection by the TLS sensor 50 of the stylet magnetic assembly is depicted on the display 30 during ECG mode. The display 30 further depicts during ECG mode an ECG electrocardiogram waveform produced as a result of patient heart's electrical activity as detected by the ECG sensor assembly of the stylet 130. In greater detail, the ECG electrical activity of the SA node, including the P-wave of the waveform, is detected by the ECG sensor assembly of the stylet (described below) and forwarded to the TLS sensor 50 and console 20. The ECG electrical activity is then processed for depiction on the display 30. clinician placing the catheter can then observe the ECG data to determine optimum placement of the distal tip 76A of the catheter 72, such as proximate the SA node in one embodiment. In one embodiment, the console 20 which includes the electronic components, such as the processor 22 (FIG. 9) necessary to receive and process the signals detected by the stylet ECG sensor assembly. In another embodiment, the TLS sensor 50 can include the necessary electronic components processing the ECG signals.

As already discussed, the display 30 is used to display information to the clinician during the catheter placement procedure. The content of the display 30 changes according to which mode the catheter placement system is in: US, TLS, or ECG. Any of the three modes can be immediately called up to the display 30 by the clinician, and in some cases information from multiple modes, such as TLS and ECG, may be displayed simultaneously. In one embodiment, as before, the mode the system is in may be controlled by the control buttons 84 included on the handheld probe 40, thus eliminating the need for the clinician to reach out of the sterile field (such as touching the button interface 32 of the console 20) to change modes. Thus, in the present embodiment the probe 40 is employed to also control some or all ECG-related functionality of the system 10. Note that the button interface 32 or other input configurations can also be used to control system functionality. Also, in addition to the visual display 30, aural information, such as beeps, tones, etc., can also be employed by the system to assist the clinician during catheter placement.

Reference is now made to FIGS. 11-12E in describing various details of one embodiment of the stylet 130 that is removably loaded into the catheter 72 and employed during insertion to position the distal tip 76A of the catheter in a desired location within the patient vasculature. As shown, the stylet 130 as removed from the catheter defines a proximal end 130A and a distal end 130B. A connector 132 is included at the proximal stylet end 130A, and a tether 134 extends distally from the connector and attaches to a handle 136. A core wire 138 extends distally from the handle 136. The stylet 130 is pre-loaded within a lumen of the catheter 72 in one embodiment such that the distal end 130B is substantially flush, or co-terminal, with the catheter opening at the distal end 76A thereof (FIG. 10), and such that a proximal portion of the core wire 138, the handle 136, and the tether 134 extend proximally from a selected one of the extension tubes 74B. Note that, though described herein as a stylet, in other embodiments a guidewire or other catheter guiding apparatus could include the principles of the embodiment described herein.

The core wire 138 defines an elongate shape and is composed of a suitable stylet material including stainless steel or a memory material such as, in one embodiment, a nickel and titanium-containing alloy commonly known by the acronym "nitinol." Though not shown here, manufacture of the core wire 138 from nitinol in one embodiment enables the portion of the core wire corresponding to a distal segment of the stylet to have a pre-shaped bent configuration so as to urge the distal portion of the catheter 72 into a similar bent configuration. In other embodiments, the core wire includes no pre-shaping. Further, the nitinol construction lends torqueability to the core wire 138 to enable a distal segment of the stylet 130 to be manipulated while disposed within the lumen of the catheter 72, which in turn enables the distal portion of the catheter to be navigated through the vasculature during catheter insertion.

The handle 136 is provided to enable insertion/removal of the stylet from the catheter 72. In embodiments where the stylet core wire 138 is torqueable, the handle 136 further enables the core wire to be rotated within the lumen of the catheter 72, to assist in navigating the catheter distal portion through the vasculature of the patient 70.

The handle 136 attaches to a distal end of the tether 134. In the present embodiment, the tether 134 is a flexible, shielded cable housing one or more conductive wires electrically connected both to the core wire 138, which acts as the ECG sensor assembly referred to above, and the tether connector 132. As such, the tether 134 provides a conductive pathway from the distal portion of the core wire 138 through to the tether connector 132 at proximal end 130A of the stylet 130. As will be explained, the tether connector 132 is configured for operable connection to the TLS sensor 50 on the patient's chest for assisting in navigation of the catheter distal tip 76A to a desired location within the patient vasculature.

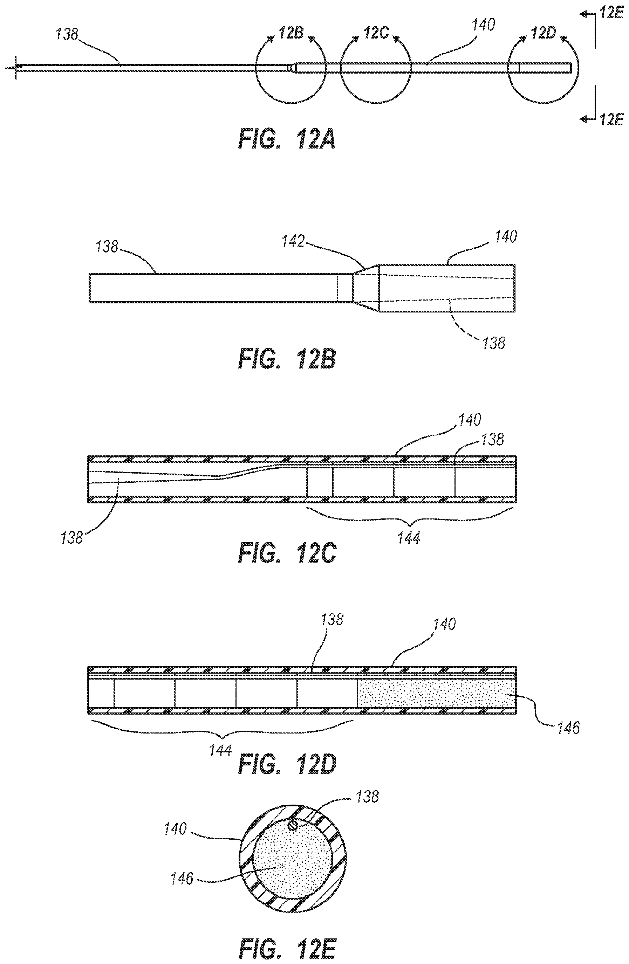

As seen in FIGS. 12B-12D, a distal portion of the core wire 138 is gradually tapered, or reduced in diameter, distally from a junction point 142. A sleeve 140 is slid over the reduced-diameter core wire portion. Though of relatively greater diameter here, the sleeve in another embodiment can be sized to substantially match the diameter of the proximal portion of the stylet core wire. The stylet 130 further includes a magnetic assembly disposed proximate the distal end 130B thereof for use during TLS mode. The magnetic assembly in the illustrated embodiment includes a plurality of magnetic elements 144 interposed between an outer surface of the reduced-diameter core wire 138 and an inner surface of the sleeve 140 proximate the stylet distal end 130B. In the present embodiment, the magnetic elements 144 include 20 ferromagnetic magnets of a solid cylindrical shape stacked end-to-end in a manner similar to the stylet 100 of FIG. 2. In other embodiments, however, the magnetic element(s) may vary from this design in not only shape, but also composition, number, size, magnetic type, and position in the stylet. For example, in one embodiment the plurality of magnets of the magnetic assembly is replaced with an electromagnetic coil that produces a magnetic field for detection by the TLS sensor. These and other variations are therefore contemplated by embodiments of the present invention.

The magnetic elements 144 are employed in the stylet 130 distal portion to enable the position of the stylet distal end 130B to be observable relative to the TLS sensor 50 placed on the patient's chest. As has been mentioned, the TLS sensor 50 is configured to detect the magnetic field of the magnetic elements 144 as the stylet advances with the catheter 72 through the patient vasculature. In this way, a clinician placing the catheter 72 is able to generally determine the location of the catheter distal end 76A within the patient vasculature and detect when catheter malposition is occurring, such as advancement of the catheter along an undesired vein, for instance.

The stylet 130 further includes the afore-mentioned ECG sensor assembly, according to one embodiment. The ECG sensor assembly enables the stylet 130, disposed in a lumen of the catheter 72 during insertion, to be employed in detecting an intra-atrial ECG signal produced by an SA or other node of the patient's heart, thereby allowing for navigation of the distal tip 76A of the catheter 72 to a predetermined location within the vasculature proximate the patient's heart. Thus, the ECG sensor assembly serves as an aide in confirming proper placement of the catheter distal tip 76A.

In the embodiment illustrated in FIGS. 11-12E, the ECG sensor assembly includes a distal portion of the core wire 138 disposed proximate the stylet distal end 130B. The core wire 138, being electrically conductive, enables ECG signals to be detected by the distal end thereof and transmitted proximally along the core wire. A conductive material 146, such as a conductive epoxy, fills a distal portion of the sleeve 140 adjacent the distal termination of the core wire 138 so as to be in conductive communication with the distal end of the core wire. This in turn increases the conductive surface of the distal end 130B of the stylet 130 so as to improve its ability to detect ECG signals.

Before catheter placement, the stylet 130 is loaded into a lumen of the catheter 72. Note that the stylet 130 can come preloaded in the catheter lumen from the manufacturer, or loaded into the catheter by the clinician prior to catheter insertion. The stylet 130 is disposed within the catheter lumen such that the distal end 130B of the stylet 130 is substantially co-terminal with the distal tip 76A of the catheter 72, thus placing the distal tips of both the stylet and the catheter in substantial alignment with one another. The co-terminality of the catheter 72 and stylet 130 enables the magnetic assembly to function with the TLS sensor 50 in TLS mode to track the position of the catheter distal tip 76A as it advances within the patient vasculature, as has been described. Note, however, that for the tip confirmation functionality of the system 10, the distal end 130B of the stylet 130 need not be co-terminal with the catheter distal end 76A. Rather, all that is required is that a conductive path between the vasculature and the ECG sensor assembly, in this case the core wire 138, be established such that electrical impulses of the SA node or other node of the patient's heart can be detected. This conductive path in one embodiment can include various components including saline solution, blood, etc.

In one embodiment, once the catheter 72 has been introduced into the patient vasculature via the insertion site 73 (FIG. 10) the TLS mode of the system 10 can be employed as already described to advance the catheter distal tip 76A toward its intended destination proximate the SA node. Upon approaching the region of the heart, the system 10 can be switched to ECG mode to enable ECG signals emitted by the SA node to be detected. As the stylet-loaded catheter is advanced toward the patient's heart, the electrically conductive ECG sensor assembly, including the distal end of the core wire 138 and the conductive material 146, begins to detect the electrical impulses produced by the SA node. As such, the ECG sensor assembly serves as an electrode for detecting the ECG signals. The elongate core wire 138 proximal to the core wire distal end serves as a conductive pathway to convey the electrical impulses produced by the SA node and received by the ECG sensor assembly to the tether 134.

The tether 134 conveys the ECG signals to the TLS sensor 50 temporarily placed on the patient's chest. The tether 134 is operably connected to the TLS sensor 50 via the tether connector 132 or other suitable direct or indirect connective configuration. As described, the ECG signal can then be process and depicted on the system display 30 (FIG. 9, 10). Monitoring of the ECG signal received by the TLS sensor 50 and displayed by the display 30 enables a clinician to observe and analyze changes in the signal as the catheter distal tip 76A advances toward the SA node. When the received ECG signal matches a desired profile, the clinician can determine that the catheter distal tip 76A has reached a desired position with respect to the SA node. As mentioned, in one embodiment this desired position lies within the lower one-third (1/3_{rd}) portion of the SVC.

The ECG sensor assembly and magnetic assembly can work in concert in assisting a clinician in placing a catheter within the vasculature. Generally, the magnetic assembly of the stylet 130 assists the clinician in generally navigating the vasculature from initial catheter insertion so as to place the distal end 76A of the catheter 72 in the general region of the patient's heart. The ECG sensor assembly can then be employed to guide the catheter distal end 76A to the desired location within the SVC by enabling the clinician to observe changes in the ECG signals produced by the heart as the stylet ECG sensor assembly approaches the SA node. Again, once a suitable ECG signal profile is observed, the clinician can determine that the distal ends of both the stylet 130 and the catheter 72 have arrived at the desired location with respect to the patient's heart. Once it has been positioned as desired, the catheter 72 may be secured in place and the stylet 130 removed from the catheter lumen. It is noted here that the stylet may include one of a variety of configurations in addition to what is explicitly described herein. In one embodiment, the stylet can attach directly to the console instead of an indirect attachment via the TLS sensor. In another embodiment, the structure of the stylet 130 that enables its TLS and ECG-related functionalities can be integrated into the catheter structure itself. For instance, the magnetic assembly and/or ECG sensor assembly can, in one embodiment, be incorporated into the wall of the catheter.

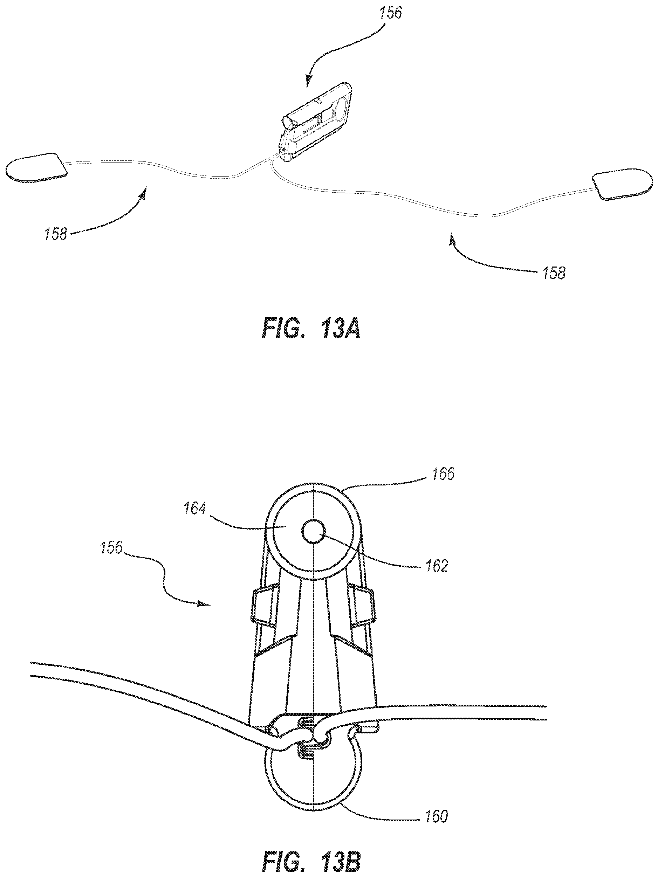

FIGS. 13A-15 describe various details relating to the passage of ECG signal data from the stylet tether 134 to the TLS sensor 50 positioned on the patient's chest, according the present embodiment. In particular, this embodiment is concerned with passage of ECG signal data from a sterile field surrounding the catheter 72 and insertion site 73, which includes the stylet 130 and tether 134, and a non-sterile field, such as the patient's chest on which the TLS sensor is positioned. Such passage should not disrupt the sterile field so that the sterility thereof is compromised. A sterile drape that is positioned over the patient 70 during the catheter insertion procedure defines the majority of the sterile field: areas above the drape are sterile, while areas below (excluding the insertion site and immediately surrounding region) are non-sterile. As will be seen, the discussion below includes at least a first communication node associated with the stylet 130, and a second communication node associated with the TLS sensor 50 that operably connect with one another to enable ECG signal data transfer therebetween.

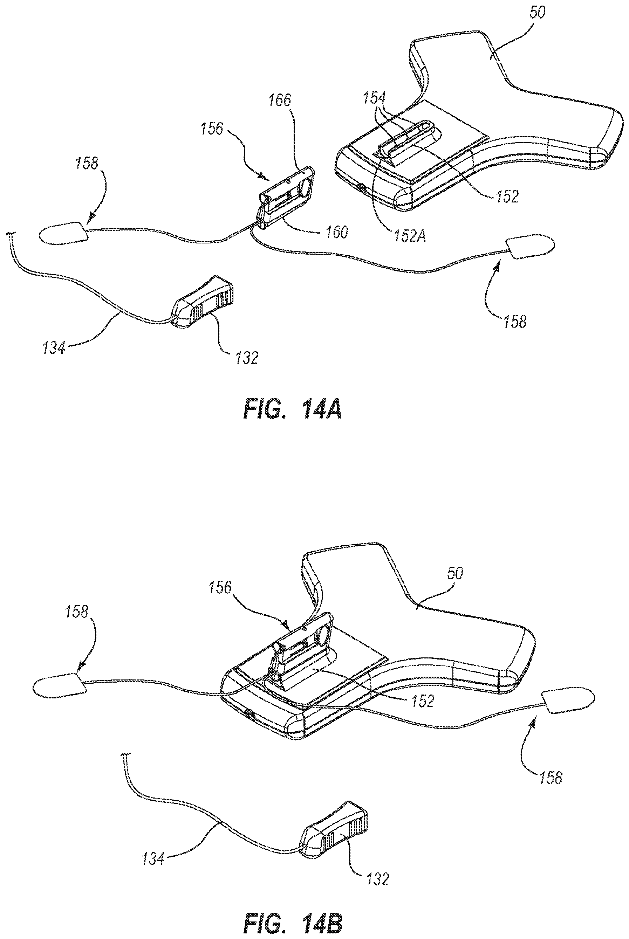

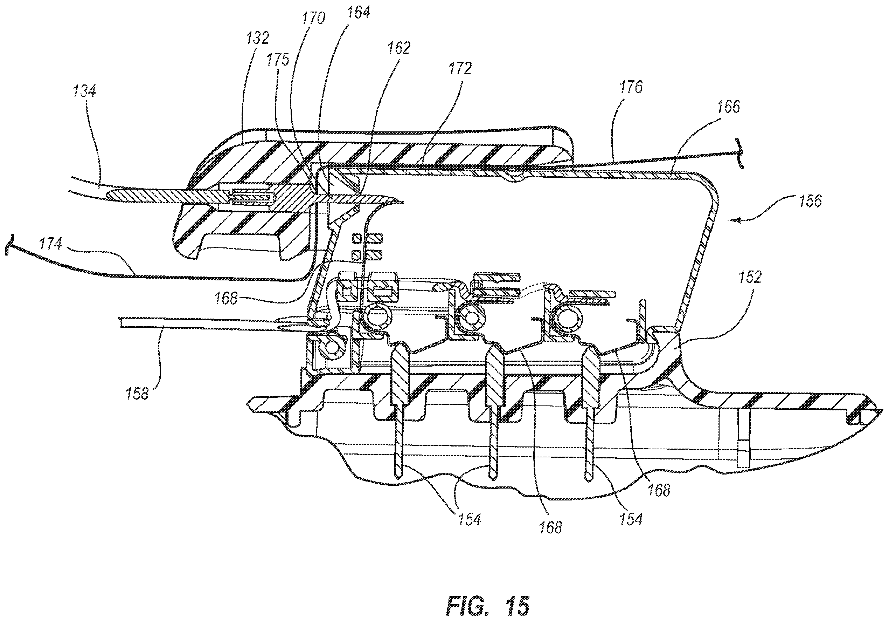

One embodiment addressing the passage of ECG signal data from the sterile field to the non-sterile field without compromising the sterility of the former is depicted in FIGS. 13A-15, which depict a "through-drape" implementation also referred to as a "shark fin" implementation. In particular, FIG. 14A shows the TLS sensor 50 as described above for placement on the chest of the patient during a catheter insertion procedure. The TLS sensor 50 includes on a top surface thereof a connector base 152 defining a channel 152A in which are disposed three electrical base contacts 154. A fin connector 156, also shown in FIGS. 13A-13D, is sized to be slidingly received by the channel 152A of the connector base 152, as shown in FIG. 14B and 15. Two ECG lead/electrode pairs 158 extend from the fin connector 156 for placement on the shoulder and torso or other suitable external locations on the patient body. The drape-piercing tether connector 132 is configured to slidingly mate with a portion of the fin connector 156, as will be described further below, to complete a conductive pathway from the stylet 120, through the sterile field to the TLS sensor 50.

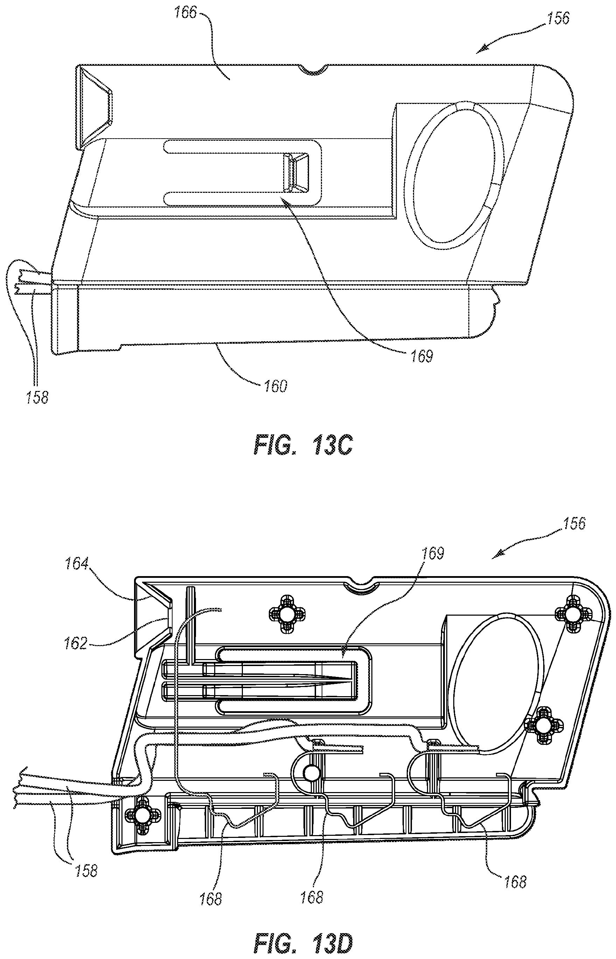

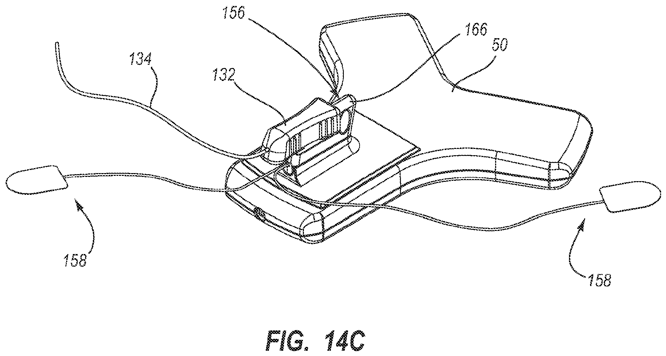

FIGS. 13A-13D show further aspects of the fin connector 156. In particular, the fin connector 156 defines a lower barrel portion 160 that is sized to be received in the channel 152A of the connector base 152 (FIGS. 14B, 15). A hole 162 surrounded by a centering cone 164 is included on a back end of an upper barrel portion 166. The upper barrel portion 166 is sized to receive the tether connector 132 of the stylet 130 (FIGS. 14C, 15) such that a pin contact 170 extending into a channel 172 of the tether connector 132 (FIG. 15) is guided by the centering hole until it seats within the hole 162 of the fin connector 156, thus interconnecting the tether connector with the fin connector. An engagement feature, such as the engagement feature 169 shown in FIGS. 13C and 13D, can be included on the fin connector 156 to engage with a corresponding feature on the tether connector 132 to assist with maintaining a mating between the two components.

FIG. 13D shows that the fin connector 156 includes a plurality of electrical contacts 168. In the present embodiment, three contacts 168 are included: the two forward-most contact each electrically connecting with a terminal end of one of the ECG leads 158, and the rear contact extending into axial proximity of the hole 162 so as to electrically connect with the pin contact 170 of the tether connector 132 when the latter is mated with the fin connector 156 (FIG. 15). A bottom portion of each contact 168 of the fin connector 156 is positioned to electrically connect with a corresponding one of the base contacts 154 of the TLS sensor connector base 152.

FIG. 14B shows a first connection stage, wherein the fin connector 156 is removably mated with the TLS sensor connector base 152 by the sliding engagement of the lower barrel portion 160 of the fin connector with the connector base channel 152A. This engagement electrically connects the connector base contacts 154 with the corresponding fin contacts 168.

FIG. 14C shows a second connection stage, wherein the tether connector 132 is removably mated with the fin connector 156 by the sliding engagement of the tether connector channel 172 with the upper barrel portion 166 of the fin connector. This engagement electrically connects the tether connector pin contact 170 with the back contact 168 of the fin connector 156, as best seen in FIG. 15. In the present embodiment, the horizontal sliding movement of the tether connector 132 with respect to the fin connector 156 is in the same engagement direction as when the fin connector is slidably mated to the sensor connector base channel 152A (FIG. 14B). In one embodiment, one or both of the stylet 130/tether connector 132 and the fin connector 156 are disposable. Also, the tether connector in one embodiment can be mated to the fin connector after the fin connector has been mated to the TLS sensor, while in another embodiment the tether connector can be first mated to the fin connector through the surgical drape before the fin connector is mated to the TLS sensor.

In the connection scheme shown in FIG. 14C, the stylet 130 is operably connected to the TLS sensor 50 via the tether connector 132, thus enabling the ECG sensor assembly of the stylet to communicate ECG signals to the TLS sensor. In addition, the ECG lead/electrode pairs 158 are operably connected to the TLS sensor 50. In one embodiment, therefore, the tether connector 132 is referred to as a first communication node for the stylet 130, while the fin connector 156 is referred to as a second communication node for the TLS sensor 50.

Note that various other connective schemes and structures can be employed to establish operable communication between the stylet and the TLS sensor. For instance, the tether connector can use a slicing contact instead of a pin contact to pierce the drape. Or, the fin connector can be integrally formed with the TLS sensor. These and other configurations are therefore embraced within the scope of embodiments of the present disclosure.

As seen in FIG. 15, a sterile drape 174 used during catheter placement to establish a sterile field is interposed between the interconnection of the tether connector 132 with the fin connector 156. As just described, the tether connector 132 includes the pin contact 170 that is configured to pierce the drape 174 when the two components are mated. This piercing forms a small hole, or perforation 175, in the sterile drape 174 that is occupied by the pin contact 170, thus minimizing the size of the drape perforation by the pin contact. Moreover, the fit between the tether connector 132 and the fin connector 156 is such that the perforation in sterile drape made by piercing of the pin contact 170 is enclosed by the tether connector channel 172, thus preserving the sterility of the drape and preventing a breach in the drape that could compromise the sterile field established thereby. The tether connector channel 172 is configured so as to fold the sterile drape 174 down prior to piercing by the pin contact 170 such that the pin contact does not pierce the drape until it is disposed proximate the hole 162 of the fin connector 156. It is noted here that the tether connector 132 and fin connector 156 are configured so as to facilitate alignment therebetween blindly through the opaque sterile drape 174, *i.e.,* via palpation absent visualization by the clinician of both components.

Note further that the fin contacts 168 of the fin connector 156 as shown in FIG. 15 are configured to mate with the sensor base contacts 154 in such a way as to assist in retaining the fin connector in engagement with the sensor base channel 152A. This in turn reduces the need for additional apparatus to secure the fin connector 156 to the TLS sensor 50.

FIG. 16 shows a typical ECG waveform 176, including a P-wave and a QRS complex. Generally, the amplitude of the P-wave varies as a function of distance of the ECG sensor assembly from the SA node, which produces the waveform 176. A clinician can use this relationship in determining when the catheter tip is properly positioned proximate the heart. For instance, in one implementation the catheter tip is desirably placed within the lower one-third (1/3^{rd}) of the superior vena cava, as has been discussed. The ECG data detected by the ECG sensor assembly of the stylet 130 is used to reproduce waveforms such as the waveform 176, for depiction on the display 30 of the system 10 during ECG mode.

Reference is now made to FIG. 17 in describing display aspects of ECG signal data on the display 30 when the system 10 is in ECG mode, the third modality described further above, according to one embodiment. The screenshot 178 of the display 30 includes elements of the TLS modality, including a representative image 120 of the TLS sensor 50, and can the icon 114 corresponding to the position of the distal end of the stylet 130 during transit through the patient vasculature. The screenshot 178 further includes a window 180 in which the current ECG waveform captured by the ECG sensor assembly of the stylet 130 and processed by the system 10 is displayed. The window 180 is continually refreshed as new waveforms are detected.

Window 182 includes a successive depiction of the most recent detected ECG waveforms, and includes a refresh bar 182A, which moves laterally to refresh the waveforms as they are detected. Window 184A is used to display a baseline ECG waveform, captured before the ECG sensor assembly is brought into proximity with the SA node, for comparison purposes to assist the clinician in determining when the desired catheter tip location has been achieved. Windows 184B and 184C can be filed by user-selected detected ECG waveforms when the user pushes a predetermined button on the probe 40 or the console button interface 32. The waveforms in the windows 184B and 184C remain until overwritten by new waveforms as a result of user selection via button pushes or other input. As in previous modes, the depth scale 124, status/action indicia 126, and button icons 128 are included on the display 30. An integrity indicator 186 is also included on the display 30 to give an indication of whether the ECG lead/electrode pairs 158 are operably connected to the TLS sensor 50.

As seen above, therefore, the display 30 depicts in one embodiment elements of both the TLS and ECG modalities simultaneously on a single screen, thus offering the clinician ample data to assist in placing the catheter distal tip in a desired position. Note further that in one embodiment a printout of the screenshot or selected ECG or TLS data can be saved, printed, or otherwise preserved by the system 10 to enable documentation of proper catheter placement.

Although the embodiments described herein relate to a particular configuration of a catheter, such as a PICC or CVC, such embodiments are merely exemplary. Accordingly, the principles of the present invention can be extended to catheters of many different configurations and designs.

### II. Assisted Guidance for Needle/Medical Component

Embodiments of the present invention described herein are generally directed to a guidance system for locating and guiding a needle or other medical component during ultrasound-based or other suitable procedures for accessing with the needle a subcutaneous vessel of a patient, for instance. In one embodiment, the guidance system enables the position, orientation, and advancement of the needle to be superimposed in real-time atop the ultrasound image of the vessel, thus enabling a clinician to accurately guide the needle to the intended target. Furthermore, in one embodiment, the guidance system tracks the needle's position in five degrees of motion: x, y, and z spatial coordinate space, needle pitch, and needle yaw. Such tracking enables the needle to be guided and placed with relatively high accuracy.

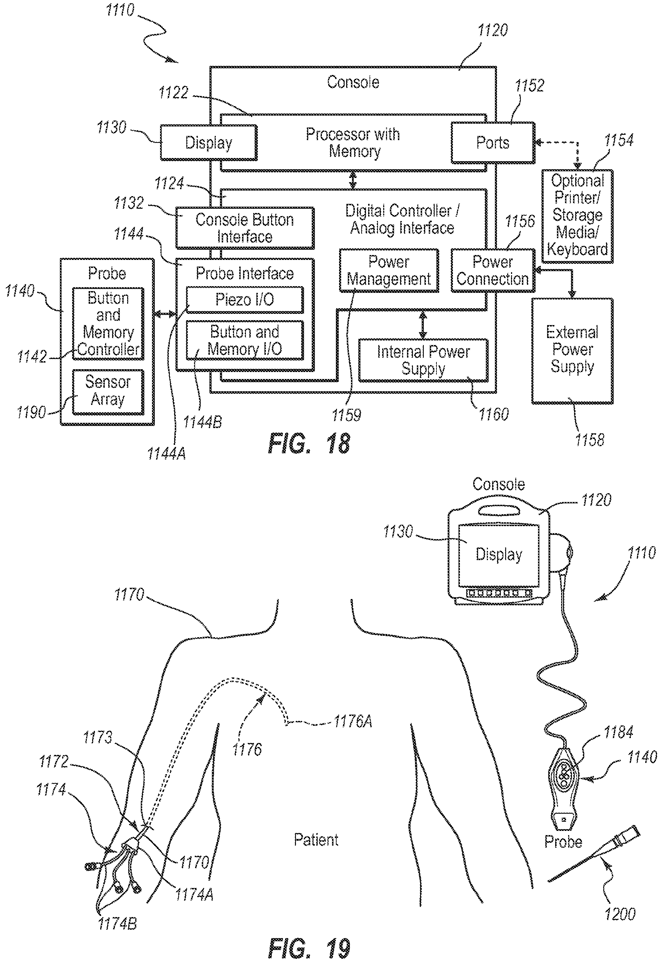

Reference is first made to FIGS. 18 and 19, which depict various components of an ultrasound-based needle guidance system ("system"), generally designated at 1110, configured in accordance with one embodiment of the present invention. As shown, the system 1110 generally includes an ultrasound ("US") imaging portion including a console 1120, display 1130, and probe 1140, each of which is described in further detail below. Note that the system 1110 bears similarity to the system 10 shown in FIG. 1 with respect to some components, in one embodiment. It should be noted, however, that the ultrasound imaging portion can be configured in one of a variety of ways in addition to what is shown and described herein.

The ultrasound imaging portion of the system 1110 is employed to image a targeted internal portion of a body of a patient prior to percutaneous insertion of a needle or other device to access the target. As described below, in one embodiment insertion of the needle is performed prior to the subsequent insertion of a catheter into a vein or other portion of the vasculature of the patient. It is appreciated, however, that insertion of a needle into the body of a patient can be performed for a variety of medical purposes.

FIG. 19 shows the general relation of the above-described components to a patient 1170 during a procedure to ultimately place a catheter 1172 into the patient vasculature through a skin insertion site 1173, according to one embodiment. FIG. 19 shows that the catheter 1172 generally includes a proximal portion 1174 that remains exterior to the patient and a distal potion 1176 that resides within the patient vasculature after placement is complete. The system 1110 is employed to ultimately position a distal tip 1176A of the catheter 1172 in a desired position within the patient vasculature. In one embodiment, the desired position for the catheter distal tip 1176A is proximate the patient's heart, such as in the lower one-third (1/3^{rd}) portion of the Superior Vena Cava ("SVC"). Of course, the system 1110 can be employed to place the catheter distal tip in other locations.

The catheter proximal portion 1174 further includes a hub 1174A that provides fluid communication between the one or more lumens of the catheter 1172 and one or more extension legs 1174B extending proximally from the hub. As mentioned, placement of a needle into the patient vasculature at the insertion site 1173 is typically performed prior to insertion of the catheter, though it is appreciated that other placement methods can be employed. Further, it is appreciated that the above discussion is only one example for use of the system 1110; indeed it can be employed for a variety of uses, such as the placement of needles preparatory to insertion of a catheter as above, the insertion of a needle for other uses, or for the insertion of other medical components into the body of a patient, including x-ray or ultrasound markers, biopsy sheaths, ablation components, bladder scanning components, vena cava filters, etc.

In greater detail, the console 1120 houses a variety of components of the system 1110 and it is appreciated that the console can take one of a variety of forms. A processor 1122, including non-volatile memory such as EEPROM for instance, is included in the console 1120 for controlling system function and executing various algorithms during operation of the system 1110, thus acting as a control processor. A digital controller/analog interface 1124 is also included with the console 1120 and is in communication with both the processor 1122 and other system components to govern interfacing between the probe 1140 and other system components.

The system 1110 further includes ports 1152 for connection with additional components such as optional components 1154 including a printer, storage media, keyboard, etc. The ports in one embodiment are USB ports, though other port types or a combination of port types can be used for this and the other interfaces connections described herein. A power connection 1156 is included with the console 1120 to enable operable connection to an external power supply 1158. An internal battery 1160 can also be employed, either with or exclusive of an external power supply. Power management circuitry 1159 is included with the digital controller/analog interface 1124 of the console to regulate power use and distribution.

The display 1130 in the present embodiment is integrated into the console 1120 and is used to display information to the clinician during the placement procedure, such as an ultrasound image of the targeted internal body portion attained by the probe 1140. In another embodiment, the display may be separate from the console. In one embodiment, a console button interface 1132 and control buttons 1184 (FIG. 19) included on the probe 1140 can be used to immediately call up a desired mode to the display 1130 by the clinician to assist in the placement procedure. In one embodiment, the display 1130 is an LCD device.

FIG. 19 further depicts a needle 1200 used to gain initial access to the patient vasculature via the insertion site 1173. As will be described in further detail below, the needle 1200 is configured to cooperate with the system 1110 in enabling the system to detect the position, orientation, and advancement of the needle during an ultrasound-based placement procedure.

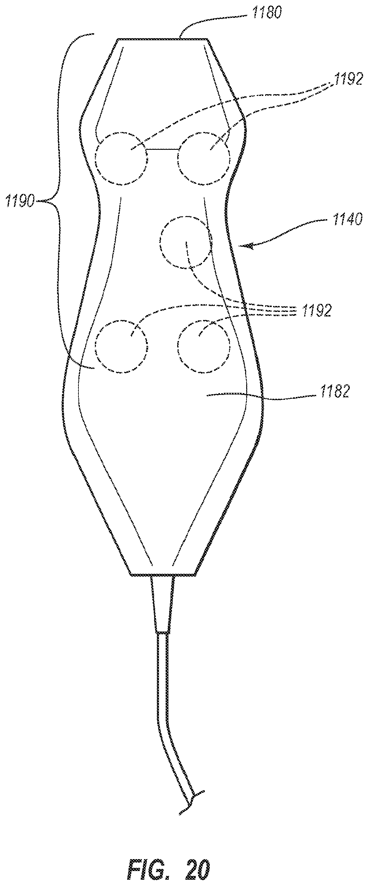

FIG. 20 depicts features of the probe 1140 according to one embodiment. The probe 1140 is employed in connection with ultrasound-based visualization of a vessel, such as a vein, in preparation for insertion of the needle 1200 and/or catheter 1172 into the vasculature. Such visualization gives real time ultrasound guidance and assists in reducing complications typically associated with such introduction, including inadvertent arterial puncture, hematoma, pneumothorax, etc.

The handheld probe 1140 includes a head 1180 that houses a piezoelectric array for producing ultrasonic pulses and for receiving echoes thereof after reflection by the patient's body when the head is placed against the patient's skin proximate the prospective insertion site 1173 (FIG. 19). The probe 1140 further includes a plurality of control buttons 1184 (FIG. 19) for controlling the system, thus eliminating the need for the clinician to reach out of the sterile field, which is established about the patient insertion site prior to establishment of the insertion site, to control the system 1110.

As such, in one embodiment a clinician employs the ultrasound imaging portion of the system 1110 to determine a suitable insertion site and establish vascular access, such as with the needle 1200, prior to introduction of the catheter 1172 for ultimate advancement thereof through the vasculature toward an intended destination.

FIG. 18 shows that the probe 1140 further includes a button and memory controller 1142 for governing button and probe operation. The button and memory controller 1142 can include non-volatile memory, such as EEPROM, in one embodiment. The button and memory controller 1142 is in operable communication with a probe interface 1144 of the console 1120, which includes a piezo input/output component 1144A for interfacing with the probe piezoelectric array and a button and memory input/output component 1144B for interfacing with the button and memory controller 1142.

As seen in FIG. 20, the probe 1140 includes a sensor array 1190 for detecting the position, orientation, and movement of the needle 1200 during ultrasound imaging procedures, such as those described above. As will be described in further detail below, the sensor array includes a plurality of magnetic sensors 1192 embedded within the housing of the probe. The sensors 1192 are configured to detect a magnetic field associated with the needle 1200 and enable the system 1110 to track the needle. Though configured here as magnetic sensors, it is appreciated that the sensors 1192 can be sensors of other types and configurations, as will be described. Also, though they are shown in FIG. 20 as included with the probe 1140, the sensors 1192 of the sensor array 1190 can be included in a component separate from the probe, such as a separate handheld device. In the present embodiment, the sensors 1192 are disposed in a planar configuration below a top face 1182 of the probe 1140, though it is appreciated that the sensors can be arranged in other configurations, such as in an arched or semi-circular arrangement.

In the present embodiment, each of the sensors 1192 includes three orthogonal sensor coils for enabling detection of a magnetic field in three spatial dimensions. Such three dimensional ("3-D") magnetic sensors can be purchased, for example, from Honeywell Sensing and Control of Morristown, NJ. Further, the sensors 1192 of the present embodiment are configured as Hall-effect sensors, though other types of magnetic sensors could be employed. Further, instead of 3-D sensors, a plurality of one dimensional magnetic sensors can be included and arranged as desired to achieve 1-, 2-, or 3-D detection capability.

In the present embodiment, five sensors 1192 are included in the sensor array 1190 so as to enable detection of the needle 1200 in not only the three spatial dimensions (*i.e.,* X, Y, Z coordinate space), but also the pitch and yaw orientation of the needle itself. Note that in one embodiment, orthogonal sensing components of two or more of the sensors 1192 enable the pitch and yaw attitude of the magnetic element 1210, and thus the needle 1200, to be determined.

In other embodiments, fewer or more sensors can be employed in the sensor array. More generally, it is appreciated that the number, size, type, and placement of the sensors of the sensor array can vary from what is explicitly shown here.

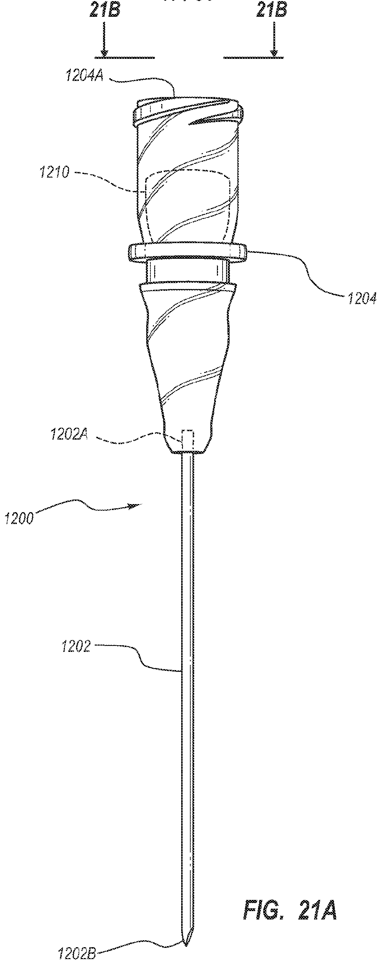

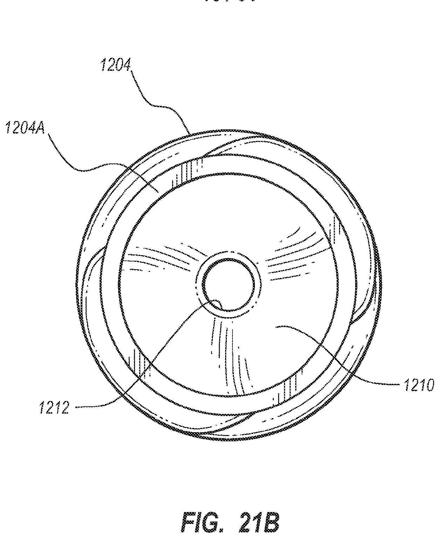

FIGS. 21A and 21B show details of one example of the needle 1200 that can be used in connection with the guidance system 1110 in accessing a targeted internal body portion of the patient, as shown in FIG. 19, according to one embodiment. In particular, the needle 1200 includes a hollow cannula 1202, which defines a proximal end 1202A and a distal end 1202B. A hub 1204 is attached to the proximal end 1202A of the cannula 1202 and includes an open end 1204A that is configured as a connector for connecting with various devices, in the present embodiment. Indeed, the open end 1204A of the hub 1204 is in communication with the hollow cannula 1202 such that a guide wire, stylet, or other component may be passed through the hub into the cannula.

As shown in FIGS. 21A and 21B, a magnetic element 1210 is included with the hub 1204. As best seen in FIG. 21B, the magnetic element 1210 in the present embodiment is a permanent magnet, including a ferromagnetic substance for instance, and is ring-shaped so as to define hole 1212 that is aligned with the hollow cannula 1202. So configured, the magnetic element 1210 produces a magnetic field that is detectable by the sensor array 1190 of the ultrasound probe 1140 so as to enable the location, orientation, and movement of the needle 1200 to be tracked by the system 1110, as described further below.

In other embodiments, it is appreciated that many other types, numbers, and sizes of magnetic elements can be employed with the needle 1200 or other medical component to enable tracking thereof by the present guidance system.

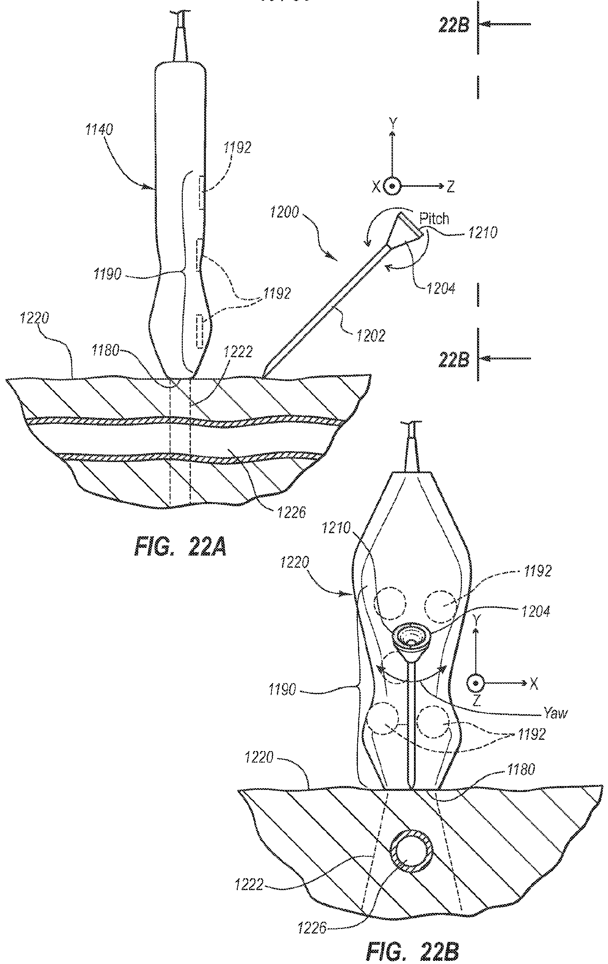

Reference is now made to FIGS. 22A and 22B, which show the ultrasound probe 1140 of the system 1110 and the needle 1200 in position and ready for insertion thereof through a skin surface 1220 of a patient to access a targeted internal body portion. In particular, the probe 1140 is shown with its head 1180 placed against the patient skin and producing an ultrasound beam 1222 so as to ultrasonically image a portion of a vessel 1226 beneath the patient skin surface 1220. The ultrasonic image of the vessel 1226 can be depicted on the display 1130 of the system 1110 (FIG. 19).

As mentioned above, the system 1110 in the present embodiment is configured to detect the position, orientation, and movement of the needle 1200 described above. In particular, the sensor array 1190 of the probe 1140 is configured to detect a magnetic field of the magnetic element 1210 included with the needle 1200. Each of the sensors 1192 of the sensor array 1190 is configured to spatially detect the magnetic element 1210 in three dimensional space. Thus during operation of the system 1110, magnetic field strength data of the needle's magnetic element 1210 sensed by each of the sensors 1192 is forwarded to a processor, such as the processor 1122 of the console 1120 (FIG. 18), which computes in real-time the position and/or orientation of the magnetic element 1210.

Specifically, and as shown in FIGS. 22A and 22B, the position of the magnetic element 1210 in X, Y, and Z coordinate space with respect to the sensor array 1190 can be determined by the system 1110 using the magnetic field strength data sensed by the sensors 1192. Moreover, FIG. 22A shows that the pitch of the magnetic element 1210 can also be determined, while FIG. 22B shows that the yaw of the magnetic element can be determined. Suitable circuitry of the probe 1140, the console 1120, or other component of the system can provide the calculations necessary for such position/orientation. In one embodiment, the magnetic element 210 can be tracked using the teachings of one or more of the following U.S. patents: 5,775,322; 5,879,297; 6,129,668; 6,216,028; and 6,263,230. The contents of the afore-mentioned U.S. patents are incorporated herein by reference in their entireties.

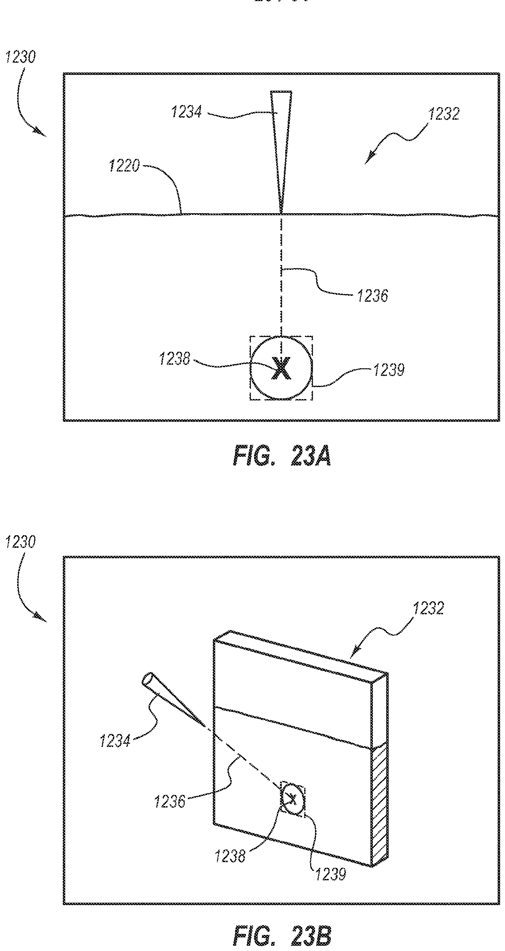

The above position and orientation information determined by the system 1110, together with the length of the cannula 1202 and position of the magnetic element 1210 with respect to the distal needle tip as known by or input into the system, enable the system to accurately determine the location and orientation of the entire length of the needle 1200 with respect to the sensor array 1190. Optionally, the distance between the magnetic element 1210 and the distal needle tip is known by or input into the system 1110. This in turn enables the system 1110 to superimpose an image of the needle 1200 on to an image produced by the ultrasound beam 1222 of the probe 1140. FIGS. 23A and 23B show examples of such a superimposition of the needle onto an ultrasound image. Specifically, FIGS. 23A and 23B each show a screenshot 1230 that can be depicted on the display 1130 (FIG. 19), for instance. In FIG. 23A, an ultrasound image 1232 is shown, including depiction of the patient skin surface 1220, and the subcutaneous vessel 1226. The ultrasound image 1232 corresponds to an image acquired by the ultrasound beam 1222 shown in FIGS. 22A and 22B, for instance.

The screenshot 1230 further shows a needle image 1234 representing the position and orientation of the actual needle 1200 as determined by the system 1110 as described above. Because the system is able to determine the location and orientation of the needle 1200 with respect to the sensor array 1190, the system is able to accurately determine the position and orientation of the needle 1200 with respect to the ultrasound image 1232 and superimpose it thereon for depiction as the needle image 1234 on the display 1130. Coordination of the positioning of the needle image 1234 on the ultrasound image 1232 is performed by suitable algorithms executed by the processor 1122 or other suitable component of the system 1110.

The sensors 1192 are configured to continuously detect the magnetic field of the magnetic element 1210 of the needle 1200 during operation of the system 1110. This enables the system 1110 to continuously update the position and orientation of the needle image 1234 for depiction on the display 1130. Thus, advancement or other movement of the needle 1200 is depicted in real-time by the needle image 1234 on the display 1130. Note that the system 1110 is capable of continuously updating both the ultrasound image 1232 and the needle image 1234 on the display 1130 as movements of the probe 1140 and the needle 1200 occur during a placement procedure or other activity.

FIG. 23A further shows that in one embodiment the system 1110 can depict a projected path 1236 based on the current position and orientation of the needle 1200 as depicted by the needle image 1234. The projected path 1236 assists a clinician in determining whether the current orientation of the needle 1200, as depicted by the needle image 1234 on the display 1130, will result in arriving at the desired internal body portion target, such as the vessel 1226 shown here. Again, as the orientation and/or position of the needle image 1234 changes, the projected path 1236 is correspondingly modified by the system 1110. A target 1238, indicating the point where the projected path 1236 crosses the plane of the ultrasound image 1232, can also be depicted on the display 1130 by the system 1110. As shown in FIG. 23A, in the present example the target 1238 is located within the vessel 1226 depicted in the ultrasound image 1232. Note that the position of the target 1238 on the display 1130 can also be modified as the needle 1200 and/or the ultrasound image 1232 are adjusted. The screenshot 1230 also includes an area of probability 1239, here depicted as a box, which indicates any possible margin of error of the system due to needle length, needle rigidity and flex, field strength of the magnetic element, magnetic interference, possible discrepancy in alignment of the magnetic axis of the magnetic element with the longitudinal axis of the needle, orientation of the sensor array with respect to the ultrasound imaging plane, etc.

FIG. 23B shows that, in one embodiment, the screenshot 1230 can be configured such that the ultrasound image 1232 and the needle image 1234 are oriented so as to be displayed in a three dimensional aspect. This enables the angle and orientation of the needle 1200, as depicted by the needle image 1234, to be ascertained and compared with the intended target imaged by the ultrasound image 1232. It should be noted that the screenshots 1230 are merely examples of possible depictions produced by the system 1110 for display; indeed, other visual depictions can be used. Note further that the particular area of the body being imaged is merely an example; the system can be used to ultrasonically image a variety of body portions, and should not be limited to what is explicitly depicted in the accompanying figures. Further, the system as depicted and described herein can be included as a component of a larger system, if desired, or can be configured as a stand-alone device. Also, it is appreciated that, in addition to the visual display 1130, aural information, such as beeps, tones, etc., can also be employed by the system 1110 to assist the clinician during positioning and insertion of the needle into the patient.

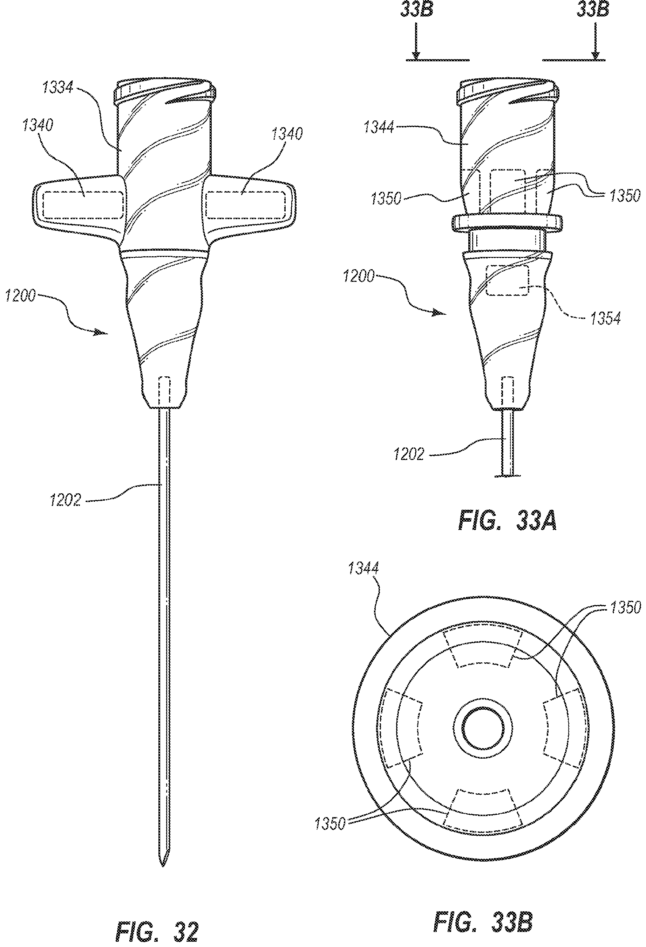

As mentioned above, in one embodiment it is necessary for the system 1110 to know the total length of the needle 1200 and the location of the magnetic element 1210 thereon in order to enable an accurate depiction of the needle image 1234 and other features of the screenshots 1230 of FIGS. 23A and 23B to be made. The system 1110 can be informed these and/or other pertinent parameters in various ways, including scanning by the system of a barcode included on or with the needle, the inclusion of a radiofrequency identification ("RFID") chip with the needle for scanning by the system, color coding of the needle, manual entry of the parameters by the clinician into the system, etc. For instance, an RFID chip 1354 is included on the needle 1200 shown in FIG. 33A. The probe 1140 or other component of the system 1110 can include an RFID reader to read the information included on the RFID chip 1354, such as the type or length of the needle 1200, etc. These and other means for inputting the needle parameters into the system 1110 or detecting the parameters are therefore contemplated.

In one embodiment, a length of the needle (or other aspect of a medical component) can be determined by measurement by the probe/system of a characteristic of the magnetic element, such as its field strength. For instance, in one embodiment the magnetic element of the needle can be positioned at a predetermined distance from the probe or at a predetermined location with respect to the probe. With the magnetic element so positioned, the sensor array of the probe detects and measures the field strength of the magnetic element. The system can compare the measured field strength with a stored list of possible field strengths corresponding to different lengths of needles. The system can match the two strengths and determine the needle length. The needle location and subsequent needle insertion can then proceed as described herein. In another embodiment, instead of holding the magnetic element stationary at a predetermined location, the magnetic element can be moved about the probe such that multiple field strength readings are taken by the probe. Aspects that can be modified so as to impart different field strengths to a set of magnetic element include size, shape, and composition of the magnetic element, etc.

Further details are given here regarding use of the system 1110 in guiding a needle or other medical device in connection with ultrasonic imaging of a targeted internal body portion ("target") of a patient, according to one embodiment. With the magnetic element-equipped needle 1200 positioned a suitable distance (e.g., two or more feet) away from the ultrasound probe 1140 including the sensor array 1190, the probe is employed to ultrasonically image, for depiction on the display 1130 of the system 1110, the target within the patient that the needle is intended to intersect via percutaneous insertion. A calibration of the system 1110 is then initiated, in which algorithms are executed by the processor 1122 of the console 1120 to determine a baseline for any ambient magnetic fields in the vicinity of where the procedure will be performed. The system 1110 is also informed of the total length of the needle 1200, and/or position of the magnetic element with respect to the distal needle tip such as by user input, automatic detection, or in another suitable manner, as has been discussed above.

The needle 1200 is then brought into the range of the sensors 1192 of the sensor array 1190 of the probe 1140. Each of the sensors 1192 detects the magnetic field strength associated with the magnetic element 1210 of the needle 1200, which data is forwarded to the processor 1122. In one embodiment, such data can be stored in memory until needed by the processor. As the sensors 1192 detect the magnetic field, suitable algorithms are performed by the processor 1122 to calculate a magnetic field strength of the magnetic element 1210 of the needle 1200 at predicted points in space in relationship to the probe. The processor 1122 then compares the actual magnetic field strength data detected by the sensors 1192 to the calculated field strength values. Note that this process is further described by the U.S. patents identified above. This process can be iteratively performed until the calculated value for a predicted point matches the measured data. Once this match occurs, the magnetic element 1210 has been positionally located in three dimensional space. Using the magnetic field strength data as detected by the sensors 1192, the pitch and yaw (*i.e.,* orientation) of the magnetic element 1210 can also be determined. Together with the known length of the needle 1200 and the position of the distal tip of the needle with respect to the magnetic element, this enables an accurate representation of the position and orientation of the needle can be made by the system 1110 and depicted as a virtual model, *i.e.,* the needle image 1234, on the display 1130. Note that the predicted and actual detected values must match within a predetermined tolerance or confidence level in one embodiment for the system 1110 to enable needle depiction to occur.

Depiction of the virtual needle image 1234 of the needle 1200 as described above is performed in the present embodiment by overlaying the needle image on the ultrasound image 1232 of the display 1130 (FIGS. 23A, 23B). Suitable algorithms of the system 1110 as executed by the processor 1122 or other suitable component further enable the projected path 1236, the target 1238, and area of probability 1239 (FIGS. 23A, 23B) to be determined and depicted on the display 1130 atop the ultrasound image 1232 of the target. The above prediction, detection, comparison, and depiction process is iteratively performed to continue tracking the movement of the needle 1200 in real-time.

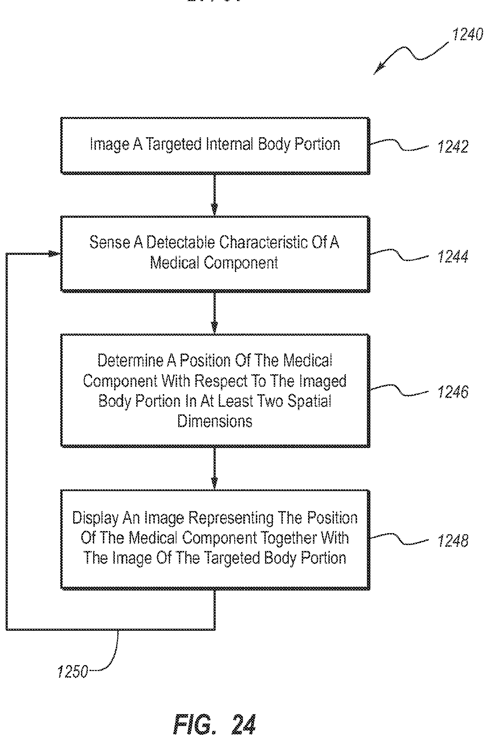

In light of the foregoing and with reference to FIG. 24, it is appreciated that in one embodiment a method 1240 for guiding a needle or other medical component includes various stages. At stage 1242, a targeted internal body portion of a patient is imaged by an imaging system, such as an ultrasound imaging device for instance.

At stage 1244, a detectable characteristic of a medical component such as a needle is sensed by one or more sensors included with the imaging system. In the present embodiment, the detectable characteristic of the needle is a magnetic field of the magnetic element 1210 included with the needle 1200 and the sensors are magnetic sensors included in the sensor array 1190 included with the ultrasound probe 1140.

At stage 1246, a position of the medical component with respect to the targeted internal body portion is determined in at least two spatial dimensions via sensing of the detectable characteristic. As described above, such determination is made in the present embodiment by the processor 1122 of the console 1120.

At stage 1248, an image representing the position of the medical component is combined with the image of the targeted internal body portion for depiction on a display. Stage 1250 shows that stages 1244-1248 can be iteratively repeated to depict advancement or other movement of the medical component with respect to the imaged target, such as percutaneous insertion of the needle 1200 toward the vessel 1226 (FIGS. 23A, 23B), for instance.

It is appreciated that the processor 1122 or other suitable component can calculate additional aspects, including the area of probability 1239 and the target 1238 (FIGS. 23A, 23B) for depiction on the display 1130.

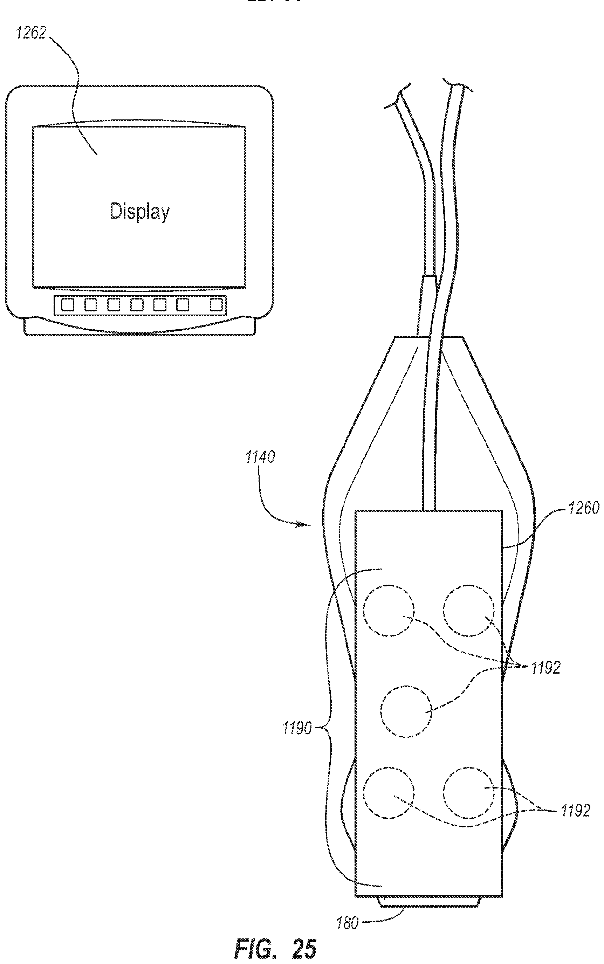

It is appreciated that in one embodiment the sensor array need not be incorporated natively into the ultrasound imaging device, but can be included therewith in other ways. FIG. 25 shows one example of this, wherein an attachable sensor module 1260 including the sensors 1192 of the sensor array 1190 is shown attached to the ultrasound probe 1140. Such a configuration enables needle guidance as described herein to be achieved in connection with a standard ultrasound imaging device, *i.e.,* a device not including a sensor array integrated into the ultrasound probe or a processor and algorithms configured to locate and track a needle as described above. As such, the sensor module 1260 in one embodiment includes a processor and algorithms suitable for locating and tracking the needle or other medical component and for depicting on a display the virtual image of the needle for overlay on to the ultrasound image. In one embodiment, the sensor module 1260 can be included with a module display 1262 for depiction of the needle tracking. These and other configurations of the guidance system are therefore contemplated.

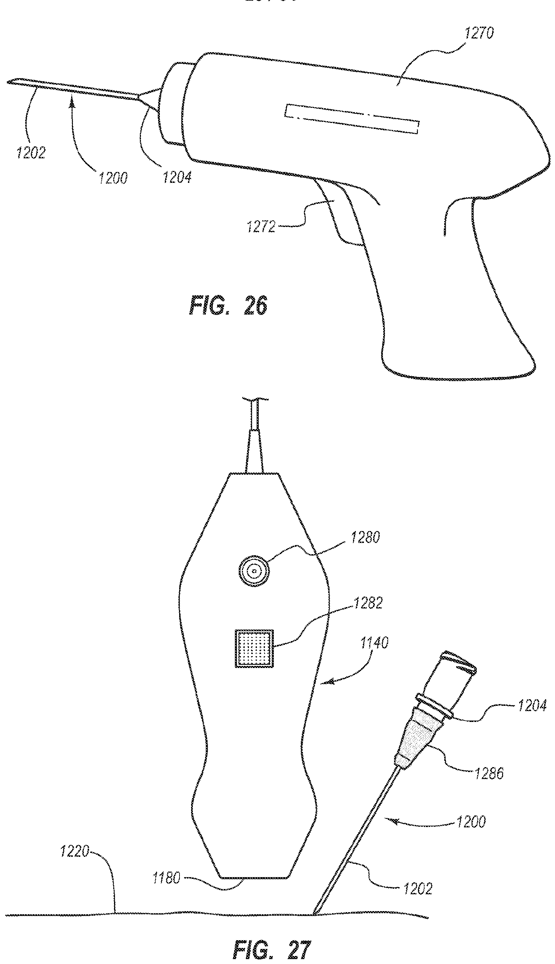

FIG. 26 shows that in one embodiment, a needle holder can be employed to hold and advance the needle 1200 during the ultrasound imaging and needle guidance procedure performed by the system 1110 as has been described. As shown, the needle holder 1270 is pistol-shaped and includes a trigger 1272 for selectively advancing the needle 1200 or other suitable medical component by moving the needle longitudinally away from the barrel of the holder upon pressing of the trigger. So configured, the needle holder 1270 facilitates ease of needle handling with one hand of the clinician while the other hand is grasping and manipulating the ultrasound probe 1140. In addition, the needle holder 1270 can provide needle movement/rotation assistance such as via a motor, ratcheting, hydraulic/pneumatic drivers, etc. Moreover, a clocking feature can be included on the needle holder 1270 to assist with determining the orientation of the distal tip of the needle 1200 and for facilitating rotation of the needle.

In one embodiment, the needle holder 1270 can be operably connected to the system 1110 such that advancement by the needle holder is automatically stopped when the distal end 1202B of the needle cannula 1202 reaches the targeted internal body portion or the needle intercepts the ultrasound plane. In yet another embodiment the magnetic element can be included with the needle holder instead of the needle itself. The needle, when temporarily attached to the needle holder, can thus be located and guided by the guidance system without the need for a magnetic element to be attached directly to the needle.

Note that other sensor configurations can also be employed. In one embodiment, an annular sensor can be configured to receive through a hole defined thereby the cannula of the needle. So disposed, a magnetic element of the needle is positioned proximate the annular sensor, which enables ready detection of the magnetic element and location of the needle by the system. The annular sensor can be attached to a surface of the probe, in one embodiment.

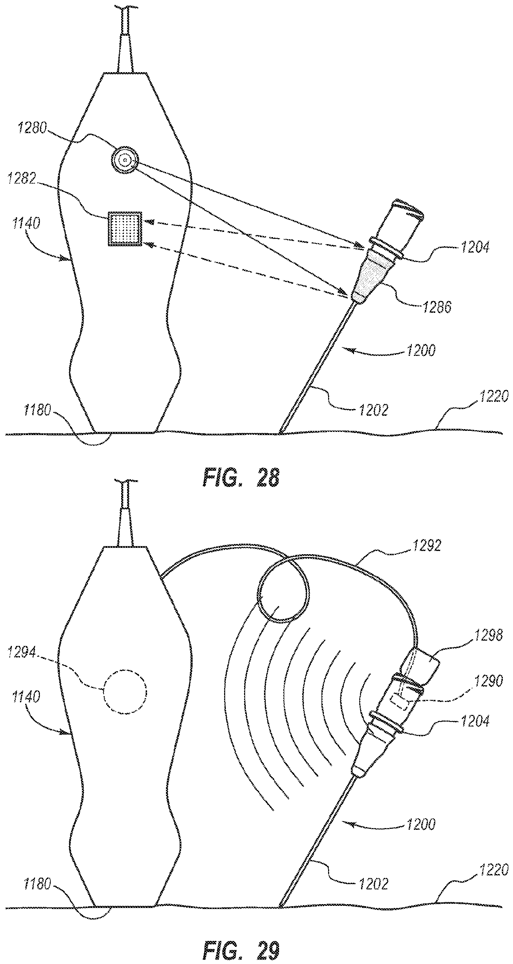

FIGS. 27 and 28 depict components of the guidance system 1110 according to another embodiment, wherein an optical-based interaction between the probe 1140 and the needle 1200 is employed to enable tracking and guidance of the needle. In particular, the probe 1140 includes a optical/light source, such as an LED 1280, and a photodetector 1282 positioned on the probe surface. It is appreciated that the light source and detector can be configured to produce and detect light signals of a variety of ranges including visible, infrared, etc.

The needle hub 1204 includes a reflective surface 1286 capable of reflecting light produced by the LED 1280 and incident thereon. As shown in FIG. 28, light emitted by the LED 1280 is reflected by the reflective surface 1286 of the needle 1200, a portion of which is received and sensed by the photodetector 1282. As in previous embodiments, the processor 1122 of the system console 1120 can be employed to receive the sensed data of the photodetector 1282 and compute the position and or orientation of the needle 1200. As before, the length of the needle 1200 and/or the position of the reflective surface with respect to the distal end of the needle 1200 are input into or otherwise detectable or known by the system 1110. Note that the reflective surface can be included at other locations on the needle.

In light of the above, it is appreciated that in the present embodiment the detectable characteristic of the needle 1200 includes the reflectivity of the reflective surface 1286, in contrast to the magnetic field characteristic of the magnetic element 1210 of previous embodiments, and the sensor includes the photodetector 1282, in contrast to the magnetic sensors 1192 of previous embodiments. It should be appreciated that in one embodiment, the above-described configuration can be reversed, wherein an optical source is included with the needle or medical component. In this case, light is emitted from the needle and detected by the photodetector 1282 included with the probe 1140 so as to enable location and tracking of the needle. A power source can be included with the needle, such as a watch battery or the like, in order to power the light source of the needle.

More generally, it is appreciated that the needle or medical component can include one or more of these or other detectable characteristics to enable the needle to be tracked and guided toward a target within the body of the patient. Non-limiting examples of other detectable characteristic modalities include electromagnetic or radiofrequency ("RF") (*see*, *e.g*., FIGS. 29-30 below), and radioactivity. With respect to RF modalities, it is appreciated that one or more synchronously or asynchronously pulsed frequency sources can be included with the needle as to enable detection thereof by a suitable sensor(s). Or, an RF first source can be coupled with a passive magnet as a second source.

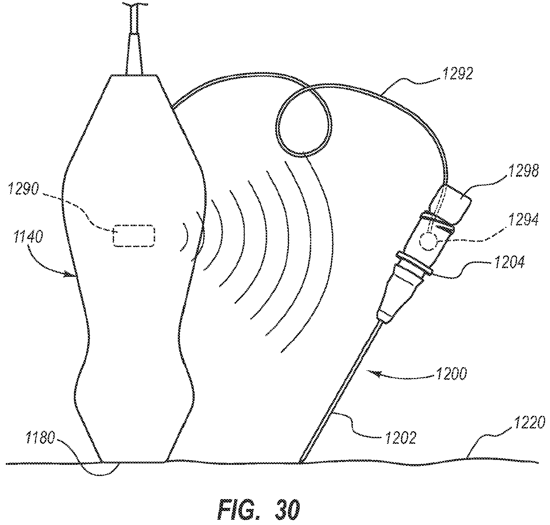

FIGS. 29 and 30 depict components of a guidance system according to one embodiment, wherein EM signal interaction between the probe 1140 and the needle 1200 is employed to enable tracking and guidance of the needle. In particular, in FIG. 29 the needle 1200 includes a stylet 1298 disposed therein. The stylet 1298 includes an EM coil 1290 that is operably connected to the probe 1140 via a tether 1292. In this way, the EM coil 1290 can be driven by suitable components included in the probe 1140 or system console 1120 such that the EM coil emits an EM signal during operation.

A sensor 1294 suitable for detecting EM signals emitted by the EM coil 1290 of the stylet 1298 is included in the probe 1140. In the present embodiment, the sensor 1294 is a three-axis sensor for detecting corresponding orthogonal components of the EM signal, though other coil and sensor configurations can also be employed. So configured, the position and orientation of the needle 1200 can be determined, by EM signal triangulation or other suitable process, and displayed by the system in a manner similar to that already described above. As in previous embodiments, the processor 1122 of the system console 1120 (FIG. 18) can be employed to receive the sensed data of the EM sensor 1294 and compute the position and/or orientation of the needle 1200. As before, the length of the needle 1200 and/or the position of the EM coil 1290 with respect to the distal end of the needle 1200 are input into or otherwise detectable or known by the system.

FIG. 30 shows a variation of the EM configuration of FIG. 29, wherein the respective positions of the EM components is reversed: the EM coil 1290 is included in the probe 1140 and the EM sensor 1294 is included with the stylet 1298 disposed in the needle 1200. Note that in the embodiments of FIGS. 29 and 30, the operable connection between the EM coil 1290 and the EM sensor 1294 via the tether 1292 enables the component disposed in the stylet 1298 to be driven by the system 1110. This also enables correspondence of the particular EM frequency/frequencies emitted by the EM coil 1290 and detected by the EM sensor 1294 to be made. In one embodiment, the configuration shown in FIG. 29 can be varied, wherein no tether operably connects the EM coil and the EM sensor; rather, the EM coil of the stylet operates as a separate component from the probe and its EM sensor and is powered by an independent power source, such as a battery. In this case, the probe/system includes suitable signal processing components configured to detect the EM signal emitted by the EM coil and to process it as necessary in order to locate the needle.

Note that the EM coil and EM sensors can be included at other locations than what is depicted herein. For instance, the EM coil can be included on the needle itself, or on a connector that is attachable to the proximal end of the needle.

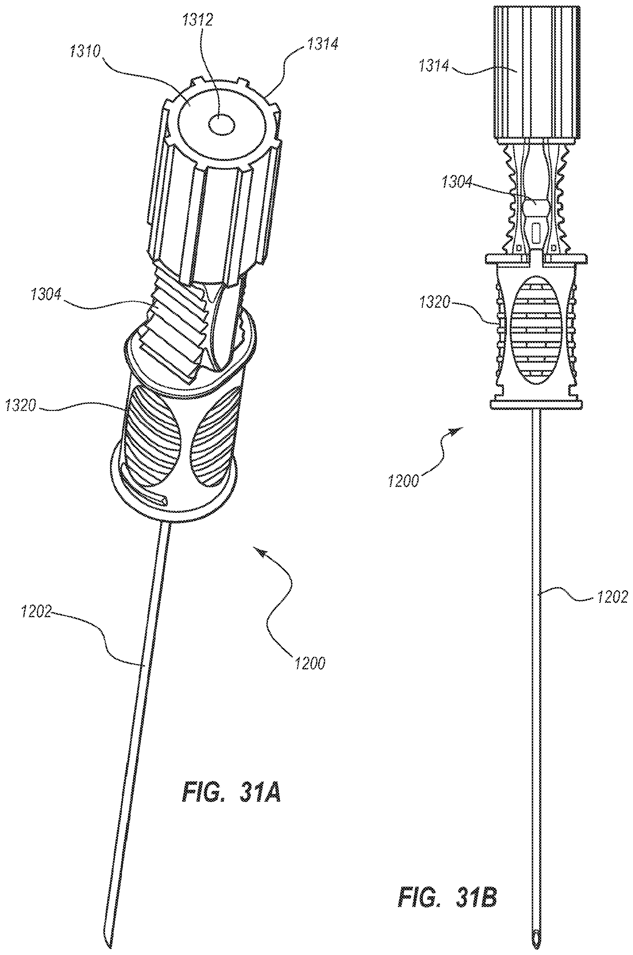

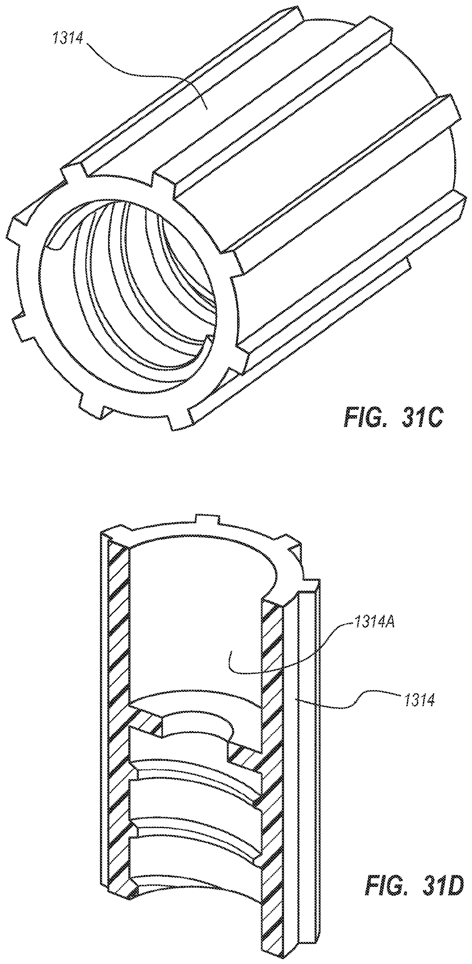

FIGS. 31A-31D give further details of the needle 1200 configured according to one embodiment, wherein the needle includes a hub 1304 from which extends the cannula 1202. A magnetic element 1310 defining a hole 1312 is included in a cavity 1314A of a housing 1314. The housing 1314 includes threads so as to threadably engage the needle hub 1304 or other suitable component of the needle or medical component. In this way, the magnetic element 1310 is removably attachable to the needle 1200 via the housing 1314. Thus, the magnetic element 1310 need not be permanently affixed or included with the needle 1200, but rather can be removed therefrom when magnetic-based needle guidance is no longer needed. In addition, this enables the magnetic element to be attached to many different types and sizes of needles. Note that in the present embodiment the needle 1200 further includes a distally slidable needle safety component 1320 for safely isolating the distal tip of the needle upon removal of the needle from the patient. Note further that other removable magnetic elements can be employed in addition to what is explicitly shown and described herein.

FIGS. 32-33B give further examples of the needle 1200 including a magnetic element. In FIG. 32, two bar-like magnetic elements 1340 are disposed so as to orthogonally extend from a hub 1334 of the needle 1200, illustrating that the magnetic element need not be oriented parallel to the longitudinal axis of the needle. In FIGS. 33A-33B, four magnetic elements 1350 are included in the needle hub 1344, showing that more than one magnetic element can be included with the needle. Such a configuration may be employed, for example, where limited space prevents one magnetic element from being used. Note the number, shape, and placement of the magnetic elements here is only one example of many possible configurations.

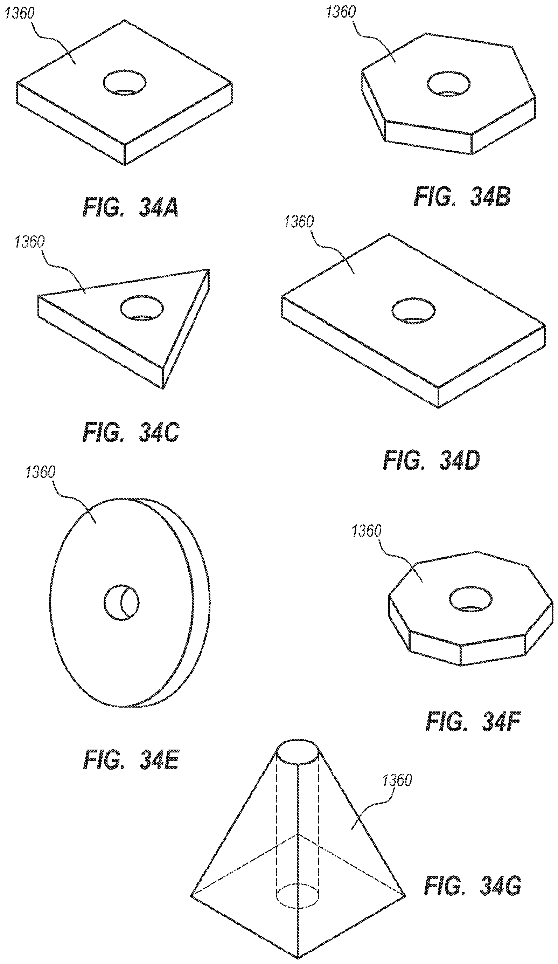

FIGS. 34A-34G give various example configurations of a magnetic element 1360 that defines a hole for receiving the cannula of the needle therethrough. Various shape configurations for the magnetic element 1360 are shown, including a square (FIG. 34A), a hexagon (FIG. 34B), a triangle (FIG. 34C), a rectangle (FIG. 34D), an oval (FIG. 34E), an octagon (FIG. 34F), and a four-sided pyramid (FIG. 34G). The magnetic elements shown in the accompanying figures are merely examples of the broad number of geometric and other shapes that can be used to define the magnetic element; indeed other shapes not shown explicitly herein are also contemplated.

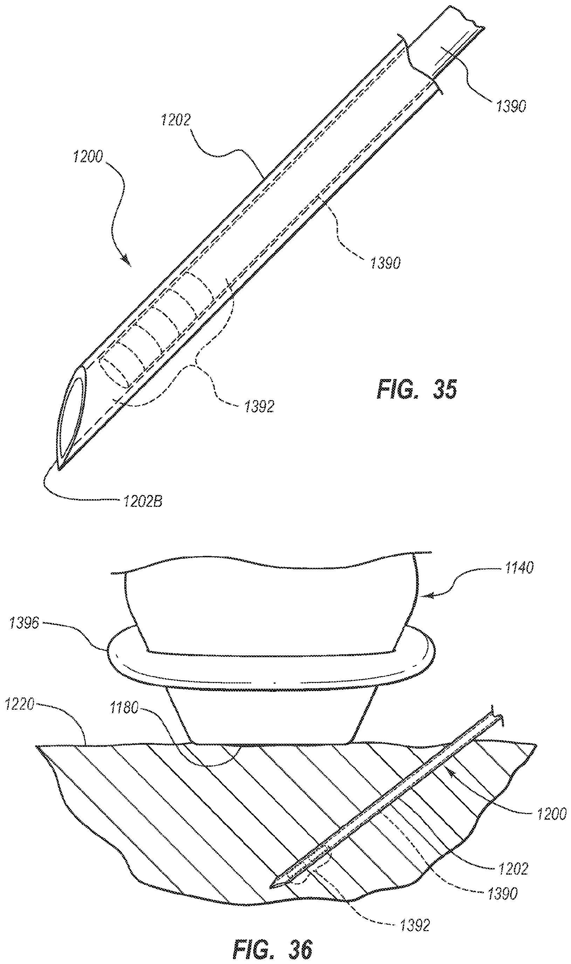

FIGS. 35 and 36 depict yet another embodiment, wherein a stylet 1390 is included for removable insertion into the hollow cannula 1202 of the needle 1200. A plurality of permanent magnets 1392, such as solid, cylindrically shaped ferromagnets stacked end-to-end with each other, is included at a distal end of the stylet 1390. As shown in FIG. 36, the stylet 1390 is received within the needle cannula 1202 during insertion of the needle 1200 into the patient. A sensor ring 1396 or other suitable magnetic sensor can be included with or in proximity to the probe 1140 to enable detection of the magnetic field of the magnets 1392, thus enabling the guidance system to detect the position and orientation of the needle 1200 and superimpose an image thereof atop the ultrasound image produced by the probe 1140 in a manner similar to that described in connection with FIGS. 5A-7.

FIGS. 35 and 36 thus illustrate that the magnetic element(s) can be configured in any one of a variety of ways. In one embodiment, for example, the magnetic elements can be disposed more proximally along the stylet length. In another embodiment, the stylet itself can be magnetized or composed of magnetic materials. It is appreciated that the stylet can be configured in one of many different ways, analogous examples of which can be found in U.S. Patent No. 5,099,845 entitled "Medical Instrument Location Means," and. U.S. Patent Application Publication No. 2007/0049846, filed August 23, 2006, and entitled "Stylet Apparatuses and Methods of Manufacture," both of which are incorporated herein by reference in their entireties. These and other variations are therefore contemplated.

It should be appreciated herein that "stylet" as used herein can include any one of a variety of devices, including guidewires, configured for removable placement within a lumen of the needle to assist in the placement thereof within the patient. In one embodiment, the stylet can include a sharp end that distally extends past a blunt distal end of the needle cannula so as to enable a blunt needle to be inserted into a patient. Note that the stylet in one embodiment stiffens the needle so as to minimize unintended bending thereof during insertion.

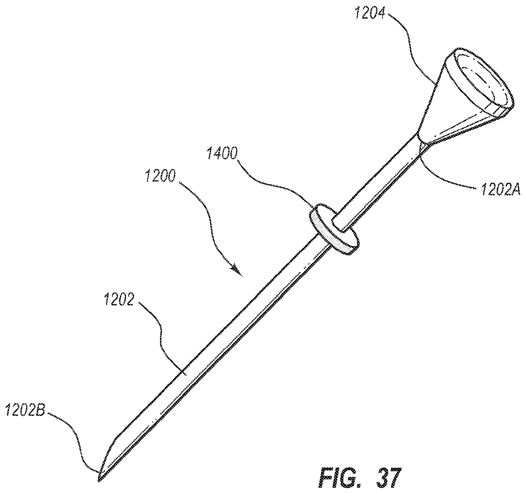

FIG. 37 depicts yet another possible embodiment, wherein the needle 1200 includes an annular or donut-shaped magnet 1400 disposed distal to a proximal end 1202A of the needle cannula 1202. Note that the magnet 1400 can be positioned in one of several positions along the length of the cannula 1202, in other embodiments. Positioning of the magnet 1400 relatively closer to the distal needle tip reduces the effects that unintended bending of the needle has on determining and displaying the position of the needle. In yet another embodiment, the needle itself can be magnetized. Note further that the relative places of the sensor and source (*e.g*., magnet) of the system can be reversed. These and other configurations are also contemplated. Further, note that the discussion herein can be applied to other imaging modalities in addition to ultrasound, including MRI, x-ray and CT scanning, etc.

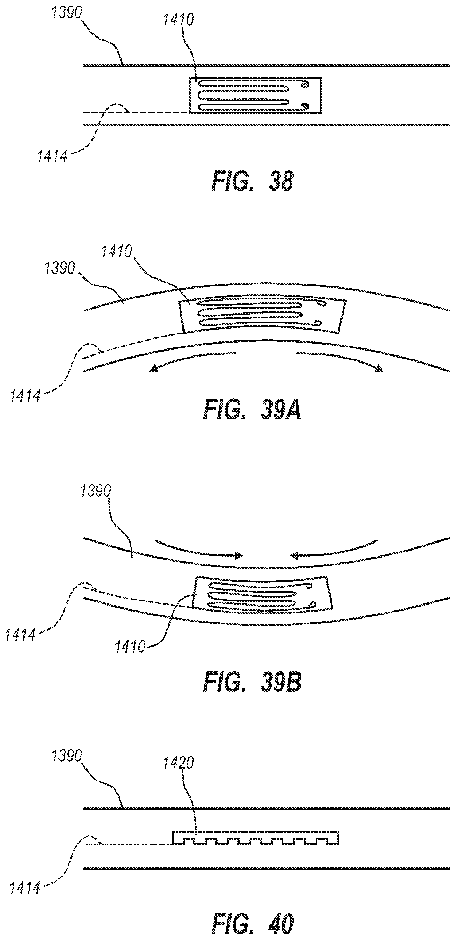

FIG. 38 depicts a strain gauge 1410 included on a stylet, such as the stylet 1390 shown in FIGS. 35 and 36 for instance. The strain gauge 1410 can be operably connected to the probe 1140, console 1120 (FIG. 18), or other component of the system 1110 via a conductive path 1414. One example of the conductive path 1414 includes one or more conductive wires disposed in or along the stylet 1390, for instance. So connected, the strain gauge 1410 acts as a transducer and can provide data relating to bending of the needle in which the stylet 1390 is disposed during needle insertion procedures, given that bending of the needle 1200 will cause similar bending to occur in the stylet 1390.

These data sensed via bending of the strain gauge 1410 can be forwarded to and interpreted by the processor 1122 (FIG. 18) or other suitable component of the system 1110 so as to include such bending together with detection of the magnetic element by the probe sensors 1192 (FIG. 20) in computing the position of the needle 1200, especially the distal tip thereof. This results in enhanced accuracy for locating and depicting the position of the needle distal tip. Indeed, FIG. 39A shows flexure of the strain gauge 1410 in one direction as caused by bending of the stylet 1390, wherein FIG. 39B shows flexure of the strain gauge in another direction. Such stylet bending is thus detected by the strain gauge 1410 (via changes in electrical resistance within the strain gauge in one embodiment) and forwarded to the system 1110 for use in computing needle position. Note that other suitable sensors and gauges can optionally be used for measuring needle/stylet bending, including a flex sensor 1420, as shown in FIG. 40 for instance, and capacitance and fiber optic-based strain gauges/sensors. Also, the sensor/gauge may be placed directly on the needle/medical component, in one embodiment.

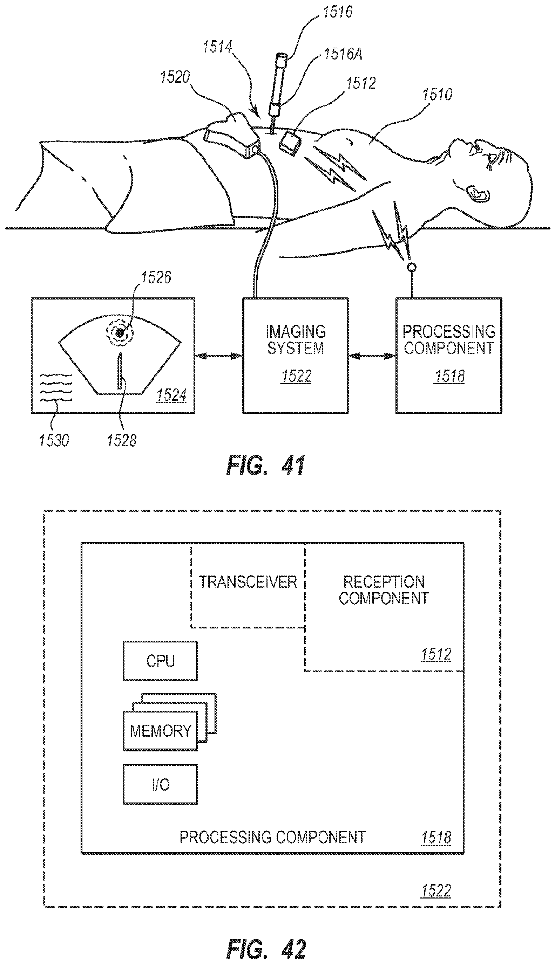

FIGS. 41-82 provide various further details regarding guidance systems and medical devices that can be guided thereby, in accordance with present embodiments. By way of further introduction, it is appreciated that minimally invasive systems are increasingly popular in clinical medicine. Minimally invasive systems assist in diagnosis and therapy in a manner that is safer, faster, and less expensive than traditional procedures, and minimally invasive systems typically result in reduced patient discomfort and a shorter recovery time.

Laparoscopy is an example of a minimally invasive procedure. Prior to the 1980's, a patient's gall bladder was typically removed with a conventional, surgical cholecystectomy. Following a cholecystectomy, a hospital stay of three or four days and a month away from work was not uncommon. Today, a patient's gall bladder is routinely removed using laparoscopy with no or one day in the hospital and often, the patient can return to work in less than a week. The improvement provided for the patient by a minimally invasive laparoscopy procedure is dramatic.

Another example of a minimally invasive medical procedure occurs in neurosurgery. One system involves precisely tracking a small, rare earth magnet in a patient's body using sets of magnetic position sensors. In neurosurgery, the magnetically guided system is used, for example, to determine the position of a valve in a ventricular-pleural shunt, ventricular-peritoneal shunt, or other shunt device in the ventricular system. In another example, magnetic detectors or other devices that cooperate with the magnetically guided system can be use to locate the tip of a peripherally inserted central catheter (PICC) line during placement.

The laparoscopy, neurosurgical shunt, and PICC procedures have improved safety, increased efficiency, and reduced the cost of the procedures over conventional methods. It has been recognized, however, that both laparoscopy and the neurosurgical shunt procedures, and other minimally invasive medical procedures can be improved.

In accordance with the foregoing, a system is disclosed herein for accurately tracking the movement and location of a substantially rigid, or non-rigid, medical device with substantial precision to a particular area of concern. Non-limiting examples of substantially rigid medical devices include, for instance, needles, brushes, biopsy forceps, etc., that are generally passed into a patient's body for diagnostic, therapeutic, or other purposes (e.g., Seldinger or other percutaneous procedures). Rigid medical devices can be straight, curved, spiraled, or may be another shape. Further, not only rigid medical devices, but semi-rigid devices, composite devices, devices of varying rigidity, malleable devices, and non-rigid devices can be employed in connection with the present disclosure. Indeed, the medical device can be malleable in one configuration and sufficiently stiff in another configuration. As such, it should be appreciated that "rigid medical device" as used herein includes substantially rigid, semi-rigid, and non-rigid medical and other devices as detailed above.

In the embodiments described herein, several procedures are described wherein a medical device is guided to a particular area of concern with substantial precision. In the embodiments, "substantial precision," and other descriptions of like terms, are used to indicate that the medical device is guided to a location at or near the area of concern to within a desirable distance.

Further in the embodiments described herein, the several procedures describe guidance of a medical device to the particular area of concern. The "particular area of concern," target area, and other similar descriptions typically indicate an internal location of a patient's anatomy. The area of concern may be a tumor, cyst, bleeding vessel, injury, anomaly, device, structure, or any other desirable area of interest to medical practitioner.

In the new system, one locus of the rigid medical device can be accurately tracked when at least one magnetic element, such as a permanent magnet, is placed at a different locus of the medical device. In one embodiment, the medical device is a needle and a rare earth magnet is placed at the base of the needle such that the tip of the needle is tracked as it travels through the patient's body. As mentioned, other types of medical and non-medical devices can also be tracked.

As used herein, a medical device includes a proximal end and a distal end. The proximal end of the rigid medical device generally corresponds to the end of the device that is held or controlled by a medical practitioner. The proximal end is the end that is generally outside of the patient's body during a procedure. In contrast, the distal end of the rigid medical device generally corresponds to the end of the device that is advanced inside of the patient's body during a procedure. For example, the base of a needle corresponds to the proximal end, and the tip of the needle corresponds to the distal end.

In the embodiments described herein, neither the proximal end nor the distal end of a rigid medical device has a strict starting point or ending point. The portion of the device defined as the proximal end is not necessarily the same in size or extent as the portion of the device defined as the distal end. In addition, in some embodiments the proximal end or the distal end of a rigid medical device extends beyond the mid-point of the device. The proximal end and distal end of the device are also referred to herein as the proximal portion and distal portion, respectively, of the device.

In one embodiment, a magnetic tracking device is able to determine the position of the magnetic element-equipped needle base in at least three dimensions and in real time, as described further above in connection with previous embodiments. From the determination of the position of the base of the needle, the system can further determine the position of the tip of the needle, as has been discussed.

In one embodiment and as has already been described further above, the magnetic tracking device is further operable to provide information representative of the needle's position to a real time imaging system such as medical sonography (e.g., ultrasound), computerized tomography (CT), medical radiography (e.g. fluoroscopy), nuclear medical imaging, medical thermography, or any other imaging technology that will not insurmountably interfere or be affected by the magnetic guidance system. In such cases, the location of the needle can be shown as an integrated or overlay image on the imaging system display concurrent with the display of the patient's internal anatomy. An operator of the real time imaging system can accurately guide the tip of the needle to a desired location in a patient while imaging the patient's anatomy. In some cases, the magnet-bearing base on the proximal portion of the needle remains outside the patient's body.

Further, and in light with the discussion in connection with the previous discussions further above, embodiments of the rigid medical device tracking system, including the above-described magnetic tracking device for example, can facilitate diagnostic procedures, therapeutic procedures, planning procedures (e.g., virtual image tracking), and other procedures. For example, such embodiments can be used to plan the travel path of a rigid medical device such as a needle that is passed into a patient's body. That is, the system can inform the medical practitioner of the needle's direction and depth of insertion, which permits the medical practitioner to guide the needle as it is advanced. In addition, the system can further be operated so as to confirm the location of the needle tip in the patient's body and prevent undesired contact with particular anatomical or artificial structures.

The rigid medical device tracking system can provide benefits in many diagnostic medical procedures. For example, in some medical procedures a needle is inserted into a patient for the aspiration of fluid (e.g., cerebrospinal fluid (CSF), arterial blood, venous blood, gall bladder fluid, abscesses, cysts, and other fluid collections). In diagnostic procedures that include fluid aspiration, the rigid medical device tracking system helps medical practitioners perform the procedure more efficiently and safely than previously known.

There are many other diagnostic medical procedures where the rigid medical device tracking system can be used. Some non-limiting embodiments include biopsy forceps insertion, manual and automatic biopsy devices, brush insertion to obtain tissue for cytology, device insertion to study blood flow using thermal dilution methods, urinary system drain placement, lumbar puncture, and extraction of tissue for biomarkers.

Still other medical procedures where the rigid medical device tracking system can be used involve safely accessing a particular situs inside of a patient's body. In one example, particular contrast enhancing fluids or other materials are introduced to a particular organ or other location in the patient's body for the purpose of detection within medical imaging system. Such enhancing materials are beneficial to x-ray diagnostics, ultrasound diagnostics, magnetic resonance imaging (MRI) diagnostics, and other imaging modalities. The opportunity for medical practitioners to introduce such materials with an improvement in precision generally results in better diagnosis and reduced discomfort for the patient.

In another example, particular markers that are attracted to and bind with specific tissue types (*e.g*., tumor) can be injected with highly desirable precision. Once injected, the marker can be detected with a variety of techniques such as ultrasound and nuclear medicine. In some cases, the marker can be used at a later point in time *(e.g.,* by a surgeon), and in other cases the marker can be used in real time. Some real time examples include a marker used by an endoscopist to locate an area, a marker used by a radiation therapist to direct external beam therapy or to know where to locate seeds for local radioactive therapy, and by a radiologist to follow an area of concern with such methods including ultrasound, x-ray, CT, MRI, PET, angiography, and others.

In the examples described herein, and in others, the procedures can be conducted directly on a patient even if the patient is a fetus. For example the rigid medical device tracking system can be used for prenatal therapy of the fetus and the mother. Examples include amniocentesis, fetal taps to the renal system, ventricular central nervous system (CNS), bladder, intestinal lumen, and others.

The rigid medical device tracking system can also provide benefits in many therapeutic medical procedures. Some examples include the injection of fluid to sclerose tissue, the injection of agents to clot tissue, the application of cold to freeze tissue, the application of heat to coagulate or kill tissue by using energy delivered by laser, radiofrequency (RF) electrical energy, resistive electrical energy, microwave, infrared, and others via a monopolar, bipolar, or multi-polar device, and the application of heat to coagulate tissue using a catheter with a directly heating tip. Other examples include the fulguration of tissue (with RF, laser, or other method), the infusion of material into an artery or vein, the placement of an arterial or venous line for infusion, and the injection of new material such as bone material, cartilage, or growth factors into abnormal bone areas (e.g., cysts, fracture, neoplasm, and others) or into joints that may be abnormal from trauma, arthritis, tumors, or other reasons. Still other examples include the placement of mesh or other supportive structures, the injection of chemotherapeutic agents to treat tumors, the injection of anesthetic agents to produce analgesia by effect of the agent on the nerves (e.g., nerve block, regional block), the placement of a needle or antenna for microwave heating, the placement of a light source to interact with photodynamic agents in diagnosis and therapy, and the placement of a radiation emitting catheter for localized radiotherapy.

Additional examples where the rigid medical device tracking system can also provide benefits include prenatal therapy in a fetus (in utero and ex utero), as mentioned above. For example the rigid medical device tracking system may be useful for any patient, including a fetal patient, for draining cysts, placing ventricular shunts, placing bladder drains, placing stents, correcting cardiac abnormalities, and nearly any diagnostic and therapeutic medical procedure that involves a medical practitioner introducing a rigid medical device into the body of a patient.

Ophthalmologic procedures may also benefit by the use of the medical device tracking system described herein. For example, some embodiments of the system permit a medical practitioner to determine the distance from a planned or actual entry point on an eye to the retina or other internal structure. The medical device tracking system can be used to place and control devices for prosthetic and/or organs insertion such as cataracts and corneal transplant. In such ophthalmologic procedures, the rigid medical device tracking system may be used with a corresponding imaging system.

In Seldinger technique applications, the rigid medical device tracking system described herein may be used to place a rod into a particular hollow organ in the patient's body *(e.g.,* via an over tube). In such cases, when the tip of the rod is in position, an anchor may be operated in the target area *(e.g.,* cyst, tumor). After placement of the Seldinger-type device, a variety of devices, medicines, and/or other therapies can be directed to the area of concern through and over the initial device.

FIG. 41 shows a patient undergoing a medical procedure with equipment including a rigid medical device tracking system working in cooperation with a medical imaging system. In FIG. 41, the patient 1510 is lying supine. The patient is undergoing a particular medical procedure. A rigid medical device tracking system includes both a reception component 1512 and a processing component 1518. As shown in FIG. 41, the reception component 1512 communicates wirelessly to the processing component 1518 via any one of several suitable wireless protocols. In another embodiment, however, the communications could be wired.

The rigid medical device tracking system in accordance with present embodiments determines the position of a rigid medical device 1514. Particularly, the rigid medical device 1514 includes at least one magnetic element, such as a magnet 1516. The magnet 1516 in the present embodiment is located on a proximal portion of the medical device 1514 such that it remains external with respect to the patient 1510 after placement of a distal portion of the medical device has been inserted into the patient. Note, however, that in accordance with other embodiments already described further above, the magnetic element can be disposed in a portion of the medical device that is indeed inserted into the patient.

In FIG. 41, the reception component 1512 of the rigid medical device tracking system detects magnetic properties produced by the magnet 1516 of the rigid medical device 1514. The reception component 1512 derives position information from the detected magnetic properties and provides the information to the processing component 1518. From the information provided by the reception component 1512, the processing component 1518 determines information associated with the three dimensional position and orientation of the magnet. In addition, in combination with information related to the particular structural parameters of the rigid medical device 1514, the processing component 1518 is able to generate information associated with the three dimensional location and orientation of some or all parts of the rigid medical device, such as the distal tip. Note that in one embodiment, determination of the position and orientation of the magnet, and thus the distal tip or other part of the medical device, is performed in a manner similar to that described above in connection with FIGS. 18-24, or by using the other methods described herein.

In FIG. 41, the magnet 1516 is located on a proximal end of the rigid medical device 1514 (*i.e.,* the end held and used by the medical practitioner to guide the device). One reason that the magnet 1516 would be located away from the distal end of a rigid medical device is to preserve the shape, size, or other features of the distal end of the rigid medical device. It is recognized that if the magnet is located on the proximal end of the rigid medical device and if particular information about the rigid medical device is known, then the distal end of the rigid medical device can be tracked with substantial accuracy, as has been described.

As mentioned, the reception component 1512 provides information to the processing component 1518, and the processing component 1518 determines the position of the magnet 1516. Subsequently, the processing component 1518 uses information associated with the structural parameters of the rigid medical device 1514 (*e.g.,* length, diameter, location of magnet on the device, and the like), to determine the position and/or orientation of the tip of the rigid medical device 1514. In some cases, the determined position/orientation information is absolute, and in other cases, the information is relative to a known or determinable point of reference. For instance, knowing the length of the medical device, such as a needle, together with knowledge of the distance of the magnet from the distal tip of the device, enables the processor to determine the position and orientation of the distal tip of the device, as has been described further above in connection with previous embodiments, such as those discussed in connection with FIGS. 18-37, further above.

The magnet 1516 illustrated in FIG. 41 may include one or more magnets. In cases where at least two magnets are used, the plurality of magnets may be used separately or in combination to provide information for tracking the rigid medical device 1514. In some cases, each of the plurality of magnets is placed in close proximity to the other magnets. In other cases, one magnet may be desirably spaced apart from another magnet.

In cases where magnets are spaced apart, one embodiment may include a first magnet 1516 immediately near a proximal end of the rigid medical device 1514 and a second magnet 1516A further from the proximal end of the rigid medical device 1514. Such an embodiment provides an opportunity to improve the accuracy of tracking information via mathematical calculations using information associated with the position of each of the plurality of magnets.

In FIG. 41, the processing component 1518 of the rigid medical device tracking system bi-directionally communicates with a medical imaging system 1522. The medical imaging system of FIG. 41 is an ultrasound system, but other medical imaging systems could also be used in other embodiments. An imaging pod 1520, which is cooperatively used with the medical imaging system 1522, provides information over a wired or wireless link to the medical imaging system 1522. The information provided by the imaging pod 1520 permits the medical imaging system 1522 to generate images representative of internal anatomy and/or structures of the patient 1510. Generally, the images are displayable on a display device 1524 associated with the medical imaging system 1522.

In FIG. 41, the imaging pod 1520 is illustrated as separate from the reception component 1512. In other embodiments, however, the reception device 1512 may be integrated physically and/or electronically with the imaging pod 1520, analogous to the integration of the sensor array 1190 in the ultrasound probe 1140 as shown in FIGS. 20 and 22A-22B. Further, multiple imaging pods 1520 and multiple reception devices 1512 can be used independently or cooperatively, and such multiple devices can be integrated or separate in any desirable combination. In some cases, the multiple reception devices 1512 are all magnetic, and in other cases, the reception devices 1512 are a combination of magnetic devices and other technologies.

In FIG. 41, the medical imaging system includes an ultrasound imaging system similar to that shown in FIGS. 18-19. As such the imaging pod 1530 is an ultrasound probe, similar to the probe 1140 shown in FIGS. 18-20 and 22A-22B. Note therefore that the system 1110 of FIGS. 18-19 and the medical imaging system/medical device tracking system can share many similar aspects. A real time, ultrasound image stream is displayed on the display device 1524. The image stream includes representative images of a tumor 1526 and a needle 1528. The needle image 1528 is a representation of the rigid medical device 1514 that is being manipulated and/or advanced into the patient 1510. Further information 1530 related to the rigid medical device 1514 is also displayed on the display device 1524.

In the embodiment of FIG. 41, a medical practitioner is able to visually observe a representative image, *i.e.,* the needle image 1528 of a rigid medical device "inside" the patient 1510. The rigid medical device may not be detectable by the medical imaging system 1522, and even if the rigid medical device is detectable, it may only be detectable with very poor clarity and very little accuracy. In contrast, however, the cooperative use of information from the rigid medical device tracking system permits representative imagery to be generated with substantial accuracy and depicted on the display of the medical imaging system.

FIG. 42 illustrates a combination useful in several embodiments for a rigid medical device tracking system. The processing component 1518 includes a central processing unit, memory, and input/output circuitry. In one embodiment the processing component 1518 is similar to the processor 1122 shown in FIG. 18. In some embodiments, the receiving component 1512 is an integral part of the processing component 1518, but in some embodiments, such as in FIG. 41, the components are separate. For instance, in comparison to previous embodiments, the receiving component 1512 here is similar to the sensor array 1190 of FIG. 18, while the processing component 1518 is similar to the processor 1122 housed in the console 1120 of FIG. 18. Of course, several variations of these designs are contemplated.

The processing component 1518 of the rigid medical device tracking system may also include one or more transceivers, wired or wireless. The optional transceivers, if they are present, may be used to communicate information between the processing component 1518, the receiving component 1512, a medical imaging system 1522, and other devices.

FIG. 42 also illustrates that the rigid medical device tracking system may optionally be integrated with the medical imaging system 1522 into a single device. In some cases, as in FIG. 41, the rigid medical device tracking system and the medical imaging system 1522 are separate, but it is understood that the whole or separate components of both systems can be separate or integrated (as in the embodiment shown in FIGS. 18-19) in any acceptable manner.

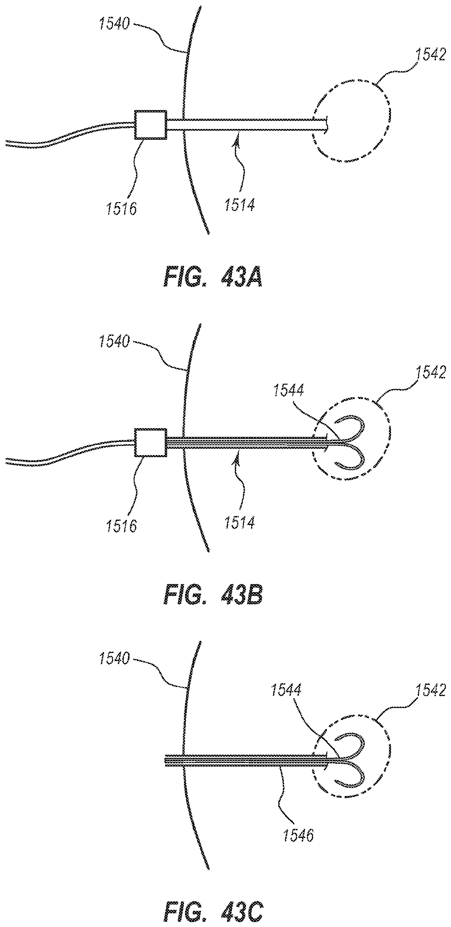

FIGS. 43A-43C illustrate the introduction of a rigid medical device through the skin 1540 of a patient into a particular area of concern, such as a tumor 1542. After the rigid medical device reaches its destination, an anchor 1544 is operated, and the rigid medical device is exchanged for a tube 1546 which can carry one or more devices to the tumor.

In one embodiment, the rigid medical device system may be used with a corresponding imaging system by a medical practitioner to conduct a virtual procedure as a first, virtual method. Subsequently, in real-time, the medical practitioner may use the rigid medical device system to conduct the actual procedure as a second, real procedure.

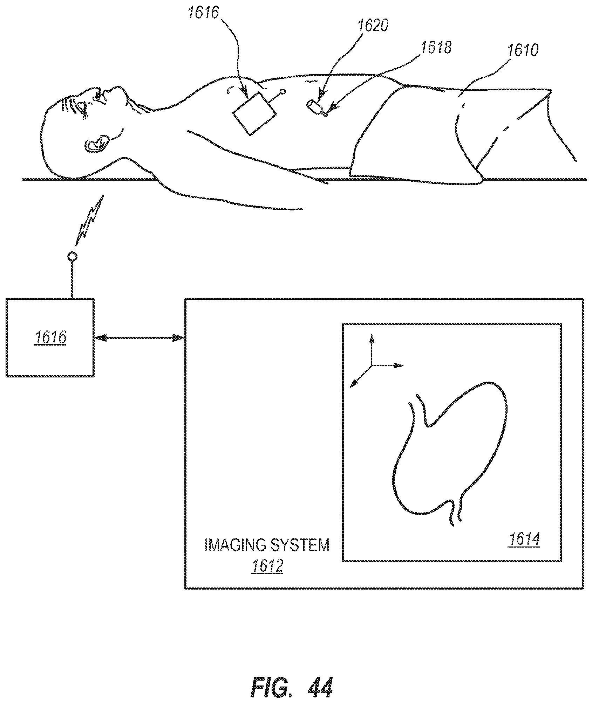

As an example of the foregoing, FIG. 44 illustrates the use of a rigid medical device tracking system in a virtual procedure. In particular, the rigid medical device tracking system is operated with a corresponding ultrasound imaging system, although a different imaging system could also be used. A patient 1610 is lying in a supine position in a medical situation. An imaging system 1612 provides a multi-dimensional representation of the internal anatomy of the patient 1610 on a display device 1614. In particular, the ultrasound imaging system 1612 provides a three dimensional image of the patient's internal organs.

A rigid medical device tracking system 1616 is illustrated as a separate device that provides input to the ultrasound imaging system 1612 being used by the medical practitioner. In some embodiments, and as has been mentioned, the rigid medical device tracking system 1616 can be integrated with the imaging system. Some benefits of separately configuring the imaging system and the medical device tracking system as shown in FIG. 44 include reduced costs, greater configurability, and the opportunity to retrofit older and newer technologies together. Some benefits of integrating the imaging system and the medical device tracking system into a single apparatus include one-handed operation by a medical practitioner, economies of scale when producing technically complex medical devices, and medical cleanliness.

In the virtual procedure of FIG. 44, a virtual needle 1618 is used in cooperation with the medical device tracking system. According to the invention, the virtual needle 1618 includes a proximal base but does not include a conventional distal tip. Instead, the virtual needle 1618 includes only a base or "stub" portion. The virtual needle 1618 may be weighted like a conventional needle that is used in the procedure, and may otherwise feel like a real needle to the medical practitioner. When the virtual needle 1618 is manipulated by the medical practitioner in a simulation of the actual procedure, however, no part of the virtual needle 1618 is advanced into the body of the patient 1610.

The proximal base of the virtual needle 1618 includes at least one magnetic element, such as a permanent magnet 1620. In some cases, the magnet 1620 is located at very close or right at the end of the virtual needle 1618, as shown in FIG. 44. In other cases, the magnet is located some distance from the end of the virtual needle. In one embodiment and as has been mentioned, the magnet on an actual medical device used in connection with the present tracking system is located at some point on the needle (or any other suitable medical device) that is away from the distal end so as to preserve the shape, size, or other feature of the distal end of the device. In addition, it is appreciated that other technologies may be employed instead of magnetic detection, as has been described further above.

The virtual needle 1618 illustrated in FIG. 44 also includes a virtual distal tip. The virtual needle 1618 represents an actual needle having substantially known structural characteristics (*i.e.,* physical parameters). The substantially known structural characteristics may include length, caliber, gauge, diameter, curvature, coefficient of elasticity, sharpness, shape, taper, and the like. Accordingly, when the virtual needle 1618 is manipulated in the presence of the rigid medical device tracking system 1616 by the medical practitioner, a corresponding image of the represented actual needle can be displayed on the imaging system 1612 in cooperation with the anatomical image produced by the imaging system 1612. In this way, the virtual procedure may be conducted so as to inform the medical practitioner of where the tip of an actual needle will go in the patient as the medical practitioner watches the display device 1614. Subsequently, when the medical practitioner conducts the actual procedure, the practitioner will reasonably know that the actual needle will move in a particular direction and to a particular depth.

In some cases, the virtual needle only comprises a proximal base, and the manipulation of the virtual needle's non-existent distal tip is performed in cooperation with the imagination and procedural planning of the medical practitioner. In such cases, various representations of an actual needle can be rendered on the imaging system under the direction of an operator. In this way, the medical practitioner can select an actual needle having a desirable length, diameter, curvature, rigidity, and other characteristics suitable for the real procedure.

In other cases, the virtual needle may have a real physical structure, but the virtual needle is constructed in such a way that it is not actually introduced into the patient. Instead, the physical structure of the virtual needle is formed in such a way as to enable the practitioner to better visualize and practice the procedure. According to the invention, a virtual needle may be constructed of soft rubber so that the medical practitioner can determine and feel the amount and direction of pressure that will be applied to the actual needle during the actual procedure. Another example includes a virtual needle formed so as to be collapsible. Such a virtual needle permits the medical practitioner to accurately guide the virtual needle toward the target as if the device was actually advancing into the patient. During the virtual procedure, the medical practitioner can watch the image of virtual needle on the imaging system moving toward the target when in fact no real object is entering the patient at all.

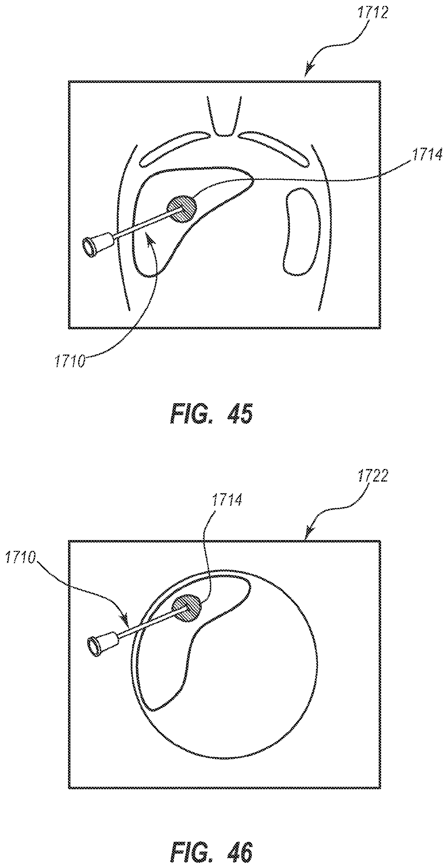

As one example of the foregoing, FIG. 45 shows a virtual image 1710 of a needle including selected structural characteristics is shown as overlaid on top of a CT image 1712 including a tumor image 1714. In FIG. 46, the virtual image 1710 of the needle including selected structural characteristics is shown as overlaid on top of an ultrasound image 1722 including the tumor image 1714.

According to some embodiments described herein, a medical practitioner can use a rigid medical device tracking system in cooperation with an imaging system to plan a medical procedure. Issues that arise in a virtual procedure can be considered and planned for in the actual procedure. Such issues include: a pathway to be followed, the length of rigid medical device, the caliber of rigid medical device, the ability or inability to get around interfering anatomical or pathological structures, the avoidance of the patient's essential structures, and the shape of rigid medical device (*e.g*., straight, curved, spiral, or another shape). In this way, for example, the medical practitioner can manipulate a virtual needle to see what will happen when a particular needle or other rigid medical device is used, before the actual device is placed into the patient.

The virtual planning procedures described herein, and other virtual procedures, include planning procedures for amniocentesis and accessing fetal structures in utero or ex utero including ventricles, renal collecting systems, bladder, heart, and others. The virtual planning procedures are also useful for abdominal paracentesis for fluid removal for diagnosis or therapy especially if adhesions are present, placing a tube into the gall bladder when strictures or adhesions are present, placing a tube into a pseudocyst of the pancreas, placing a tube into pleural or pericardial fluid, placing a tube into an abcess or other fluid collecting site, finding an osseous defect in a bone and guiding the device to this defect, and others.

In many cases, imaging systems provide representations in various colors. The rigid medical device tracking system in cooperation with the imaging system can also take advantage of color representations. In one example, a needle representation uses one color (*e.g.,* orange) for a virtual needle's image and a second color (*e.g.,* blue) for the actual needle's image. Thus, for example, with ultrasound, the virtual needle used for planning can be shown on the real time ultrasound image. Providing both a virtual needle representation and an actual needle representation can be useful to the medical practitioner to help guide the procedure.

In other representations of virtual and actual needles (*i.e.,* rigid medical devices), particular colors can be changed as the needle representation approaches particular structures inside the body of the patient. For example, the representation of the needle may be one color if it is determined that there will be no obstructions to the path that the needle is currently following. Then, if analysis of the imagery determines that the path of the needle is approaching a particular structure (*e.g*., a denser anatomical structure), the color of the representation of the needle may be changed. In some cases, the color change may be abrupt, and in other cases, the color change may be gradual. Other features include particular techniques to alert the medical practitioner such as text, audio, flashing, tactile feedback, and the like.

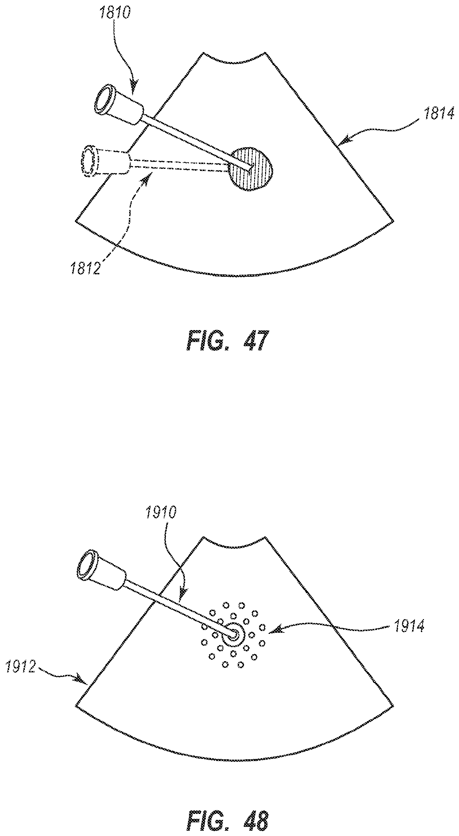

In FIG. 47, an ultrasound image is illustrated with both a virtual image overlay 1810 and a real time image overlay 1812. The virtual image overlay 1810 illustrates a representation of a rigid medical device (*e.g.,* a needle) created during the manipulation of a virtual needle. The real time image overlay 1812 illustrates a representation of a rigid medical device that is currently introduced into the patient. In some embodiments, the virtual needle image 1810 and the real time needle image 1812 include different colors, textures, or other visually recognizable features. In the embodiment of FIG. 47, the medical practitioner uses the virtual needle image 1810 to help guide the real rigid medical device into the patient's body via the real time needle image 1812.

In FIG. 48, an ultrasound image is illustrated having a virtual image 1910 of a rigid medical device (*e.g.,* a needle) introduced into a patient's body. In the embodiment of FIG. 48, an ultrasound image 1912 also shows a therapy indicator 1914, embodied as a virtual heating pattern displayed around the distal tip of the virtual image 1910 of the rigid medical device. The pattern may indicate a zone of heating (as here), radiation, freezing, or any other therapy. In some cases, the particular pattern may vary over time.

The embodiment of FIG. 48 may illustrate a planning procedure or an actual therapy. That is, in a planning procedure a medical practitioner may manipulate the virtual image 1910 of the rigid medical device and watch the ultrasound image 1912 on the display of a medical imaging system to see the virtual tip advance to the target. In the planning procedure, the medical practitioner can then view a simulation of the procedure and see particular details such as the size of therapy indicator 1914 under certain heating or freezing conditions (*e.g*., greater than 60 degrees Celsius), the time calculated for the zone of therapy to form to a certain diameter or area (*e.g*., using RF heating), and other details. Subsequently, an actual procedure can be performed by the medical practitioner with increased confidence after having performed the simulation.

In greater detail, the rigid medical device tracking system in one embodiment is used to perform a virtual procedure by manipulating just a proximal base of a rigid medical device. In the present embodiment, the rigid medical device includes a proximal base but does not include a distal end (which distal end would be present in an actual rigid medical device). In such embodiments, one or more magnets would be included on the proximal base of the rigid medical device, or "hub." In operation, the rigid medical device could be manipulated in a virtual procedure, prior to the actual procedure, so that a medical practitioner can see the effect of advancing a diagnostic or therapeutic device into a patient before the real device actually placed. In the virtual procedure, the medical practitioner can move the hub near to the patient in the desired orientation. As the hub is moved, the medical practitioner can observe on a display the representative image of the device tip as it travels in the patient to the simulated depth and angle amongst the intervening bones, organs, blood vessels, and other internal structures. Such virtual operations can be very effective for planning complex interventions (*e.g*., fetal puncture for diagnosis and therapy) and for improving safety and efficacy and reducing the time typically taken to perform the procedure.

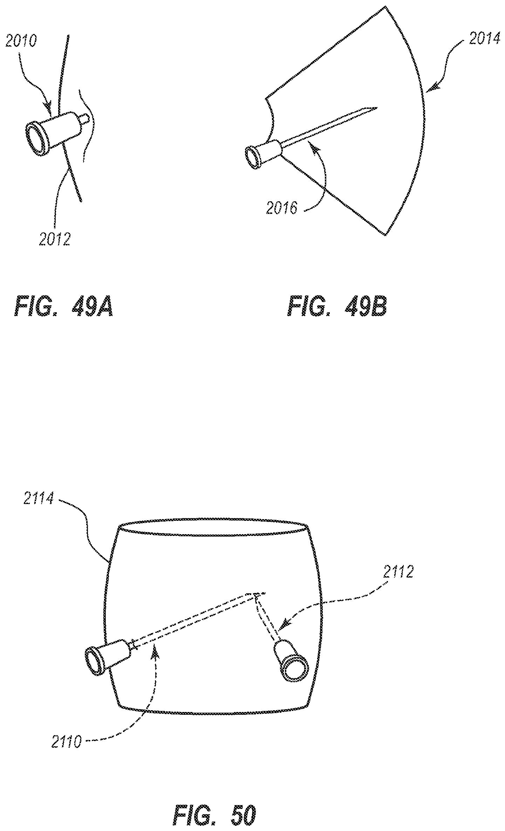

In FIGS. 49A-49B, a virtual rigid medical device 2010 is placed close to a patient 2012 so that a medical practitioner can anticipate what will actually happen when a real rigid medical device is inserted. FIGS. 49A-49B also illustrates a depiction or image 2014 on a display device of an imaging system, such as an ultrasound device, showing a simulated representation, or image 2016, or the virtual medical device 2010.

As has been discussed, the rigid medical device tracking system can be used in real time to introduce and place an actual rigid medical device into the body of a patient. In some cases, previously performed virtual procedures may permit the real time procedures that follow to be more effective and safe, faster and less expensive.

For example, after a virtual procedure is conducted where a virtual image representation of a rigid medical device has been tracked into a patient's body, an actual rigid medical device can be advanced on the virtual track previously determined. By integrating information from the rigid medical device tracking system with image data produced by the medical imaging system, the course of the rigid medical device can be seen on the display device. Observation of the display device permits the medical practitioner to follow the location of the tip of the rigid medical device with substantial accuracy. In one embodiment, the rigid medical device tracking system provides sufficient information so that the medical practitioner can be alerted to the particular location of the tip of the rigid medical device with various audio or visual cues (*e.g.,* flashing, contrasting colors, signal tones, and the like).

As described in some embodiments herein, a virtual procedure can be conducted in such a way that a virtual representation of a rigid medical device is tracked on a display device as the device is "virtually" introduced into a patient's body and advances to a target of particular concern. In some cases, the virtual representation is formed and stored as a sequence of still pictures and in other cases, the virtual representation is formed and stored as a "movie." The storage of the images that are generated during the virtual procedure may be stored in the rigid medical device tracking system, the imaging system, or any other suitable location.

A virtual procedure can also be used to train medical practitioners. For example, one medical practitioner, such as a radiologist, can perform a virtual procedure. The medical practitioner can use the virtual procedure to describe such things as diagnosis, therapy, and use of the medical devices, to a second medical practitioner. Subsequently, the first medical practitioner can guide the second medical practitioner during an actual procedure.

In some embodiments, one or more rigid medical device tracking systems can be used together to track one or more rigid medical devices. In other embodiments, different medical device tracking systems can be operated in proximity to the rigid medical device tracking system. For example, a rigid medical device tracking system as described herein can track two or more rigid medical devices and concurrently, a radio frequency (RF), radiographic, or other non-magnetic guidance system can track a different device in such a way that does not create interference.

In FIG. 50, a rigid medical device 2110 is being tracked in a patient's body 2114, and concurrently, another rigid medical device 2112 is also being tracked in the patient's body. In such embodiments, a particular target area can be envisioned by a medical practitioner and multiple methods of diagnosis or therapy can be employed to provide care to the patient. In other embodiments, more than two rigid medical devices may be tracked. For example, in some embodiments, the first rigid medical device 2110 is inserted into the target area, and then the second rigid medical device 2112 can be inserted. In some cases, the magnet from the first rigid medical device 2110 is removed after the device is located at the targeted location and before the second rigid medical device 2112 is tracked. In some embodiments, more than two rigid medical devices can be placed.

In the illustration of FIG. 50, the second medical device 2112 is being tracked with an RF tracking system, but a different system or several different systems can also be used as described further above in the present disclosure. For example, other techniques to provide imaging technologies also include ultrasound (*e.g*., A-mode, B-mode using mechanical sector scanning, electrical systems (phased or linear array), with or without three-dimensional reconstruction, and the like), x-ray (*e.g*., planar, biplane, three-dimensional, tomography, CT, angiography, and the like), MRI, PET, nuclear medicine, angiography, optical, RF, and others. In some cases, even pressure indicating imaging systems that indicate hardness or elasticity of tissue (*e.g*., by sensing pressure changes) such as systems used to detect breast masses or plaque hardness in the carotid artery can be used.

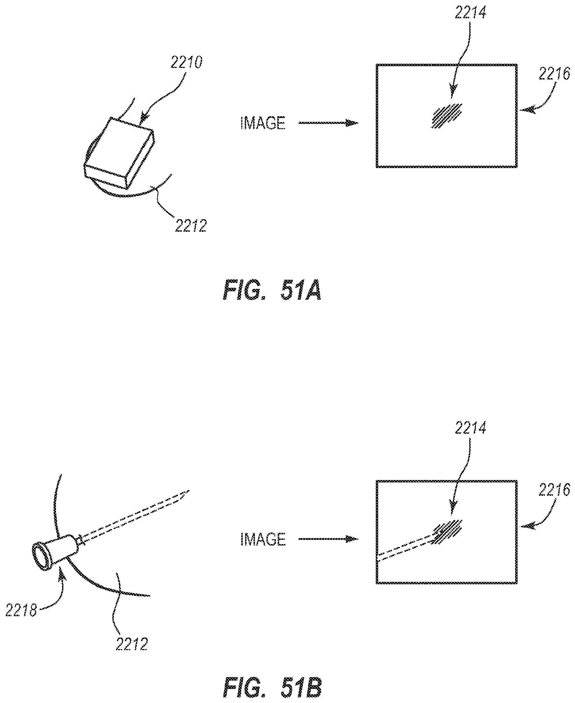

In FIGS. 51A-51B, an imaging device 2210 using a pressure sensitive technology (*e.g.*, pressure ink) is used to perform a diagnostic medical procedure on a patient's breast 2212. A representative outline of a breast mass 2214 is visible on a display image 2216 that operates in cooperation with the imaging device 2210. In a second part of the procedure, a rigid medical device tracking system is used to provide further diagnosis or therapy in the area of the breast mass.

As illustrated in FIGS. 51A-51B, in the second part of the procedure, a rigid medical device 2218 is introduced into the breast 2212. The rigid medical device tracking system provides tracking information representative of the path of the rigid medical device 2218 as it is manipulated and/or advanced. The representation of the breast mass 2214 produced by the imaging device 2210 in the first part of the procedure persists on the display image 2216 or is otherwise recalled and redisplayed in the display image. FIGS. 51A-51B show that the path of the rigid medical device 2218 is visible in the display image 2216 as it travels toward and into the area of the breast mass image that was previously produced.

In one embodiment, the rigid medical device tracking system can be used with imaging technologies that produce surgical maps and measurements. In such cases, the representative tracking information of a rigid medical device is linked with the surgical maps and measurement information in images that are output to a display device.

In one embodiment, the rigid medical device tracking system can be used with vibrometry-based imaging technologies. In such cases, the representative tracking information of a rigid medical device is linked with data produced by the vibrometry system.

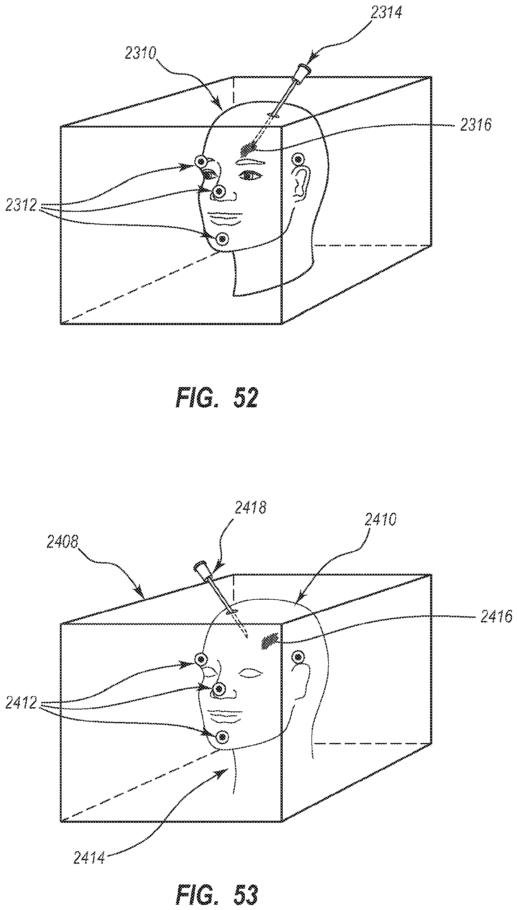

In FIG. 52, an imaging device is used to produce a three dimensional representative image of part of a patient's anatomy. In FIG. 52, a series of fiducial markers can be placed on the patient's anatomy allowing one to construct the three dimensional image with computed tomography (CT), ultrasound, or some other methodology. Such an image would typically be produced before the intervention procedure.

In some cases, the fiducial markers might be placed inside the patient's anatomy. Such fiducial markers can be used visually or in cooperation with particular medical devices such as ultrasound, x-ray, and others.

In FIG. 52, a patient's head 2310 is shown with fiducial markers 2312. In the present embodiment, a medical practitioner can place a needle or other rigid medical device 2314 into the patient while marking the fiducial markers 2312, thus allowing a superimposition of the real time rigid medical device 2314 tracking onto the three dimensional image. Also shown in FIG. 52 is a mass 2316, which is an area of interest in the patient.

In some embodiments, image generation and the provided guided therapy are generated with the same methodology. In other embodiments, the three dimensional image is generated with one methodology, *e.g.,* CT, and the rigid medical device used to provide therapy is tracked with another methodology, *e.g.,* ultrasound. For example, in a first procedure, a first image based on the configuration shown in FIG. 52 is generated with the fiducial markers 2312 marked. Subsequently, a needle is inserted and tracked in real time on a second image that has been superimposed onto the prior first image. The first image can be produced by CT and the real time second image can be produced by ultrasound. For each technique, the fiducial markers 2312 are sufficiently clear to a medically acceptable level.

In the embodiments described above, combinations of systems are shown to advantageously provide therapy to patients. In addition to using the fiducial markers to overlay the rigid medical device tracking system, a medical practitioner could also use the fiducial markers to overlay an image from a second imaging technique onto the image from first imaging technique, *e.g.,* different combinations might allow an ultrasound image to be superimposed onto a CT image. The CT image in this case would be "remote" time-wise and the ultrasound image would be considered "real-time" time-wise. The use of fiducial markers helps to provide confidence to the medical practitioner that the two imaging systems are appropriately overlapped.

In addition, more systems operating according to particular imaging technologies could also be used, such as ultrasound imaging atop CT and/or MRI images, for example. In one case, the medical practitioner might use ultrasound imaging at the time of the CT to define how the ultrasound definition points appear on the CT two dimensional or three dimensional images. Subsequently, during the actual procedure, the medical practitioner could use ultrasound with substantial confidence to recognize how the ultrasound relates to the CT obtained before the procedure. In such cases, the fiducial markers used can include skin fiducials as well as osseous or other fiducials.

In another example, the rigid medical device tracking system can be used to improve real-time mammography. In one embodiment, a mammogram is correlated with a hardness imaging system. The combination is advantageous to the patient because the combination helps the medical practitioner understand where a mammography abnormality is with respect to the hardness image. Subsequently, the medical practitioner could use the hardness image in real time to perform a biopsy procedure enabled by a rigid medical device tracking system.

In another embodiment, a directional device (*e.g.,* a compass) could be employed to establish the angle of an ultrasound image onto a CT scan image from two different angles. Such combination of images would typically be done prior to a therapy procedure. Subsequently, during the actual therapy procedure, the medical practitioner can use the previously generated three dimensional images to provide information regarding the direction to aim the ultrasound. When properly aimed, an ultrasound image can be superimposed onto the CT scan with substantial accuracy.

Accordingly, in the described embodiment, the real time ultrasound imaging system is used during the preliminary anatomic study (*e.g*., three dimensional CT) so that during the real time therapy procedure, the ultrasound procedure can be used just as it was in the preliminary procedure. The combination of particular imaging systems increases the likelihood of the ultrasound image registering correctly on the CT image and further increases the accuracy of rigid medical device tip placement as directed by a rigid medical device tracking system. In such an embodiment, the real-time ultrasound image helps the medical practitioner guide the tip of the rigid medical device 2314 to the mass 2316 as represented on the CT scan.

In other embodiments, one or more diagnostic or pre-therapy images can be generated prior to a subsequent diagnostic or therapy procedure. The diagnostic or pre-therapy images can be one dimensional (*e.g*., by ultrasound mode A) or two- or three-dimensional (*e.g*., x-ray, ultrasound, nuclear medicine, MRl, CT, PET, or the like). Subsequently, with fiducial markers, a rigid medical device tracking system can generate information for real time images that are superimposed onto the diagnostic or pre-therapy images. In these embodiments, only the imaging generated by the rigid medical device tracking system is produced in real time during the therapy procedure.

In FIG. 53, an image 2408 depicts an image 2410 of a patient's head together with images of detected fiducial markers 2412. The images illustrated in the embodiment of FIG. 53 are generated with information from four imaging systems, however, more or less than four imaging systems may be used in other embodiments. In the embodiment of FIG. 53, a pre-therapy CT image 2414 is illustrated with superimposed PET images 2416 of a tumor and fiducial markers 2412. In addition, A-mode ultrasound information is also integrated into the pre-therapy images. During real time operation of the therapy procedure, FIG. 53 illustrates information from a rigid medical device tracking system including a image 2418 of a needle being guided toward the tumor.

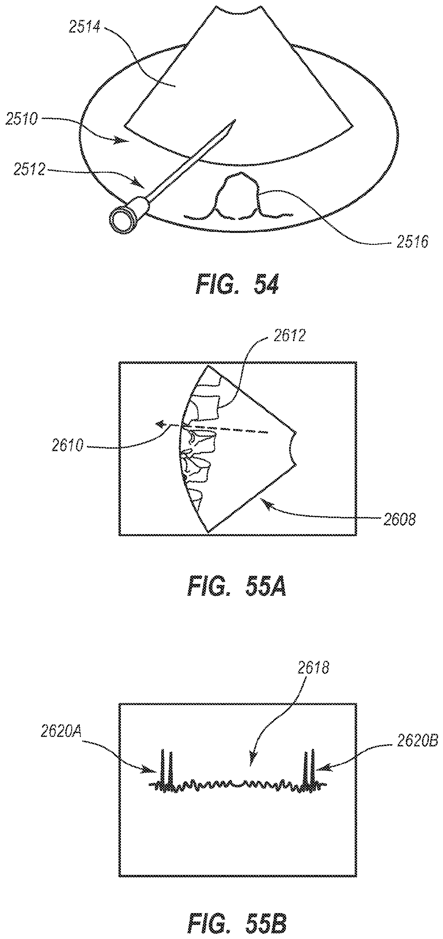

FIG. 54 illustrates another embodiment wherein imaging information from one or more medical imaging systems is used to produce a representative view of the structures inside a patient's body. In the embodiment, the imaging information may be perceived from one direction while a rigid medical device tracking system helps a medical practitioner guide a rigid medical device from a different direction. For example, in the embodiment of FIG. 54, a medical practitioner performing a procedure on a patient's spine can view soft tissue 2510 in an ultrasound image area 2514, such as the anterior abdomen, yet place a rigid medical device 2512 (*e.g*., needle) through paraspinal muscles proximate vertebrae bone 2516 via imagery generated with the rigid medical device tracking system.

In some embodiments, contrast enhancement agents may be cooperatively used with the medical imaging systems. Such agents, which can be used with x-ray, ultrasound, PET, MRI, nuclear medicine, and other imaging systems, increase the viewability of the particular images generated by the medical imaging systems.

Some embodiments of the rigid medical device tracking system may employ motion compensation algorithms. Such embodiments include compensation for respiratory movement, cardiac movement, GI motility, and other functions. For example, in a cardiac procedure, the motion compensation algorithms may be triggered on the P wave phase of a cardiac cycle and thereby correct for cardiac movement. Such compensation may allow access by the medical practitioner to coronary arteries, aorta, carotids, and other related anatomy. Other examples in pulmonary system procedures may compensate for respiration. Still other examples may apply to the abdominal organs (*e.g.,* to compensate for respiration pushing down on the diaphragm and GI motility), intestinal tract, muscular system, and any other parts of the patient's anatomy.

The rigid medical device tracking system, as described herein, may be used for particular study and therapy of many different organs. In fact, the rigid medical device tracking system can improve many conventional medical procedures. Some advantages provided by use of the rigid medical device tracking system include reducing patient discomfort, reducing procedure cost, and reducing procedure time.

Examples of pulmonary procedures that may receive the benefits of the rigid medical device tracking system described herein include draining pleural fluid or blood, draining an abscess, inserting a needle into a pneumothorax, biopsy of a mass in the pleura or lung, placement of a device in the trachea for tracheostomy (*e.g*., using A-mode ultrasound), and accessing a pulmonary artery to extract or dissolve a pulmonary embolism clot, and others.

Examples of ear, nose, and throat (ENT) procedures that may receive the benefits of the rigid medical device tracking system described herein include biopsy of a mass, biopsy of lymph nodes, insertion of a catheter into a duct (*e.g*., salivary), treatment of tumors with radiation therapy (RT), heat, and the like, insertion of devices to reduce snoring, and others.

Examples of neurosurgical procedures that may receive the benefits of the rigid medical device tracking system described herein include lumbar peritoneal (LP) procedures when it is difficult to place a needle into the sub-arachnoid space in the lumbar spine, creation of a small hole to insert a device into a mass in the brain or to drain fluid or blood above or below dura (*e.g*., by mechanical sector ultrasound scanner or A-mode ultrasound) for diagnosis or therapy, treatment of a variety of neurological diseases via guidance surgery such as to a herniated disk, and others.

In FIGS. 55A-55B, details of a procedure to perform a difficult lumbar puncture are shown. In particular, FIG. 55A shows that a B-mode ultrasound image 2608 is used to locate a path 2610 for a needle between various bone structures 2612. Correspondingly, FIG. 55B shows details of an A-mode ultrasound image 2618 that is used to locate a path for a needle, including a skin signal 2620A and a dura signal 2620B that indicates the presence of dura within the patient body. These figures therefore show that information from a rigid medical device tracking system can be used cooperatively with images produced by an ultrasound medical imaging system to allow a medical practitioner to perform a procedure, such as a difficult lumbar puncture.

Examples of cardiology procedures that may receive the benefits of the rigid medical device tracking system described herein include procedures to access heart tissue, procedures to access heart chambers (RA, RV, LA, LV), procedures to access coronary arteries, procedures to access pericardial space for fluid assessment, fluid removal, and other pericardial space therapy, and others. In some embodiments, motion correction and/or motion compensation may be used.

Examples of vascular procedures that may receive the benefits of the rigid medical device tracking system described herein include procedures to access an aorta and other major vessels such as carotid, vertebrals, femoral, popliteal, brachial, and coronary arteries, procedures to assist in arterial puncture for diagnosis (*e.g*., to draw arterial blood or to start an arterial line), procedures for therapy to introduce drugs or fluids into an artery, procedures for arterial puncture to allow access to another area of the patient such as a bleed from a separate arterial puncture, and other vascular procedures that include accessing veins for diagnosis and therapy.

Examples of gastrointestinal procedures that may receive the benefits of the rigid medical device tracking system described herein include procedures to drain a pancreatic cyst (pseudocyst or real cyst in a pancreas or peripancreatic location), procedures to diagnose and drain other cysts, biopsy of the liver, biopsy of a liver mass, biopsy of a gall bladder, draining of a gall bladder for decompression, placement of a device to dissolve or disrupt gall stones, procedures to access hepatic duct, hepatic vein, or hepatic artery, procedures to assist non invasive placement of a portocaval shunt, procedures to drain a liver cyst, and procedures to assist placement of a PEG tube with fluid inside a gastric lumen so that ultrasound can see the stomach lumen (*e.g*., placement of a contrast agent fluid into the stomach so ultrasound imaging can suitably image the gastric lumen). Subsequently, a needle can be guided magnetically to enter the stomach as seen on an ultrasound display to then accomplish a PEG procedure. Such procedures do not require inflation of the stomach with air, endoscopy, or x-ray). Other suitable gastrointestinal procedures include procedures to place a tube into a jejunum, colon, cecum for percutaneous cecostomy or colostomy, etc., procedures related to peritoneal fluid drain (a particular problem with adhesions or masses) so as to avoid hitting a mass, an aorta, and other structures, and other procedures.

Examples of genito-urinary (GU) procedures that may receive the benefits of the rigid medical device tracking system described herein include placement of a drain into a bladder using A-mode or B-mode ultrasound, x-ray, CT, MRI or some other imaging mechanism, placement of a tube into a renal collecting system with ultrasound, intravenous pyelogram (IVP), x-ray guidance or other suitable imaging mechanism, draining fluid in the urinary tract, biopsy in the urinary tract, injection of therapy in the urinary tract such as chemotherapy of tumors, procedures to guide renal biopsy of mass, and others.

Examples of gynecological procedures that may receive the benefits of the rigid medical device tracking system described herein include procedures to access a cyst in an ovary to drain fluid or biopsy for diagnosis, placement of a drain in a cyst, procedures to target an ectopic pregnancy and treat with sclerosis, heat, or another therapy, procedures to access an ovary to remove an egg for in vitro fertilization (IVF), procedures to target an ovary for treatment of cyst or neoplasm, procedures to access a uterus for biopsy, procedures to access a uterus for insertion of a therapy device to treat fibroid with heat or to treat fibroid with sclerosis to shrink the fibroid, procedures to drain fluid or blood in a peritoneal cavity for diagnosis and therapy (particularly adhesions or a loculated mass), procedures to guide amniocentesis in a pregnant patient (*e.g*., A-mode or B-mode ultrasound), procedures to access or puncture an umbilical cord, procedures for intervention in a fetus's bladder, ventricles (cNS), heart, or other organs, and other procedures.

Examples of orthopedic procedures that may receive the benefits of the rigid medical device tracking system described herein include procedures to locate and access a screw hole, procedures to locate and access or place a screw, procedures to aspirate a bone cyst, procedures to biopsy and treat a tumor, procedures to access and repair a herniated disk, procedures to assist arthroscopy of a shoulder, a hip, a knee, an ankle, digits, procedures to assist anesthesia and to place a scope, procedures to assist with insertion of new materials into a joint such as cartilage, procedures to inject a contrast agent for x-ray, CT, MRI, or another imaging system for diagnosis of joint health (*e.g*., arthrogram), procedures to aspirate fluid from a joint (particularly if adhesions or a mass are present and it is difficult to locate and access the fluid), procedures to insert new support or bone growth stimulating materials into a non-healing fracture or cyst, and others.

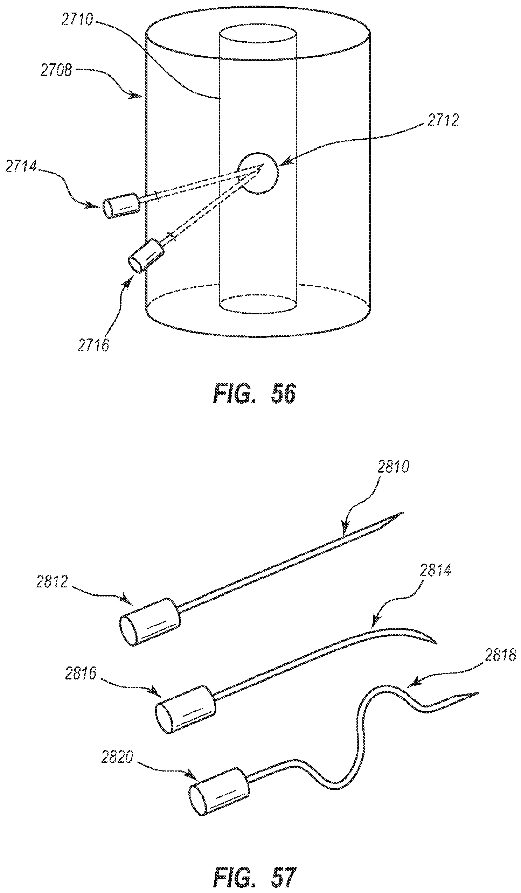

In FIG. 56, an orthopedic procedure is illustrated. In particular, an image 2710 of a bone is illustrated in a medical imaging system image 2708. The bone image 2710 includes an image 2712 of an observable bone defect 2712. In a first virtual phase of the procedure, a virtual rigid device, represented by a virtual device image 2714, is manipulated and virtually advanced to bone defect image 2712. The path and depth of the virtual rigid device is represented by the image 2714 on a display device of the medical imaging system. In a second real time phase of the procedure, a real rigid device, represented by a real device image 2716 is advanced along the same path as the virtual rigid device. During the second real time phase, the virtual path can be generated or maintained on the display device in a different color, pattern, or other contrasting way so that the virtual image can be separated visually from the real time image.

Examples of muscular skeletal procedures that may receive the benefits of the rigid medical device tracking system described herein include procedures to access, diagnose (*e.g*., biopsy, cytology), and/or treat a mass with local radiation, heat, chemotherapy, or some other therapy, procedures to study a ligament or a tendon, procedures to place an electrode for conduction studies, and others.

There are many general surgery procedures that may receive the benefits of the rigid medical device tracking system described herein. These procedures include acts to treat a gunshot, shrapnel, or other foreign object wounds (*e.g*., locating and removing a bullet or fragment wherein the foreign object is seen by an imaging technique, intervening anatomy is seen by an imaging technique, and a needle, probe, or forceps tip is guided to the afflicted area by the rigid medical device tracking system). Other procedures include those that treat Crohn's disease by defining fistulous tracts *(e.g.,* a device is passed into a fistula and when the tip is located and the probe fails to pass further in, the origin of the fistula has been located), and additional procedures for removing foreign bodies or mesh.

Additional examples of general surgery procedures that may receive the benefits of the rigid medical device tracking system include procedures to direct a tube to an abscess or cyst for initial drainage and for placement of drains for longer drainage, and procedures to biopsy or remove a breast mass (*e.g*., using ultrasound, mammography, a hardness image, an elastomeric study overlying an image, or some combination of imaging techniques to guide placement of a rigid medical device, and to confirm that the tip of the device is in the target area). Other examples include procedures for lymph node biopsy (*e.g*., use of ultrasound, nuclear medicine, PET, x-ray, CT, MRI or some other imaging system alone or in combination, to locate a lymph node). In the lymph node biopsy procedure, a variety of agents can target the lymph node and carry an imaging agent to the lymph node, and the rigid medical device tracking system can confirm placement of a rigid medical device into a lymph node. The rigid medical device tracking system can also be used for therapy by injection of chemotherapy or other chemicals to treat abnormal lymph nodes.

Examples of ophthalmological procedures that may receive the benefits of the rigid medical device tracking system described herein include procedures to locate structures in the retina, lens, and other parts of a patient's eye with A-mode or B-mode ultrasound, x-ray, CT, MRI, and other imaging systems and subsequently use the rigid medical device tracking system to reach the target structure while not being advanced too far and injuring the structure or eye, and other ophthalmological procedures. Such procedures may be useful in diagnosis and treatment of retinal diseases, diseases of the lens, and other eye related maladies.

Examples of anesthetic procedures that may receive the benefits of the rigid medical device tracking system described herein include procedures to locate nerves with A-mode or B-mode ultrasound, x-ray, nuclear medicine imaging CT, MRI, PET, and other imaging systems alone or in combination and subsequently use the rigid medical device tracking system to track the tip of a rigid medical device entering the target area (*e.g.,* for nerve block), placement of epidural blocks (*e.g.,* via lumbar puncture (LP) shunt), and other anesthetic procedures.

In the rigid medical device tracking system described herein, a variety of rigid medical devices may be cooperatively used. The rigid medical device tracking system is provided with particular information regarding the physical parameters of the associated rigid medical devices that are to be tracked. Cooperatively, the rigid medical device tracking system applies information representative of the location of one or more magnets associated with the rigid medical device to particular algorithms in order to identify the location of the tip of the rigid medical device with substantial precision as has been described further above. The physical parameter information is also used in the generation of images representing the shape, location, and path of the rigid medical device as it is manipulated in a patient's body.

FIG. 57 illustrates three non-limiting embodiments of rigid medical devices that can be tracked with the rigid medical device tracking system. A first illustrated rigid medical device is a straight rigid medical device 2810 including at least one magnet 2812 on its proximal end. A curved rigid medical device 2814 is also illustrated including at least one magnet 2816 on its proximal end. A spiral rigid medical device 2818 is further illustrated including at least one magnet 2820 on its proximal end. The rigid medical devices illustrated in FIG. 57 have a non-limiting variety of physical parameters including length, caliber, gauge, diameter, curvature, coefficient of elasticity, sharpness, shape, taper, and the like. In addition, the rigid medical devices do not have to be constructed of a shape, size, or material that can be detected by a medical imaging system.

In some cases, the rigid medical devices of the types illustrated in FIG. 57 are malleable in one configuration and rigid in another configuration. Such devices may be used in procedures where a medical practitioner advances a medical device to a target that is obstructed by particular tissue, bone, or some other structure. The target is not preferably accessible from the entry point outside of the patient in a straight line to the target. In such cases, the rigid medical device may be malleable in a first condition permitting the medical practitioner to advance the device around the obstruction. Subsequently, the device may be made sufficiently rigid in a second configuration such as illustrated in FIG. 57 or in some other shape. In the cases where the rigid medical device may be made malleable, the rigid medical device tracking system is operable to determine the position of the device in the second configuration when the device is sufficiently rigid.

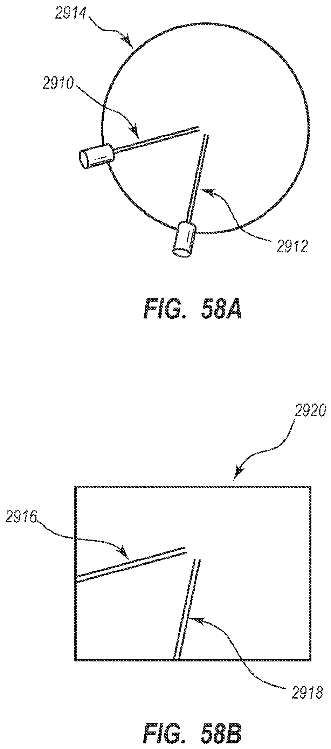

It is appreciated that in one embodiment the rigid medical device tracking system described herein can track one or more rigid medical devices. FIGS. 58A-58B illustrate an example of this, wherein a rigid medical device tracking system tracks two separate and distinct rigid medical devices 2910 and 2912. In some cases, information for imaging a plurality of devices is used to provide images of multiple rigid medical devices on a display device. In some cases, the images are interlaced into a single, composite video stream, and in other cases the images are rapidly and alternately multiplexed to the display device.

In the embodiment of FIGS. 58A-58B, an area of a patient's anatomy 2914 is undergoing a procedure. The display 2920 of a medical imaging system shows a representation of the structures inside the patient. A first rigid medical device 2910 is advanced into the anatomy 2914, and an image 2916 of the first rigid medical device is shown on the display 2920. Subsequently, a second rigid medical device 2912 is advanced into the anatomy 2914, and an image 2918 of the second rigid medical device is also shown on the display 2920.

In the rigid medical device tracking system of FIGS. 58A-58B, the images 2916 and 2918 of the two rigid medical devices are displayed in an interlaced pattern, e.g., "ABABAB." That is, the image 2916 of the first medical device is displayed, and then the image 2918 of the second medical device is displayed, and the pattern repeats. Thus, in the embodiment of FIGS. 58A-58B, the two images are iteratively displayed.

In some embodiments, the tracking of a plurality of rigid medical devices is accomplished in a series of sequential procedures. For example, the rigid medical device tracking system first records a first baseline magnetic profile. Next, the system tracks a rigid medical device. Then, the rigid medical device tracking system records a second baseline magnetic profile, which differs from the first baseline magnetic profile by the inclusion of effects by the rigid medical device. Finally, the system tracks another rigid medical device. Tracking rigid medical devices as a series of sequential procedures permits a medical practitioner to track a plurality of rigid medical devices of nearly any shape and size. In one embodiment, different sets of sensors are employed to independently track each medical device.

In other embodiments, the rigid medical device tracking system tracks two or more rigid medical devices concurrently. In such embodiments, the procedures of recording static magnetic profiles and then tracking the movement of a rigid medical device are interlaced or performed in some other manner. In some cases, a single rigid medical device tracking system tracks a plurality of rigid medical devices and provides input to a medical imaging system. In some cases, two or more rigid medical device tracking systems are used to track rigid medical devices and provide input to a medical imaging system.

In some embodiments, a "u" shaped rigid medical device can be tracked. For example, the u-shaped rigid medical device may also have an oversheath. In such a device, the physical parameters of the u-shaped rigid medical device are known, and an appropriate image can be generated on a cooperating display device. The u-shaped rigid medical device can be manipulated to approach a target from the opposite side as the u-shaped rigid medical device entered the patient's body. The rigid medical device tracking system can track the location of the distal tip of the rigid medical device based on information derived from the position and orientation of the one or more magnets on the proximal end of the rigid medical device.

Similarly, in other embodiments, a "grappling hook" shaped rigid medical device may be tracked and manipulated into position. The grappling hook-shaped rigid medical device can be tracked and manipulated and then set into position inside the patient's body.

Once set, a u-shaped or grappling hook-shaped rigid medical device can be used in cooperation with over-tubes and other potentially multi-lumen devices. For example, such rigid medical devices can be used in endoscopic and laparoscopic procedures. The u-shaped or hook device can be used to stabilize the tip of a rigid medical device, and over-tubes or multi-lumen devices can be placed.

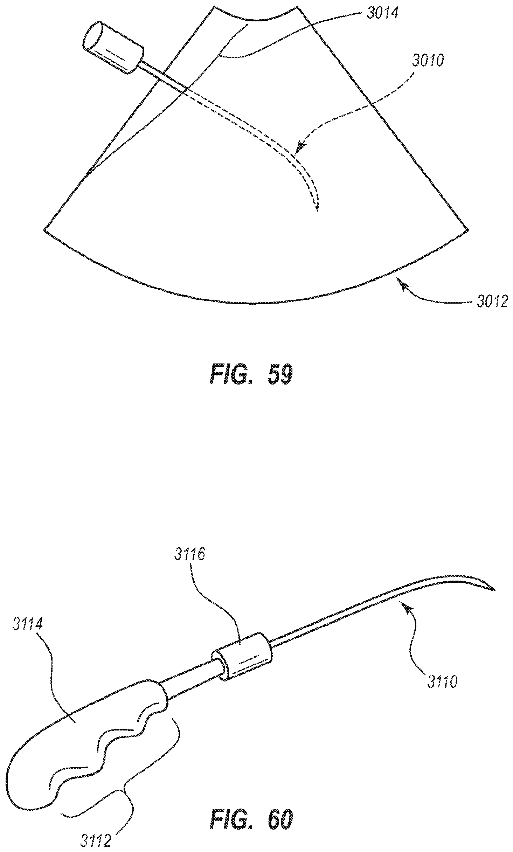

FIG. 59 illustrates the tracking of a curved rigid medical device 3010 with an ultrasound medical imaging system. Particularly, the rigid medical device 3010 is inserted into the skin 3014 of the patient within an ultrasound imaging area 3012 such that an image of the device can be displayed on a display device. The device image can then be tracked as the rigid medical device 3010 is advanced and manipulated. In the embodiment of FIG. 59, the rigid medical device tracking system receives or otherwise possesses information about the physical parameters of the curved rigid medical device 3010 such as its shape, diameter, and length. The physical parameters are used in one or more algorithms to provide information that enables display of the representative image of the rigid medical device 3010, as has been described.

FIG. 60 illustrates a rigid medical device 3110 including certain ergonomic features. In the rigid medical device 3110 illustrated, particular finger recessions 3112 are integrated into the proximal end of the device. The finger recessions 3112 illustrated in FIG. 60 are non-limiting and any other ergonomic features can also be incorporated, such as oversized gripping features, shaped features that assist with manipulation of the rigid medical device and/or provide orientation information, non-slip features, color coded features, left or right handed features, and many others. In one embodiment, visual or other cues can be provided on the handle of the medical device to enable a clinician to determine the particular orientation of curved or other non-linear features included on the distal portion of the device. For instance, a label, arrow, clocking feature or other indicia can be included on the handle or other portion of the medical device to assist with determination of the orientation of the distal portion of the device.

In some cases, the rigid medical device may be radio-opaque, "radio-invisible," "echo-dense," anechoic, sonolucent, or otherwise resistant to detection with a medical imaging system. That is, the rigid medical device may be only moderately detectible with the medical imaging system or may not be detectible at all. For example, the rigid medical device may be constructed of plastic, metal, fiberglass, or any other suitable material that is not readily detectable with a conventional ultrasound, x-ray, or other medical imaging system. In such embodiments, the one or more magnets on the proximal end of the rigid medical device provide sufficient information to the rigid medical device tracking system so that a representative image of the rigid medical device can be integrated with other images of the medical imaging system, and the rigid medical device can be tracked with substantial accuracy.

In some embodiments, the rigid medical device includes other features to provide information to a medical practitioner or even to assist in tracking. In some embodiments, the rigid medical device includes features that determine how the device will relate/react to the tissue it comes in contact with. For example, the rigid medical device may measure pH, ECG tracing, pressure, oxygen content, temperature, partial pressure of oxygen (pO2), partial pressure of carbon dioxide (pCO2), a specific chemical or biological marker, or something else.

In some embodiments, the rigid medical device can serve as an electrode. In this way, when the medical practitioner is placing a needle or other device into an amniotic sac, an ECG tracing feature, for example, will note if the fetus is touched.

In still other embodiments, the rigid medical device has functions that include sensing pressure for vascular studies, using electrodes to differentiate fluids (*e.g.,* blood from cerebrospinal fluid), and other functions.

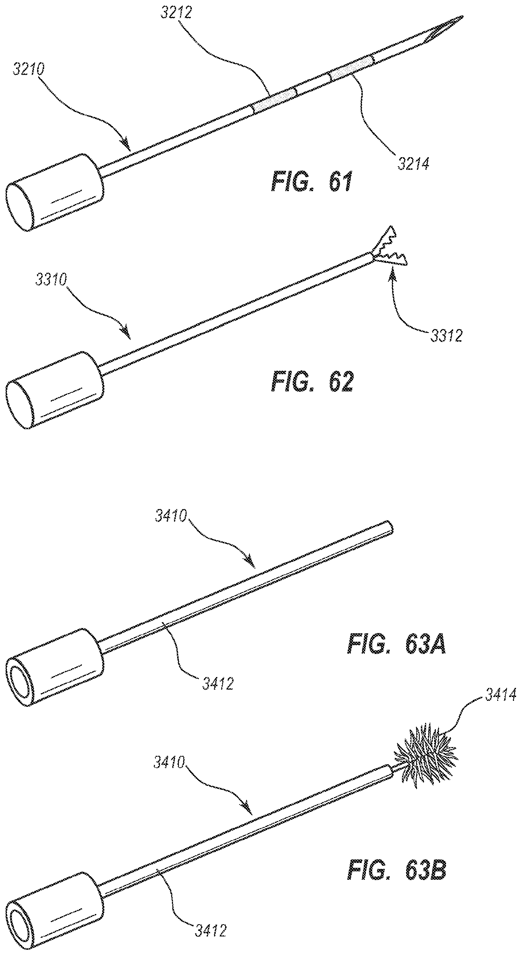

FIG. 61 illustrates a rigid medical device 3210 including components for performing multiple functions. In detail, the rigid medical device 3210 is hollow and includes a pressure sensor 3212 and an electrode 3214 integrated therewith. During a procedure involving a pregnant patient, the device can facilitate detection of the fetal heart beat when the rigid medical device is near the fetus. Additionally, the illustrated rigid medical device 3210 can also be used for therapy. For example, when a diagnosis is made, and when the tip of the rigid medical device 3210 is determined to be at a target location, the same device can deliver therapy (*e.g.,* a tube to drain a fetal bladder, a tube to deliver various types of liquid therapies, a device to deliver various types of therapy for tumors such as heat, cold, radiotherapy, microwave, laser, and the like, and many others).

Rigid medical devices that can be used with the rigid medical device tracking system described herein many have many shapes, sizes, functions, and associated accessories. For example, FIG. 62 illustrates a rigid medical device 3310 configured as a device including jaws 3312 for use in a biopsy, for example. FIGS. 63A-63B illustrate a rigid medical device 3410 including both a sheath 3412 and an integrated brush 3414 that is withdrawn into the sheath 3412 when not in use and extended out of the sheath when in use. Other functions that may be enabled with a sheathed instrument as shown in FIGS. 63A-63B, including biopsy devices, cystology devices, biomarker devices, and others.

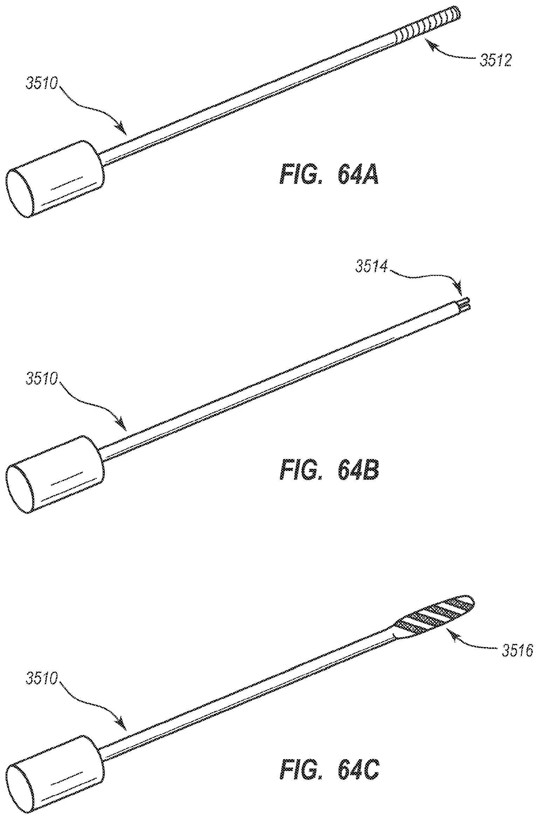

Still other non-limiting functions that may be integrated into a rigid medical device 3510 are illustrated in FIGS. 64A-64C, including an electrocoagulation monopolar electrode 3512, an electrocoagulation bipolar device 3514, and an electrocoagulation multipolar device 3516 with several poles oriented in a barbershop pole pattern. Still other non-limiting functions that may be integrated into a rigid medical device include cryotherapy functions including multiple channels in the rigid medical device, microwave antenna functions, laser waveguide functions for Argon, Nd:YAG or other lasers, double lumen tube functions for application of tissue glue, and a radiotherapy catheter functions that have a specially tipped catheter that emits radiotherapy. Other non-limiting functions may include a caliper configuration that is useful for measuring with substantial precision the length, width, diameter, or volume of a targeted area.

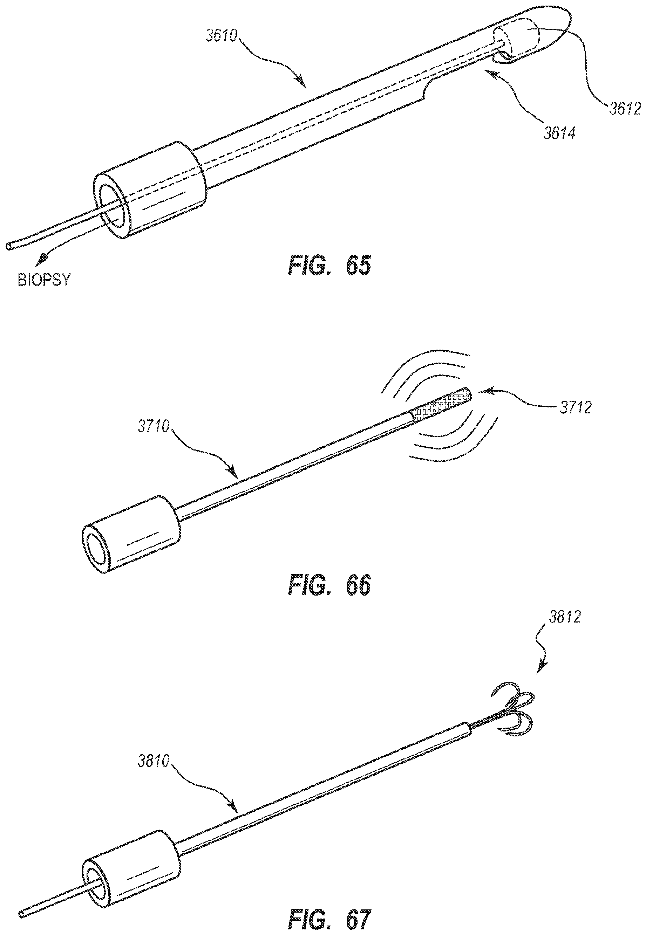

FIG. 65 illustrates a suction biopsy tube 3610 that can be integrated into a rigid medical device for cooperative use with a rigid medical device tracking system. The suction biopsy tube 3610 of FIG. 65 includes a blade 3612 and an opening 3614 such that tissue can be cut and vacuously suctioned into the opening 3614. In such a suction biopsy tube 3610, one or more biopsy samples may be cut and drawn outside the patient during a single procedure. In other embodiments, different biopsy procedure devices can be constructed as a rigid medical device. For example, spiral cutting biopters, boring biopters, hollow needle biopters, and the like can be included. Such biopsy devices can collect tissue samples via automatic, spring loaded, mechanically operated, or some other type of collection action.

FIG. 66 illustrates a heater probe 3710 including an integrated heating element 3712 for cooperative use with a rigid medical device tracking system and for directly heating a targeted area.

FIG. 67 illustrates an anchor tube 3810 including an integrated anchor 3812 as yet another example of a rigid medical device for cooperative use with a rigid medical device tracking system and for anchoring into a targeted area. Once anchored by the anchor 3812, the anchor tube 3810 can be used to direct other devices or therapies directly to the targeted area. In some embodiments, the anchoring mechanism is configured into a different type of rigid medical device. In some embodiments, the anchor tube may have an over-tube to assist advancement and placement of the device wherein the anchor may be retracted into the over-tube for part of a procedure and advanced out of the over-tube for part of the procedure.

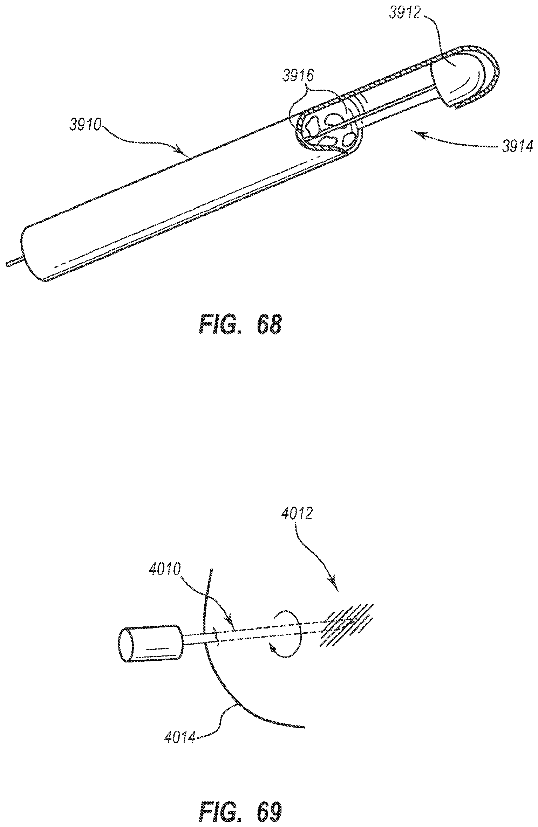

FIG. 68 illustrates a multiple biopsy tube 3910 integrated into a rigid medical device for cooperative use with a rigid medical device tracking system. In the embodiment of FIG. 68, the multiple biopsy tube 3910 includes a blade 3912 and an opening 3914 configured to enable multiple biopsy samples 3916 to be drawn into the tube tip. In some embodiments, the multiple biopsy tube 3910 has automated functionality, such as in the biopsy device described herein with respect to FIG. 65.

FIG. 69 illustrates a large biopsy tube 4010 capable of tissue removal for therapy integrated into a rigid medical device for cooperative use with a rigid medical device tracking system. In the embodiment of FIG. 69, the large biopsy tube 4010 can penetrate the skin 4014 of the patient and draw in a sample from a suspected tumor 4012 for biopsy, which can be further analyzed (e.g., by frozen pathological section). Based on results of the biopsy, the medical practitioner can remove all of the targeted tissue using the large biopsy tube 4010, if desired.

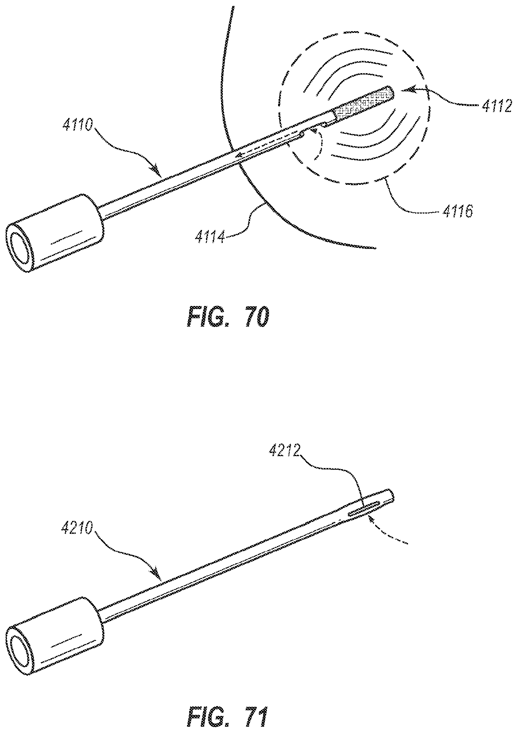

FIG. 70 illustrates another large biopsy tube 4110 integrated into a rigid medical device for cooperative use with a rigid medical device tracking system. In the embodiment of FIG. 70, the large biopsy tube 4110 can penetrate the skin 4114 of the patient and draw in a sample for biopsy. The biopsy tube 4110 while still in place within the patient can further facilitate the application of various therapies including direct tissue removal or application of particular therapies including chemotherapeutic agents, cold, heat (*e.g.,* direct heat, monopolar heat, bipolar heat, multipolar heat), laser, photodynamic dyes, microwave, radiation therapy via a catheter, and other therapies. In the illustrated embodiment, the large biopsy tube 4110 includes a heating tip 4112 for providing heat therapy to a mass 4116 or other tissue in question. In some cases, a targeted volume of tissue can also be broken up and removed partially or completely via suction or some other method.

FIG. 71 illustrates an ultrasound imaging probe 4210 integrated into a rigid medical device for cooperative use with a rigid medical device tracking system. In the embodiment of FIG. 71, the ultrasound imaging probe 4210 includes a small, high frequency ultrasound transceiver 4212 for imaging. Images generated by the ultrasound transceiver 4212 can be forwarded to and depicted by a display device. The ultrasound imaging probe 4210 can spin manually, mechanically, automatically, or by some other means. In addition, the ultrasound imaging probe 4210 can be moved in and/or out to provide ultrasound information for use by the ultrasound imaging system. The ultrasound probe can also be electronic. For example, the ultrasound probe can be configured as a linear array or phased array. The ultrasound probe can be a side or end oriented "A" mode device or some other configuration, without limitation.

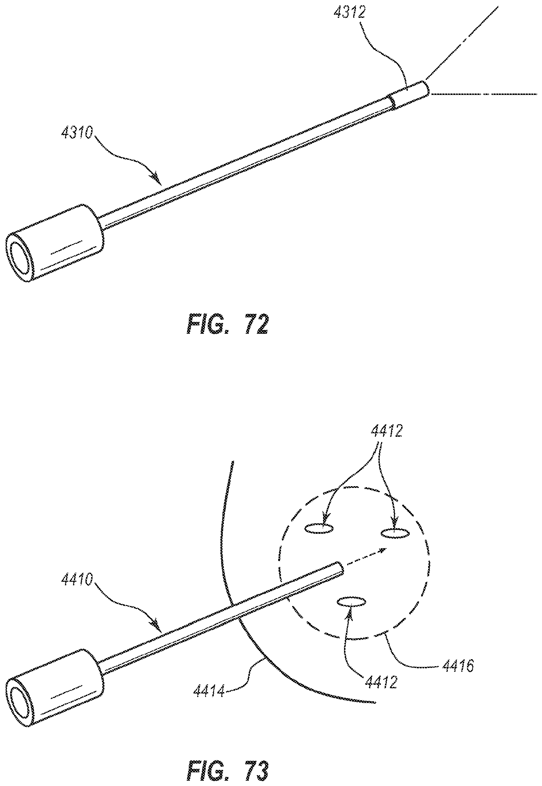

FIG. 72 illustrates a camera-enabled probe 4310 as another example of a rigid medical device for cooperative use with a rigid medical device tracking system. In the embodiment of FIG. 72, the camera-enable probe 4310 includes a small imaging device 4312, such as a charge couple device (CCD) camera, a complementary metal-oxide-semiconductor (CMOS) camera, or other suitable camera. The camera-enabled probe 4310 can be used to image particularly targeted tissue for diagnosis or therapy. The camera-enabled probe 4310 can also image optical/biological markers for diagnosis and/or photo-therapy dyes for therapy. In some cases, the probe 4310 can include only a light for photodynamic therapy.

Other non-limiting embodiments of rigid medical devices may include a tube for detection of markers to diagnose disease such as tumors, a tube for delivering therapy which interacts with a marker for therapy of tumors, and a tube to deliver DNA or other genetic material to a particular area.

FIG. 73 illustrates a marker implantation tube 4410 suitable for implanting markers 4412 that can be used for subsequent therapy such as surgery, biopsy, radiotherapy (external or internal), freezing, or other reasons. The tube 4410 is integrated into a rigid medical device for cooperative use with a rigid medical device tracking system. In the embodiment of FIG. 73, the tube 4410 can implant markers 4412 of a variety of types to enable subsequent therapy with invasive (*e.g.,* surgical) or noninvasive (*e.g.,* endoscopy or direct needle for therapy) procedures. In some embodiments, the markers are detectable by a medical imaging device such as an ultrasound or x-ray or some other type of device.

In some cases, the marker implantation tube 4410 places markers 4412 that are used to measure sizes of tissue area or other medically desired parameters with substantial accuracy. Placing such markers allows a medical practitioner to make substantially precise volume measurements of the target (*e.g.,* a tumor) over time and with therapy to determine information such as disease progression or remission. The measurements can be directly determined by detection of the markers 4412 with particular medical imaging devices such as x-ray, ultrasound, or another technology, or the measurements can be mathematically determined, via triangulation for example.

The markers 4412 can be placed inside the patient and work cooperatively with other markers that are disposed outside the patient. In some cases, the markers 4412 are fiducial markers. The markers 4412 placed by the tube 4410 may dissolve over time, remain permanently in the patient, be retrieved later, or have some other outcome.

In some cases, the tube 4410 may have an integrated or separate caliper device to measure a size of tissue area with substantial accuracy. The measuring functions of a particular tube can be used before and after therapy.

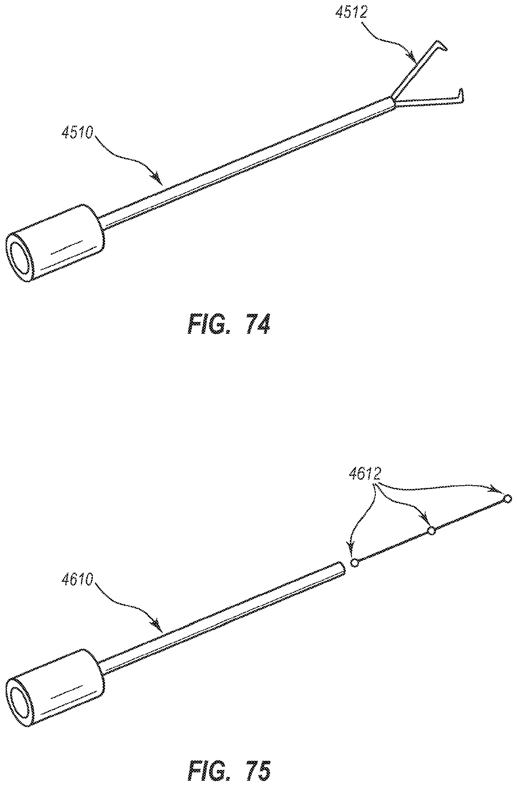

FIG. 74 illustrates a grasper tube 4510 including a grasper 4512 that can be used for holding tissue or other reasons. The grasper tube 4510 is integrated into a rigid medical device for cooperative use with a rigid medical device tracking system. In the embodiment of FIG. 74, the grasper tube 4510 can be used, for example, in a gall bladder procedure to grasp a stone.

FIG. 75 illustrates a marker implantation tube 4610 as another example of a rigid medical device for cooperative use with a rigid medical device tracking system. In the illustrated embodiment, the tube 4610 can be inserted into the patient's tissue before a plurality of markers 4612 initially carried by the tube are deployed into the tissue.

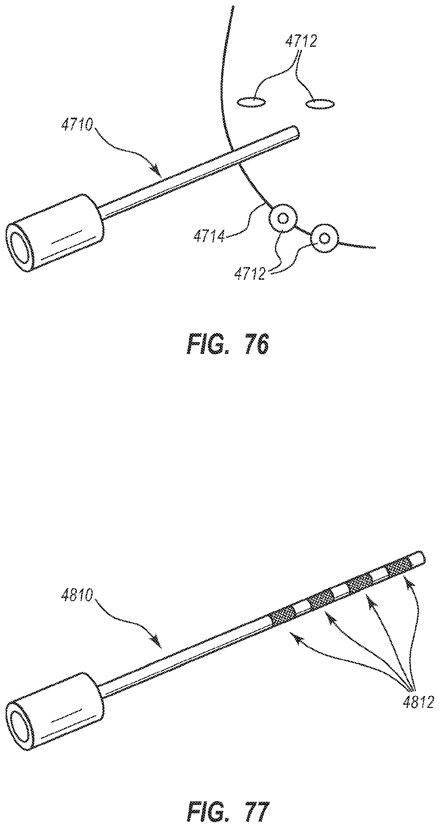

FIG. 76 illustrates an implantation tube 4710 as another example of a rigid medical device for cooperative use with a rigid medical device tracking system. In the illustrated embodiment, the implantation tube 4710 is configured for depositing fiducials 4712 on or below the skin 4714 of the patient to assist with patient therapy and other procedures.

FIG. 77 illustrates a biomarker tube 4810 that has a series of biomarkers 4812 on the tip of the tube for diagnosis in situ. In other embodiments, one or more biomarkers are placed at different locations on the biomarker tube. In a procedure, the biomarkers can be used to facilitate detection of a particular disease state or other condition.

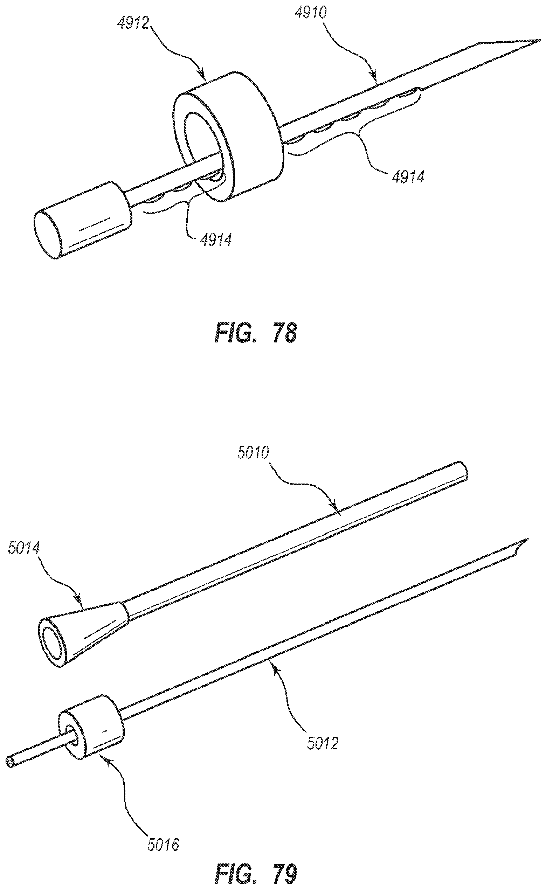

FIG. 78 illustrates a needle 4910 with marks 4914 that are readable by an encoder 4912 to indicate the depth of insertion. The needle 4910 can be integrated as a rigid medical device for cooperative use with a rigid medical device tracking system. In the embodiment of FIG. 78, the needle 4910 can be cooperatively encoded, marked, or otherwise structured for use with an encoder 4912. For example, in cases where a needle 4910 is so marked, an encoder 4912 mechanism can be used to facilitate an alert to the medical practitioner when the needle 4910 has traveled to a target depth. The alert mechanism can be in the form of a mechanical stop, electronic signal, or some other type. The constituent mechanisms can be integrated into multiple devices or channels and used in diagnosis, therapy, and other procedures.

FIG. 79 illustrates an over tube 5010 that can be used to direct a needle 5012 or other medical device to a particular area of concern in a patient's body. The over tube 5010 is integrated into a rigid medical device for cooperative use with a rigid medical device tracking system. The over tube 5010 includes a magnet 5014 used by the rigid medical device tracking system to provide location information representative of the tip of the over tube 5010. In one procedure, the tip of the over tube 5010 is manipulated and advanced into a patient's body to a particular area of concern. Subsequently, the needle 5012 is passed into the patient's body through the over tube 5010. The needle 5012 has a mechanical stop 5016 that makes contact with the proximal end of the over tube 5010 when the desired depth has been achieved.

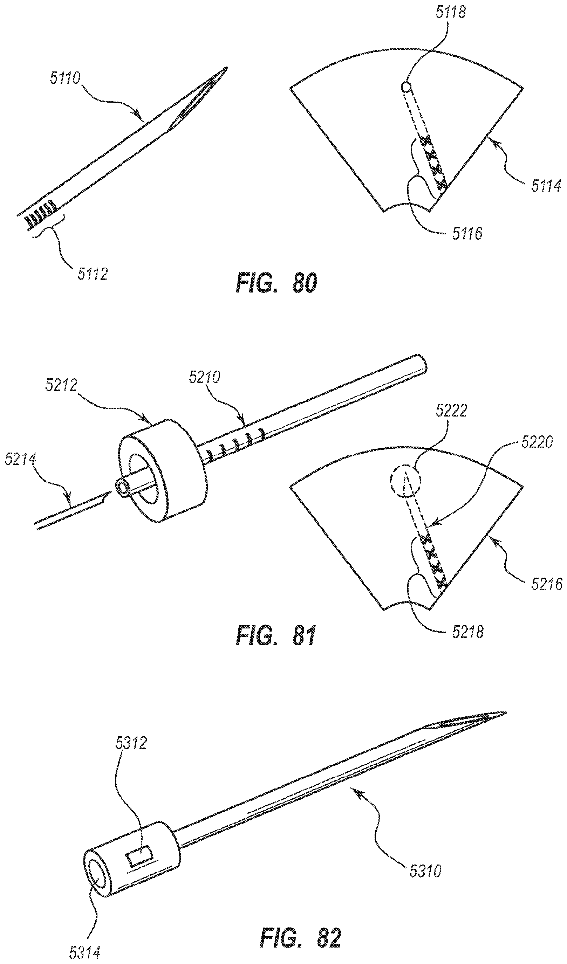

The illustration of FIG. 80 shows how an over tube 5110 is particularly encoded, according to one embodiment. The over tube 5110 can be used to direct a needle or other medical device to a particular area of concern in a patient's body. The over tube 5110 is integrated into a rigid medical device for cooperative use with a rigid medical device tracking system. In FIG. 80, a display device of a medical imaging tool works cooperatively with the rigid medical device tracking system to produce a representative image 5114. In the image, images 5116 of the encoded marks 5112 of the over tube 5110 are also visible. The encoded mark images 5116 are derived from information associated with the particular encoding of the over tube 5110.

The illustration of FIG. 81 shows an over tube 5210 according to one embodiment, and further including an optical encoder/decoder 5212 (note that the encoder/decoder may optionally include electronic, mechanical, or other suitable scheme for encoding and/or decoding functionality). In the embodiment, the encoder/decoder 5212 works cooperatively with an associated medical device 5214. The medical device 5214 is particularly encoded. As the medical device 5214 is manipulated in the over tube 5210, the encoder/decoder 5212 derives positional information from the medical device 5214. Subsequently, the rigid medical device tracking system receives positional information from the encoder/decoder 5212 and passes positional information to a medical imaging device. A display 5216 shows representative encoding marks imagery 5218, medical device imagery 5220, and an area of interest image 5222 (*e.g.,* a tumor).

FIG. 82 illustrates a rigid medical device 5310 including an identification element. The rigid medical device 5310 can be cooperatively used with a rigid medical device tracking system. In the illustrated embodiment, the rigid medical device 5310 includes a detectable identification tag 5312 (*e.g.,* a radio frequency identifier (RFID) circuit) disposed on a handle 5314 of the device. The detectable tag 5312 provides information regarding the structural parameters of the rigid medical device having identification 5310. For example, the detectable tag 5312 can provide a model number, a serial number, a manufacturing ID, device length, device type, or any other information that can assist the rigid medical device tracking system in determining positional information related to the device 5310 being tracked. In some cases, the information can be used for other purposes such as to alert a medical practitioner of a dangerous or other undesirable situation.

Embodiments of the invention may be embodied in other specific forms without departing from the present disclosure. The described embodiments are to be considered in all respects only as illustrative, not restrictive. The scope of the embodiments is, therefore, indicated by the appended claims rather than by the foregoing description.

## Claims

1. A virtual tracking system for a virtual medical device, the virtual medical device (1618) including a tangible proximal portion that is at least temporarily associated with a magnetic element (1620), and a virtual distal portion;
the system comprising:
a tracking circuit to track movement of the virtual medical device, the tracking circuit including:
at least one a reception component (1512) that detects the magnetic element (1620); and
a processor that iteratively computes position data of the virtual distal portion of the virtual medical device (1618) according to detection of the magnetic element (1620) so as to simulate insertion of a distal portion of a tangible medical device into the body of a patient (1610); and
a display (1614) for depicting an image (1722) relating to the position data of the virtual distal portion of the virtual medical device, wherein the display (1614) is configured to depict the image of the virtual distal portion of the virtual medical device and an image of the distal portion of the tangible medical device that is also tracked by the system,
**characterised in that**:
the virtual medical device (1618) is constructed of soft rubber.

2. The virtual tracking system as defined in claim 1, wherein the system is configured to guide the tangible medical device into a body of a patient.

3. The virtual tracking system as defined in claim 1,
wherein the image of the virtual distal portion is saved from a previous procedure and wherein the image of the tangible medical device is displayed in real time.

4. The virtual tracking system as defined in any of the preceding claims, wherein the display (1614) is included in an ultrasound imaging system (1612), and wherein the reception component includes a plurality of sensors disposed in a handheld probe (1140) of the ultrasound imaging system, the sensors configured to detect a field associated with the magnetic element.

5. The virtual tracking system as defined in any of the preceding claims, wherein the tangible proximal portion includes a permanent magnet (1620) disposed therewith.

6. The virtual tracking system as defined in any of the preceding claims, wherein the virtual medical device is a virtual needle (1618), and/or the tangible proximal portion of the virtual needle is a stub portion or the virtual needle is constructed of soft rubber or the virtual needle is formed so as to be collapsible.

7. The virtual tracking system as defined in any of the preceding claims, the virtual medical device further including the tangible proximal portion that is at least temporarily associated with the magnetic element.

8. The virtual tracking system as defined in any of the preceding claims, wherein the system is configured such that after a virtual procedure is conducted where a virtual image representation of a rigid medical device has been tracked into a patient's body, an actual rigid medical device is advanced on the virtual track previously determined.

9. Use of the virtual tracking system as defined in any of the preceding claims for a planning procedure, wherein no part of the virtual medical device is advanced into the body of the patient.

## Patentansprüche

1. Virtuelles Verfolgungssystem für eine virtuelle medizinische Vorrichtung, wobei die virtuelle medizinische Vorrichtung (1618) einen greifbaren proximalen Abschnitt, der mindestens vorübergehend mit einem magnetischen Element (1620) verbunden ist, und einen virtuellen distalen Abschnitt einschließt;
das System umfassend:
eine Verfolgungsschaltung zum Verfolgen von Bewegung der virtuellen medizinischen Vorrichtung, wobei die Verfolgungsschaltung einschließt:
mindestens eine Empfangskomponente (1512), die das magnetische Element (1620) erfasst; und
einen Prozessor, der iterativ Positionsdaten des virtuellen distalen Abschnitts der virtuellen medizinischen Vorrichtung (1618) entsprechend der Erfassung des magnetischen Elements (1620) berechnet, um ein Einführen eines distalen Abschnitts einer greifbaren medizinischen Vorrichtung in den Körper eines Patienten (1610) zu simulieren; und
eine Anzeige (1614) zum Darstellen eines Bildes (1722) bezüglich der Positionsdaten des virtuellen distalen Abschnitts der virtuellen medizinischen Vorrichtung, wobei die Anzeige (1614) konfiguriert ist, um das Bild des virtuellen distalen Abschnitts der virtuellen medizinischen Vorrichtung und ein Bild des distalen Abschnitts der greifbaren medizinischen Vorrichtung, die auch durch das System verfolgt wird, darzustellen,
**dadurch gekennzeichnet, dass**:
die virtuelle medizinische Vorrichtung (1618) aus weichem Gummi konstruiert ist.

2. Virtuelles Verfolgungssystem nach Anspruch 1, wobei das System konfiguriert ist, um die greifbare medizinische Vorrichtung in einen Körper eines Patienten zu führen.

3. Virtuelles Verfolgungssystem nach Anspruch 1, wobei das Bild des virtuellen distalen Abschnitts von einer vorherigen Prozedur gespeichert ist und wobei das Bild der greifbaren medizinischen Vorrichtung in Echtzeit angezeigt ist.

4. Virtuelles Verfolgungssystem nach einem der vorstehenden Ansprüche, wobei die Anzeige (1614) in einem Ultraschall-Bildgebungssystem (1612) eingeschlossen ist, und wobei die Empfangskomponente eine Vielzahl von Sensoren einschließt, die in einer handgehaltenen Sonde (1140) des Ultraschall-Bildgebungssystems angeordnet ist, wobei die Sensoren konfiguriert sind, um ein mit dem magnetischen Element verbundenes Feld zu erfassen.

5. Virtuelles Verfolgungssystem nach einem der vorstehenden Ansprüche, wobei der greifbare proximale Abschnitt einen damit angeordneten Dauermagneten (1620) einschließt.

6. Virtuelles Verfolgungssystem nach einem der vorstehenden Ansprüche, wobei die virtuelle medizinische Vorrichtung eine virtuelle Nadel (1618) ist und/oder der greifbare proximale Abschnitt der virtuellen Nadel ein Stumpfabschnitt ist oder die virtuelle Nadel aus weichem Gummi konstruiert ist oder die virtuelle Nadel gebildet ist, um zusammenklappbar zu sein.

7. Virtuelles Verfolgungssystem nach einem der vorstehenden Ansprüche, wobei die virtuelle medizinische Vorrichtung weiter den greifbaren proximalen Abschnitt einschließt, der mindestens vorübergehend mit dem magnetischen Element verbunden ist.

8. Virtuelles Verfolgungssystem nach einem der vorstehenden Ansprüche, wobei das System konfiguriert ist sodass, nach Durchführen einer virtuellen Prozedur, bei der eine virtuelle Bilddarstellung einer starren medizinischen Vorrichtung in den Körper eines Patienten verfolgt wurde, eine tatsächliche starre medizinische Vorrichtung auf der zuvor bestimmten virtuellen Spur vorgeschoben wird.

9. Verwendung des virtuellen Verfolgungssystems nach einem der vorstehenden Ansprüche für eine Planungsprozedur, wobei kein Teil der virtuellen medizinischen Vorrichtung in den Körper des Patienten vorgeschoben wird.

## Revendications

1. Système de suivi virtuel pour un dispositif médical virtuel, le dispositif médical virtuel (1618) incluant une partie proximale tangible qui est au moins provisoirement associée à un élément magnétique (1620), et une partie distale virtuelle ;
le système comprenant :
un circuit de suivi pour suivre le déplacement du dispositif médical virtuel, le circuit de suivi incluant :
au moins un composant de réception (1512) qui détecte l'élément magnétique (1620) ; et
un processeur qui calcule de manière itérative des données de position de la partie distale virtuelle du dispositif médical virtuel (1618) en fonction de la détection de l'élément magnétique (1620) de façon à simuler l'insertion d'une partie distale d'un dispositif médical tangible dans le corps d'un patient (1610) ; et
un afficheur (1614) pour représenter une image (1722) se rapportant aux données de position de la partie distale virtuelle du dispositif médical virtuel, dans lequel l'afficheur (1614) est configuré pour représenter l'image de la partie distale virtuelle du dispositif médical virtuel et une image de la partie distale du dispositif médical tangible qui est également suivi par le système,
**caractérisé en ce que** :
le dispositif médical virtuel (1618) est construit de caoutchouc mou.

2. Système de suivi virtuel selon la revendication 1, dans lequel le système est configuré pour guider le dispositif médical tangible dans un corps d'un patient.

3. Système de suivi virtuel selon la revendication 1, dans lequel l'image de la partie distale virtuelle est sauvegardée à partir d'une procédure précédente et dans lequel l'image du dispositif médical tangible est affichée en temps réel.

4. Système de suivi virtuel selon l'une quelconque des revendications précédentes, dans lequel l'afficheur (1614) est inclus dans un système d'imagerie par ultrasons (1612), et dans lequel le composant de réception inclut une pluralité de capteurs disposés dans une sonde portative (1140) du système d'imagerie par ultrasons, les capteurs étant configurés pour détecter un champ associé à l'élément magnétique.

5. Système de suivi virtuel selon l'une quelconque des revendications précédentes, dans lequel la partie proximale tangible inclut un aimant permanent (1620) disposé avec celle-ci.

6. Système de suivi virtuel selon l'une quelconque des revendications précédentes, dans lequel le dispositif médical virtuel est une aiguille virtuelle (1618), et/ou la partie proximale tangible de l'aiguille virtuelle est une partie embase ou l'aiguille virtuelle est construite de caoutchouc mou ou l'aiguille virtuelle est formée de manière à pouvoir se replier.

7. Système de suivi virtuel selon l'une quelconque des revendications précédentes, le dispositif médical virtuel incluant en outre la partie proximale tangible qui est au moins provisoirement associée à l'élément magnétique.

8. Système de suivi virtuel selon l'une quelconque des revendications précédentes, dans lequel le système est configuré de telle sorte que, après la réalisation d'une procédure virtuelle où une représentation d'image virtuelle d'un dispositif médical rigide a été suivie dans le corps d'un patient, un dispositif médical rigide réel est avancé sur le suivi virtuel préalablement déterminé.

9. Utilisation du système de suivi virtuel tel que défini dans l'une quelconque des revendications précédentes pour une procédure de planification, dans laquelle aucune partie du dispositif médical virtuel n'est avancée dans le corps du patient.
